# EUROPÄISCHE PATENTANMELDUNG

(11) **EP 2 156 843 A2**
(43) Veröffentlichungstag der Anmeldung: **24.02.2010**
(21) Anmeldenummer: 09014673.9
(22) Anmeldetag: 22.01.2005
(51) Int. Cl.: A61K 38/18, A61P 13/12, A61P 17/02

(54) **Verwendung von niedrig dosiertem Erythropoietin zur Behandlung von akuter oder chronischer Niereninsuffizienz und zur Behandlung von Wunden**

(30) Priorität: 23.01.2004 DE 102004004509
(62) Teilanmeldung aus: 05715203.5
(71) Anmelder: EPOPLUS GmbH & Co. KG, 30625 Hannover (DE)
(72) Erfinder: Bahlmann, Ferdinand Hermann, 30173 Hannover (DE); Haller Hermann, 30559 Hannover (DE)
(74) Vertreter: Schrell, Andreas

(57) **Zusammenfassung**

Die vorliegende Erfindung betrifft die Verwendung von niedrig dosiertem Erythropoietin (0,001 bis 90 IU-kg Körpergewicht und Woche) zur Stimulierung der physiologischen Mobilisierung, Proliferation und Differenzierung endothelialer Vorläuferzellen, zur Stimulierung der Vaskulogenese, zur Therapie von Krankheiten, die mit einer Dysfunktion endothelialer Vorläuferzellen im Zusammenhang stehen, und zur Herstellung pharmazeutischer Zusammensetzungen zur Behandlung derartiger Krankheiten sowie pharmazeutische Zusammensetzungen, die Erythropoietin und andere geeignete Wirkstoffe zur Stimulation endothelialer Vorläuferzellen umfassen.

## Beschreibung

Die vorliegende Erfindung betrifft die Verwendung von, insbesondere niedrig dosiertem, Erythropoietin (EPO) allein oder in Kombination mit anderen chemischen, thermischen, mechanischen sowie biologischen Agenzien zur Stimulierung der physiologischen Mobilisierung, Proliferation und Differenzierung endothelialer Vorläuferzellen, zur Stimulierung der Vaskulogenese, zur Therapie von Krankheiten, die mit einer Dysfunktion endothelialer Vorläuferzellen im Zusammenhang stehen, und zur Herstellung pharmazeutischer Zusammensetzungen zur Behandlung solcher Krankheiten sowie pharmazeutische Zusammensetzungen, die Erythropoietin und andere geeignete Wirkstoffe zur Stimulation endothelialer Vorläuferzellen umfassen sowie zur Organprotektion, Organregeneration, insbesondere Gefäß- und Gewebeneubildung und Progressionsverlangsamung von Organschäden.

Die vorliegende Erfindung betrifft auch die Verwendung von Erythropoietin, insbesondere in den erfindungsgemäßen niedrigen Dosierungen, und/oder geeigneten Wirkstoffen zur, vorzugsweise topischen, Anwendung im Rahmen einer kosmetischen Behandlung, also im Sinne der "Schönheitspflege", des menschlichen oder tierischen Körpers insbesondere zur Prophylaxe oder Reduktion von Falten und Fältchen, Stärkung des Bindegewebes, zum Schutz sowie zur Straffung der Haut, insbesondere der Gesichtshaut, gegen schädliche Umweltfaktoren und als Make up Unterlage. Des weiteren wirkt die erfindungsgemäße topische Verwendung von Erythropoietin der Entstehung und Weiterentwicklung von Altersflecken entgegen, verfeinert das Hautbild, sowie unterstützt den Erneuerungsprozess der Haut und beschleunigt den Haarwuchs.

Die vorliegende Erfindung betrifft auch die Verwendung von, vorzugsweise niedrig dosiertem, Erythropoietin, das heißt EPO vorzugsweise dosiert wie im nachstehenden Absatz "Erfindungsgemäße Dosierung von EPO" definiert, zur Herstellung einer pharmazeutischen Zusammensetzung geeignet und bestimmt zur Applikation in Anpassung an dessen natürlichen circardianen Rhythmus im menschlichen oder tierischen Körper. Die endogene Erythropoietinproduktion besitzt beim Menschen ihre Akrophase (Tagesmaximum) am späten Nachmittag, daher erfolgt die erfindungsgemäße Gabe von wie vorstehend definierten niedrig dosiertem Erythropoietin vorzugsweise vormittags, insbesondere im Zeitraum von 6:00 bis 10:00, um so einen maximalen biologischen und damit auch therapeutischen Effekt zu erzielen. Innerhalb dieses Zeitraums kann das EPO als Einzeldosis oder in Mehrfachdosierung verabreicht werden. Diese Verwendung als Einzeldosis oder in Mehrfachdosierung wird erfindungsgemäß besonders bevorzugt vorgeschlagen für alle gemäß der vorliegenden Lehre genannten Verwendungen, insbesondere zur kosmetischen und therapeutischen Behandlung des menschlichen und tierischen Körpers oder Zelle.

Erfindungsgemäß ist in einer weiteren Ausführungsform der Verwendung von Erythropoietin vorgesehen, endotheliale Vorläuferzellen zeitgleich mit anderen zelltherapeutischen einsetzbaren Zellpopulationen unter vorheriger Inkubation mit, vorzugsweise niedrig dosiertem, Erythropoietin in vitro und/oder lokaler oder systemischer Applikation von, vorzugsweise erfindungsgemäß niedrig dosiertem, Erythropoietin in vivo zu applizieren, um so das Einheilen der zelltherapeutischen Gewebszellen mit suffizienter Anbindung an das Gefäßsystem sicherzustellen.

Die Erfindung betrifft daher auch die Verwendung von, vorzugsweise niedrig dosiertem, Erythropoietin in vivo, vorzugsweise für eine Applikation morgens in einem Zeitraum vom 06:00 bis 10:00, bei der Applikation von endothelialen Vorläuferzellen mit mindestens einer zelltherapeutisch einsetzbaren Zellpopulation zur Verbesserung des Einheilens der zelltherapeutisch einsetzbaren Zellpopulation mit suffizienter Anbindung an das Gefäßsystem. Die Erfindung betrifft auch die Verwendung von, vorzugsweise niedrig dosiertem, Erythropoietin in vitro zur Inkubation mit endothelialen Vorläuferzellen und mindestens einer zelltherapeutisch einsetzbaren Zellpopulation zur Verbesserung des Einheilens der zelltherapeutisch einsetzbaren Zellpopulation mit suffizienter Anbindung an das Gefäßsystem.

Die Erfindung betrifft auch die Verwendung von Erythropoietin, insbesondere in niedriger Dosierung, insbesondere für die Herstellung einer pharmazeutischen Zusammensetzung oder eines Kits zur Prophylaxe oder Behandlung von Krankheiten oder eingesetzt im Rahmen von Transplantationen oder Implantationen, in sequenzieller zeitlich aufeinanderfolgender Gabe mit mindestens einem anderen chemischen, thermischen, mechanischen oder biologischen Agens, insbesondere pharmakologischen Wirkstoff, zur Erhöhung der Zahl und Funktion endothelialer Vorläuferzellen und/oder zur Regeneration beziehungsweise zur Progressionsverlangsamung von Gewebeschäden.

Die Erfindung betrifft auch die Verwendung von Erythropoietin, insbesondere für die Herstellung einer pharmazeutischen Zusammensetzung oder eines Kits, zur Prophylaxe oder Behandlung von Krankheiten oder eingesetzt im Rahmen von Transplantationen oder Implantationen, insbesondere in niedriger Dosierung, für die gleichzeitige Gabe von Erythropoietin und mindestens einem anderen chemischen, thermischen, mechanischen oder biologischen Agens zur Erhöhung der Zahl und Funktion endothelialer Vorläuferzellen und/oder zur Regeneration beziehungsweise Progressionsverlangsamung von Gewebeschäden.

Die Erfindung betrifft daher die vorzugsweise sequenzielle, zeitlich aufeinanderfolgende oder gleichzeitige Gabe von niedrig dosiertem Erythropoietin sowie in bevorzugter Ausführung einer oder mehrerer anderer pharmakologischer Wirkstoffe, z.B. VEGF; GM-CSF, M-CSF, Thrombopoietin, SDF-1, SCF, NGF, PIGF, einem HMG-Co-Reduktase-Inhibitor, einem ACE-Hemmer, einem AT-1 Hemmer und einem NO-Donator, um so die Zahl und Funktion endothelialer Vorläuferzellen zu erhöhen und/oder die Regeneration bzw. Progressionsverlangsamung von Gewebeschäden herbeizuführen. Dabei soll erfindungsgemäß die Sequenz A) Quantitative und qualitative Optimierung von Stammzellen und/oder endotheliale Vorläuferzellen im Knochenmark oder in gewebestämmigen "Stammzell"-Nischen B) Mobilisierung von Stammzellen und/oder endotheliale Vorläuferzellen aus dem Knochenmark oder anderen "Stammzell"-Nischen ins periphere Blut C) Quantitative und qualitative Optimierung von Stammzellen und/oder endotheliale Vorläuferzellen im peripheren Blut und/oder ex vivo unter selektiven Kulturbedingungen, vorzugsweise die Kultur unter hypoxischen Bedingungen mit einem Sauerstoffanteil von 0,1% bis 10% D) Homing von Stammzellen und/oder endothelialen Vorläuferzellen an den Ort der Schädigung E) Adhäsion und Migration von Stammzellen und/oder endotheliale Vorläuferzellen in das Zielgewebe F) Neovaskularisierung durch endotheliale Vorläuferzellen beeinflusst werden.

Die Erfindung betrifft also auch, insbesondere in vivo und in vitro, die sequenzielle, zeitlich aufeinanderfolgende oder gleichzeitige Gabe von erfindungsgemäß niedrig dosiertem Erythropoietin sowie gegebenenfalls einer oder mehrerer anderer chemischer, thermischer, mechanischer sowie biologischer Agenzien, um so die Zahl und Funktion endothelialer Vorläuferzellen zu erhöhen und/oder die Regeneration bzw. Progressionsverlangsamung von Gewebeschäden herbeizuführen, optional und vorzugsweise in der vorstehend beschriebenen Verwendung in Anpassung an den natürlichen circadianen Rhythmus der körpereigenen EPO-Produktion, also in einer Applikationsform, die für eine Gabe im Zeitraum von 6:00 bis 10:00 morgens geeignet und bestimmt ist.

Die vorliegende Erfindung betrifft auch die Verwendung von erfindungsgemäß niedrig dosiertem Erythropoietin zur Stimulierung der physiologischen Mobilisierung, Proliferation und Differenzierung endothelialer Vorläuferzellen, zur Stimulierung der Vaskulogenese, zur Therapie von Krankheiten, die mit einer Dysfunktion endothelialer Vorläuferzellen im Zusammenhang stehen, und zur Herstellung pharmazeutischer Zusammensetzungen zur Behandlung solcher Krankheiten sowie von pharmazeutischen Zusammensetzungen, die Erythropoietin und andere geeignete Wirkstoffe zur Stimulation endothelialer Vorläuferzellen umfassen, von oder für Patienten mit a) einer Dysfunktion endothelialer Vorläuferzellen und b) mit mindestens einem kardiovaskulären Risikofaktor wie Hypertonie, Hypercholesterinämie, erhöhte Asymmetrisches Dimethylarginin (ADMA) - Werte, Insulinresistenz, Hyperhomocysteinämie und c) zumindest einem Endorganschaden wie linksventrikuläre Hypertrophie, Mikroalbuminurie, kognitive Dysfunktion, Zunahme der Intima-Media-Dicke in der A. carotis, Proteinurie oder einer glomerulären Filtrationsrate (GFR)< 80ml/min, insbesondere 30, vorzugsweise 40, bis 80 ml/min. Vorzugsweise betrifft die Erfindung die vorstehend genannte Verwendung von niedrig dosiertem EPO bei der vorstehend genannten in a) bis c) definierten Patientengruppe in einer Ausführung, die geeignet und bestimmt ist, die EPO Applikation in einem Zeitraum von 6:00 bis 10:00 morgens vorzunehmen.

Das Gefäßendothel ist eine Schicht von Zellen, die die Blutgefäße auskleidet. Das Endothel trennt das Blut von anderen Gefäßschichten, wobei das Endothel nicht allein eine passive Barriere darstellt, sondern aktiv in die Regulation des Gefäßtonus eingreift. Dementsprechend wird auch von einer Endothel-abhängigen Vasodilatation gesprochen. Auf Grund seiner Lage ist das Endothel permanent dem hämodynamischen Stress und dem metabolischen Stress ausgesetzt. Bei pathogenen Zuständen, beispielsweise erhöhtem Blutdruck, erhöhten LDL-Werten, erhöhten Werte des endogenen Inhibitors der NO-Synthetase Asymmetrisches Dimethylarginin (ADMA), Hypercholersterinämie, eingeschränkter Nierenfunktion mit einer glomerulären Filtrationsrate von 30, vorzugsweise 40, bis 80ml/min, Insulinresistenz oder erhöhtem Blutzucker, kommt es daher häufig zu funktionellen Defekten des Endothels, denen dann morphologisch fassbare Schäden, wie die Bildung atherosklerotischer Plaques und/oder weiterer Endorganschäden, wie linksventrikuläre Hypertrophie, Mikroalbuminurie, Proteinurie, Neuropathien oder Mikrozirkulationsstörungen folgen können. Ein sehr frühes Zeichen einer geänderten beziehungsweise reduzierten endothelialen Funktion beziehungsweise endothelilen Dysfunktion ist eine Verminderung der Endothel-abhängigen Vasodilatation.

Bei koronarer Herzkrankheit (KHK), aber auch bei vorhandenen Risikofaktoren ohne KHK, beispielsweise Hypertonie, eingeschränkter Nierenfunktion, Hyperlipoproteinämie, Hyperhomocysteinämie, Insulinresistenz oder Diabetes, äußern sich Endothel-Funktionsdefekte in einer verminderten Produktion von NO (=EDRF) und erhöhter Endothelin-Produktion. Hohe Plasmaspiegel von Endothelin führen zu abnormalem Zusammenwachsen von Zellen, Entzündungen, Gefäßwucherungen und starken Gefäßverengungen. Endothel-Funktionsstörungen sind zudem durch eine verstärkte Produktion von Adhäsionsmolekülen wie ICAM-1 und VCAM-1 gekennzeichnet, wodurch Thrombozyten und Monozyten in erhöhtem Maße am Endothel haften. Dadurch bedingt kommt es zu einer Erhöhung des Gefäßtonus. Somit bildet sich bei verschiedensten Systemen ein Ungleichgewicht heraus, wobei Vasokonstriktion, Adhäsion, Aggregation, Koagulation, Atherosklerose und Atherothrombose begünstigt werden. Selbst mentaler Stress führt zu einer messbaren Endothel-Dysfunktion, die bis zu 4 Stunden anhalten kann.

Endothelzellen sind auch an der Bildung neuer Blutgefäße beteiligt. Die Bildung von Blutgefäßen ist bei einer Vielzahl von Vorgängen, wie zum Beispiel der Embryogenese, dem weiblichen Reproduktionszyklus, der Wundheilung, dem Tumorwachstum und der Neovaskularisierung ischämischer Bereiche, von Bedeutung. Ursprünglich wurde die postnatale Blutgefäßbildung, also die Blutgefäßbildung nach der Geburt, hauptsächlich auf angiogenetische Prozesse zurückgeführt. Unter Angiogenese wird die Ausbildung neuer Blutgefäße durch das Aussprossen von Kapillaren aus einem bereits bestehenden Gefäßsystem verstanden. Bei der Angiogenese wird zunächst die die Blutgefäße umgebende Basalmembran mittels proteolytischer Enzyme abgebaut und die extrazelluläre Matrix im perivaskulären Raum fragmentiert. Die dabei freigesetzten angiogenen Stimuli bewirken, dass bereits vorhandene ausdifferenzierte Endothelzellen in Richtung des chemotaktischen Reizes migrieren, wobei sie gleichzeitig proliferieren und umgebaut werden. Durch die Aneinanderlagerung von Endothelzellen werden dann neue Gefäßschleifen mit einem kapillarförmigen Lumen ausgebildet. Danach setzt die Synthese einer neuen Basalmembran ein.

Neuere Untersuchungen zeigen jedoch, dass die Bildung neuer Blutgefäße im adulten Organismus nicht nur auf Angiogenese beruht, sondern auch auf vaskulogenetischen Mechanismen. Unter Vaskulogenese wird die Gefäßneubildung aus sich in situ differenzierenden endothelialen Vorläuferzellen verstanden. Das Dogma, dass die Vaskulogenese auf die Embryogenese beschränkt ist, wurde durch den Nachweis endothelialer Vorläuferzellen (EPC) im peripheren Blut von gesunden Menschen und Tieren widerlegt. Unter Verwendung von Tiermodellen konnte belegt werden, dass die aus dem Knochenmark stammenden endothelialen Vorläuferzellen aktiv an der Neovaskularisation beteiligt sind. Auch wurde gezeigt, dass sich in ischämischen Regionen eine spezifische CD34-positive Untergruppe von Leukozyten festsetzt, die Endothel-spezifische Antigene exprimiert. Darüber hinaus lassen sich aus CD133+- und CD34+-Zellen in vitro endotheliale Vorläuferzellen (EPC) gewinnen, die einen signifikanten Beitrag zur Blutgefäßbildung im adulten Organismus leisten (Asahara et al., Science, 275 (1997), 964-967; Crosby et al., Circ. Res., 87 (2000), 728-730; Gehling et al., Blood, 95 (2000), 3106-3112). Ferner wurde gezeigt, dass die Injektion von isolierten CD34+-Zellen oder kultivierten endothelialen Vorläuferzellen die Wiederherstellung des Blutflusses bei diabetischen Mäusen beschleunigt (Schatteman et al., J. Clin. Invest., 106 (2000), 571-578) und die Neovaskularisierung in vivo verbessert (Asahara et al., Circ. Res., 85 (1999), 221-228; Crosby et al., Circ. Res., 87 (2000), 728-730; Murohara et al., J. Clin. Invest., 105 (2000), 1527-1536). Darüber hinaus konnte gezeigt werden, dass eine durch CD34+-Zellen induzierte Neovaskularisierung die Herzfunktion verbessert (Kocher et al., Nat. Med., 7 (2001), 430-436). Neben CD34+-Zellen können auch CD34-negative mononukleäre Blutzellen durch entsprechende Transdifferenzierung als Quelle für endotheliale Vorläuferzellen dienen.

Die der Mobilisierung und Differenzierung endothelialer Vorläuferzellen zugrunde liegenden Mechanismen sind jedoch noch nicht vollständig geklärt. Molekular- und zellbiologische Untersuchungen deuten darauf hin, dass verschiedene Cytokine und angiogene Wachstumsfaktoren stimulierend auf die Mobilisierung endothelialer Vorläuferzellen im Knochenmark wirken. So ist bekannt, dass proangiogene Faktoren wie VEGF und GM-CSF die Anzahl endothelialer Vorläuferzellen erhöhen können (Asahara et al., EMBO J., 18 (1999), 3964-3972; Takahashi et al., Nat. Med., 5 (1999), 434-438). Bei VEGF (Vascular Endothelial Growth Factor) handelt es sich um ein in verschiedenen Isoformen vorkommendes Protein, das an die beiden Tyrosinkinase-Rezeptoren VEGF-R1 (flt-1) und VEGF-R2 (flk-1) bindet, die beispielsweise auf der Oberfläche wachsender Endothelzellen vorkommen (Wernert et al., Angew. Chemie, 21 (1999), 3432-3435). Die Aktivierung der VEGF-Rezeptoren führt über den Ras-Raf-MAP-Kinase-Weg zur Expression von Proteinasen und spezifischen Integrinen auf der Oberfläche von Endothelzellen beziehungsweise endothelialen Vorläuferzellen und schließlich zur Initiierung der Proliferation und Migration dieser Zellen in Richtung des angiogenen Stimulus. Bei GM-CSF (Granulozyten-Makrophagen-Koloniestimulierender Faktor) handelt es sich um ein Cytokin, das bislang vor allem für die Stimulation weißer Blutkörperchen einschließlich Neutrophiler, Makrophagen und Eosinophiler bekannt war. Von PIGF (Plazenta-Wachstumsfaktor) ist bekannt, dass er die Mobilisierung von Endothel-Vorläuferzellen stimuliert, nicht jedoch deren Proliferation. Aus Untersuchungen von Llevadot et al. (J. Clin. Invest., 108 (2001), 399-405) geht hervor, dass HMG-CoA-Reduktase-Inhibitoren, insbesondere Statine, die als Lipid-senkende Medikamente eingesetzt werden und die Morbidität und Mortalität einer Koronarkrankheit reduzieren, endotheliale Vorläuferzellen mobilisieren können. Dimmeler et al. (J. Clin. Invest., 108 (2001), 391-397) konnten ferner zeigen, dass Statine wie Atorvastatin und Simvastatin die Differenzierung endothelialer Vorläuferzellen in mononukleären Zellen und CD34+-Stammzellen, die aus peripherem Blut isoliert wurden, in vitro und in vivo signifikant verbessern. So führte die Behandlung von Mäusen mit Statinen zu einer erhöhten Anzahl differenzierter endothelialer Vorläuferzellen, wobei Statine eine gleich starke Wirkung wie VEGF zeigten.

Der vorliegenden Erfindung liegt das technische Problem zugrunde, Mittel und Verfahren zur verbesserten Stimulation endothelialer Vorläuferzellen und zur Therapie von Erkrankungen bereitzustellen, die insbesondere im Zusammenhang mit einer Dysfunktion endothelialer Vorläuferzellen stehen sowie Mittel und Verfahren zur Protektion und Regeneration von unterschiedlichen Geweben bereitzustellen.

Die vorliegende Erfindung löst dieses technische Problem durch die Lehre, insbesondere niedrig dosiertes, Erythropoietin und/oder dessen Derivate zur Stimulation der physiologischen Mobilisierung endothelialer Vorläuferzellen, der Proliferation endothelialer Vorläuferzellen, der Differenzierung endothelialer Vorläuferzellen zu Endothelzellen und/oder der Migration endothelialer Vorläuferzellen in Richtung eines angiogenen oder vaskulogenen Stimulus in einem menschlichen oder tierischen Körper zu verwenden. Die erfindungsgemäße Stimulierung der Mobilisierung und/oder Differenzierung endothelialer Vorläuferzellen stellt eine wichtige neue therapeutische Strategie zur Erhöhung der postnatalen Neovaskularisation, insbesondere der Vaskulogenese, und zur Behandlung von Krankheiten, die im Zusammenhang mit einer Dysfunktion von endothelialen Vorläuferzellen und/oder Endothelzellen stehen, sowie der Protektion und Regeneration unterschiedlicher Gewebe durch schädigende chemische, thermische, mechanische und biologische Agenzien, dar.

Die vorliegende Erfindung löst dieses technische Problem auch durch die Lehre, niedrig dosiertes Erythropoietin und/oder dessen Derivate zur Therapie von Krankheiten oder krankhafte Zuständen, die mit einer Dysfunktion von endothelialen Vorläuferzellen und/oder Endothelzellen im Zusammenhang stehen, zu verwenden.

Des weiteren löst die vorliegende Erfindung dieses technische Problem auch durch die Lehre, niedrig dosiertes Erythropoietin und/oder dessen Derivate zur Protektion und Regeneration unterschiedlicher Gewebetypen im Zustand von Krankheiten oder krankhaften Zuständen, die mit einer Dysfunktion der jeweils spezifischen Gewebefunktion im Zusammenhang stehen, zu verwenden.

Auch wird das zugrundeliegende technische Problem durch die sequenzielle, zeitlich aufeinanderfolgende oder gleichzeitige Gabe von niedrig dosiertem Erythropoietin sowie einer oder mehrerer anderer chemischer, thermischer, mechanischer sowie biologischer Agenzien gelöst.

Die Erfindung betrifft daher insbesondere die folgenden Ausführungsformen A) bis K) jeweils allein und/oder in Kombination:
A) Verwendung von Erythropoietin, bevorzugt zur Herstellung einer pharmazeutischen Zusammensetzung, für die Prophylaxe oder Behandlung von Krankheiten, wobei das Erythropoietin oder/und die pharmazeutische Zusammensetzung geeignet und bestimmt ist für die morgendliche Applikation an einen menschlichen oder tierischen Körper in einem Zeitraum von 6:00 bis 10:00.
B) Verwendung von Erythropoietin, bevorzugt in Kombination mit Ausführungsform A), insbesondere zur Herstellung einer pharmazeutischen Zusammensetzung, für die Prophylaxe oder Behandlung von Krankheiten, wobei die pharmazeutische Zusammensetzung in ihrer niedrigen Dosierung geeignet und bestimmt ist für die Prophylaxe oder Behandlung eines menschlichen oder tierischen Patienten der a) mindestens eine Dysfunktion endothelialer Vorläuferzellen, b) mindestens einen cardiovaskulären Risikofaktor wie Hypertonie, Hypercholestrinemie, Insulinresistenz, erhöhte ADMA Werte oder Hyperhomocytenamie und c) mindestens einen Endorganschaden wie linksventrikuläre Hypertrophie, Mikroalbuminurie, kognitive Dysfunktion, Zunahme der Intima-Media-Dicke in der A. carotis, Proteinurie oder eine glomeruläre Filtrationsrate < 80 ml/min, vorzugsweise 30 bis 80 ml/min aufweist.
C) Verwendung von Erythropoietin, vorzugsweise in Kombination mit Ausführungsform A), B) oder A) und B), für die kosmetische Behandlung des menschlichen oder tierischen Körpers, insbesondere zur Behandlung von Faltenbildung, zur Stärkung des Bindegewebes, zum Schutz und zur Straffung der Haut, zum Schutz vor schädigenden Umwelteinwirkungen, zur Behandlung von Altersflecken, zur Beschleunigung der Reepithelialisierung, zur Beschleunigung des Haarwuchses und/oder als Make up-Unterlage.
D) Verwendung von Erythropoietin, vorzugsweise in Kombination mit Ausführungsform A), B) oder A) und B), zur Herstellung einer kosmetischen Präparation, insbesondere zur topischen Applikation, für die kosmetische Behandlung des menschlichen oder tierischen Körpers, insbesondere zur Behandlung von Faltenbildung, zur Stärkung des Bindegewebes, zum Schutz und zur Straffung der Haut, zum Schutz vor schädigenden Umwelteinwirkungen, zur Behandlung von Altersflecken, zur Beschleunigung der Reepithelialisierung, zur Beschleunigung des Haarwuches, und/oder als Make up-Unterlage.
E) Verwendung von Erythropoietin, bevorzugt in Kombination mit einer oder mehreren der Ausführungsformen nach A), B), C) oder D), und/oder eine Mischung von endothelialen Vorläuferzellen mit mindestens einer zelltherapeutisch einsetzbaren Zellpopulation zur Herstellung einer pharmazeutischen Zusammensetzung, enthaltend Erythropoietin und eine Mischung aus endothelialen Vorläuferzellen mit mindestens einer zelltherapeutisch einsetzbaren Zellpopulation für die Regeneration von Gewebe oder Gefäßen in einem menschlichen oder tierischen Körper, wobei die Mischung vor der Applikation in vitro mit Erythropoietin in Kontakt gebracht worden ist.
F) Verwendung von Erythropoietin, bevorzugt in Kombination mit einer oder mehreren der Ausführungsformen nach A), B), C), D) oder E), und/oder eine Mischung von endothelialen Vorläuferzellen mit mindestens einer zelltherapeutisch einsetzbaren Zellpopulation zur Herstellung einer pharmazeutischen Zusammensetzung, enthaltend Erythropoietin und/oder eine Mischung von endothelialen Vorläuferzellen mit mindestens einer zelltherapeutisch einsetzbaren Zellpopulation für die Regeneration von Gewebe oder Gefäßen in einem menschlichen oder tierischen Körper, wobei dem tierischen oder menschlichen Körper Erythropoietin vor, nach oder gleichzeitig mit der Applikation der Mischung verabreicht wird.
G) Verwendung von Erythropoietin, bevorzugt in Kombination mit einer oder mehreren der Ausführungsformen nach A) bis F), und/oder mindestens ein chemisches, thermisches, mechanisches oder biologisches Agens, insbesondere ein pharmakologischer Wirkstoff, für die Herstellung einer pharmazeutischen Zusammensetzung oder eines Kits, enthaltend Erythropoietin und das mindestens eine chemische, thermische, mechanische oder biologische Agens für die Prophylaxe oder Behandlung von Krankheiten, wobei die pharmazeutische Zusammensetzung oder der Kit geeignet und bestimmt ist für die sequenzielle, zeitlich aufeinanderfolgende oder gleichzeitige Applikation des Erythropoietin mit dem mindestens einen chemischen, thermischen, mechanischen oder biologischen Agens. Die Erfindung betrifft daher auch die Verwendung von Erythropoietin in der vorstehend unter G bezeichneten Weise, wobei es sich bei den mechanischen Agenzien um Endoprothesen, bevorzugt um Implantationskörper für den Zahn, Knochen oder Band/Sehnenersatz handelt. Die Erfindung betrifft auch die Verwendung von Erythropoietin in der vorstehend unter G) bezeichneten Weise, wobei es sich bei den biologischen Agenzien um solide Organe, wie Leber, Niere, Herz, Pankreas oder Haut handelt. Unter biologischen Agenzien werden in diesem Zusammenhang auch Haarimplantate verstanden. In besonders bevorzugter Ausführungsform betrifft die vorliegende Erfindung daher die Verwendung von Erythropoietin zur Herstellung einer pharmazeutischen Zusammensetzung oder eines Kits für die systemische oder lokale Applikation an einen Implantationsort eines biologischen Agens der vorgenannten Art oder einer Endoprothese, insbesondere eines lmplantationskörpers wie zum Beispiel einem Zahn, Zahnersatz, Zahnimplantat, Knochenersatz, Knochenimplantat, zum Beispiel Hüftgelenkprothese, Band/Sehnenersatz, zum Beispiel Kreuzband, wobei das Erythropoietin vor der Implantation des genannten biologischen oder mechanischen Agens, dass heisst zum Beispiel der Endoprothese, systemisch oder lokal appliziert wird, zum Beispiel einige Wochen vor der Implantation und anschließend die Implantation vorgenommen wird. In einer weiteren Ausführungsform ist auch vorgesehen, die Implantation des genannten biologischen oder mechanischen Agens, zum Beispiel der Endoprothese zeitgleich mit dem Einsatz von Erythropoietin vorzunehmen. In einer weiteren Ausführungsform ist vorgesehen, das Erythropoietin nach der Implantation der genannten Endoprothese beziehungsweise mechanischen oder biologischen Agens vorzunehmen. Gemäß dieser Ausführungsformen wird das Gewebe beziehungsweise die Körperstruktur, in die das Implantat, zum Beispiel der Zahn oder die Knochenprothese implantiert wird, mobilisiert beziehungsweise konditioniert, und ermöglicht so ein erheblich besseres und damit auch schnelleres Integrieren, zum Beispiel An- und Einwachsen des biologischen oder mechanischen Agens, zum Beispiel Implantats in die Körperstruktur.
H) Verwendung von Erythropoietin nach einer oder mehreren der Ausführungsformen nach A) bis G), wobei die pharmazeutische Zusammensetzung bei Applikation im menschlichen oder tierischen Körper zu keiner Erhöhung des Hämatokrit-Wertes führt, insbesondere um nicht mehr als 10 % des Hämatokrit-Wertes vor der Applikation des Erythropoietin.
I) Verwendung von Erythropoietin nach einer oder mehreren der Ausführungsformen nach A) bis H) in einer pharmazeutischen Zusammensetzung, wobei das Erythropoietin in niedriger, nicht e-rythropoietisch wirksamer Dosis für die genannten Prophylaxen, Behandlungen oder Therapien geeignet und bestimmt ist, insbesondere in einer Dosis von 0,001 IU/kg Körpergewicht/Woche bis 90, insbesondere 50 IU/kg Körpergewicht/Woche eingesetzt wird.
K) Verwendung von Erythropoietin nach einer oder mehreren der Ausführungsformen nach A) bis I), wobei die Krankheit Hypercholesterinämie, Diabetes mellitus, Insulinresistenz, Endothelvermittelten chronischen Entzündungserkrankungen, Endotheliose einschließlich Retikuloendotheliose, Atherosklerose, altersbedingter Herz-Kreislauf-Erkrankung, ischämischen Erkrankungen der Extremitäten, Präeklampsie, Raynaud-Krankheit, Lebererkrankungen wie Hepatitis, Leberzirrhose, akutes oder chronisches Leberversagen, Knochen- und Knorpelerkrankungen oder -verletzungen, Schleimhauterkrankungen oder -verletzungen, insbesondere im gastrointestinalen Trakt, Morbus Crohn, Colitis ulcerosa, schwangerschaftsinduzierter Hypertonie, chronischer oder akuter Niereninsuffizienz, insbesondere terminaler Niereninsuffizienz, Nierenfunktionseinschränkungen mit glomerulären Filtrationsraten von 30 bis 80 ml/min, Mikroalbuminurie, Proteinurie, Zustände mit erhöhten ADMA-Spiegeln oder Wunden und Folgeerkrankungen davon ist.

Die Erfindung betrifft auch die Herstellung eines Kits, enthaltend Erythropoietin, endotheliale Vorläuferzellen und mindestens eine zelltherapeutisch einsetzbare Zellpopulation, wobei das Erythropoietin vorzugsweise in niedriger Dosierung vorliegt.

Erfindungsgemäß wurde überraschenderweise festgestellt, dass eine Behandlung mit niedrig dosiertem Erythropoietin zur physiologischen Mobilisierung endothelialer Vorläuferzellen führt, wobei die Anzahl zirkulierender endothelialer Vorläuferzellen erhöht und deren Differenzierung induziert wird. Darüber hinaus werden unter bestimmten pathologischen Zuständen auftretende funktionelle Defizite der endothelialen Vorläuferzellen ausgeglichen. Erfindungsgemäß konnte gezeigt werden, dass bei Patienten mit chronischer Nierenerkrankung im Endstadium die Anzahl zirkulierender Stammzellen genauso hoch ist wie bei gesunden Probanden, diese jedoch bei diesen Patienten die Fähigkeit zur Differenzierung zu Endothelzellen über endotheliale Vorläuferzellen verloren haben. So ist bei Patienten mit chronischer Nierenerkrankung die Anzahl von Zellen, die adhäsionsfähig sind und einen endothelialen Zell-Phänotyp zeigen, gegenüber gesunden Probanden deutlich vermindert (de Groot et al; Kidney Int. 2004;66:641-6). Dieser funktionelle Abfall endothelialer Vorläuferzellen lässt sich schon bei mäßiggradiger Einschränkung der Nierenfunktion mit einer glomerulären Filtrationsrate von 30, vorzugsweise 40, bis 80 ml/min finden. Erfindungsgemäß wurde nun festgestellt, dass sowohl nach Behandlung dieser Patienten als auch nierengesunder Patienten und/oder Probanden mit erfindungsgemäß niedrig dosiertem Erythropoietin die Anzahl zirkulierender Stammzellen signifikant um mehr als 50% ansteigt. Dabei erhöht sich insbesondere die Anzahl von Zellen, die einen endothelialen Phänotyp entwickeln, dramatisch. Wie mittels eines funktionellen Zellkulturtestes nachgewiesen, erhöht sich durch die niedrig dosierte Erythropoietin-Behandlung die bei den Patienten mit chronischer Nierenerkrankung mit einer glomerulären Filtrationsrate von 30, vorzugsweise 40, bis 80ml/min beeinträchtigte Fähigkeit der endothelialen Vorläuferzellerzellen zur Adhäsion um das Dreifache, bei nierengesunden Probanden und/oder Patienten um das zwei- bis dreifache. Die Fähigkeit von sich differenzierenden endothelialen Vorläuferzellen beziehungsweise von Endothelzellen zur Adhäsion ist eine der Grundvoraussetzungen zur Ausbildung neuer Gewebe und/oder Gefäße. Erythropoietin kann auf diese Weise die Neovaskularisierung, insbesondere die Vaskulogenese, in Geweben oder Organen, zum Beispiel insbesondere in Nieren, in denen entsprechende vaskulogene oder angiogene Stimuli freigesetzt werden, induzieren.

Erfindungsgemäß kann niedrig dosiertes Erythropoietin zur Stimulierung der physiologischen Mobilisierung endothelialer Vorläuferzellen, der Proliferation endothelialer Vorläuferzellen, der Differenzierung der endothelialen Vorläuferzellen zu Endothelzellen und/oder zur Migration endothelialer Vorläuferzellen in Richtung eines vaskulogenen oder angiogenen Stimulus in einem menschlichen oder tierischen Körper, insbesondere einem adulten Organismus, verwendet werden. Erfindungsgemäß kann niedrig dosiertes Erythropoietin daher in vorteilhafter Weise zur Stimulation der Gefäßneubildung durch Vaskulogenese in Geweben oder Organen eingesetzt werden, in denen pathologische Gefäßveränderungen vorliegen. Aufgrund der Stimulation endothelialer Vorläuferzellen durch niedrig dosiertes Erythropoietin kann darüber hinaus auch die Bildung von Endothelgewebe induziert werden. Erfindungsgemäß kann niedrig dosiertes Erythropoietin daher auch zur Behandlung von Krankheiten des menschlichen oder tierischen Körpers eingesetzt werden, die im Zusammenhang mit einer Dysfunktion von endothelialen Vorläuferzellen und/oder Endothelzellen im Zusammenhang stehen. Patientenpopulationen die eine solche Dysfunktion aufweisen, weisen in der Regel kardiovaskuläre Risikofaktoren wie Hypertonie, Hypercholesterinämie, Insulinresistens, Hyperhomocysteinämie, erhöhte ADMA Spiegel, und einen Endorganschaden wie linksventrikuläre Hypertrophie, Mikroalbuminurie, Proteinurie oder eine glomeruläre Filtrationsrate (GFR) von 30, vorzugsweise 40, bis 80 ml/min auf.

Die Erfindung betrifft auch die Verwendung von niedrig dosiertem Erythropoietin zur Protektion und Regeneration funktionsgefährdeter Gewebe aufgrund des Einwirkens chemischer, thermischer, mechanischer sowie biologischer Agenzien. Die topische Applikation niedrig dosierten Erythropoietins betrifft erfindungsgemäß auch die Prophylaxe und Reduktion bereits bestehender Falten der Haut, insbesondere der Gesichtshaut, sowie den Schutz der Haut als auch die Reduktion von Altersflecken. Dabei kann erfindungsgemäß der Einsatz niedrig dosierten Erythropoietins beziehungsweise eines Abkömmlinges sequenziell, zeitlich aufeinanderfolgend oder gleichzeitig mit einer oder mehreren anderen chemischen, thermischen, mechanischen sowie biologischen Agenzien erfolgen. Erfindungsgemäß kann niedrig dosiertes Erythropoietin in Anpassung an dessen circardianen Rhythmus therapeutisch eingesetzt werden, um so einen maximalen biologische Effekt zu erzielen. Endotheliale Vorläuferzellen werden in einer bevorzugten Ausführungsform erfindungsgemäß zeitgleich mit anderen zelltherapeutischen einsetzbaren Zellpopulationen appliziert unter vorheriger Inkubation mit niedrig dosiertem Erythropoietin in vitro und/oder lokaler sowie systemischer Applikation von niedrig dosiertem Erythropoietin in vivo, um so das Einheilen der zelltherapeutischen Gewebszellen mit suffizienter Anbindung an das Gefäßsystem sicherzustellen.

Im Zusammenhang mit der vorliegenden Erfindung wird unter "Erythropoietin" beziehungsweise "EPO" ein Stoff verstanden, der in entsprechend hohen Dosierung das Wachstum, die Differenzierung und die Reifung von Stammzellen über Erythroblasten zu Erythrocyten steuert.

Erythropoietin ist ein Glycoprotein, welches 166 Aminosäuren, drei Glycosylierungsstellen und ein Molekulargewicht von etwa 34.000 Da aufweist. Im Rahmen einer EPO-induzierten Differenzierung von Erythrocyten-Vorläuferzellen wird die Globinsynthese induziert und die Synthese des Häm-Komplexes sowie die Anzahl der Ferritin-Rezeptoren gesteigert. Dadurch kann die Zelle mehr Eisen aufnehmen und funktionelles Hämoglobin synthetisieren. In den reifen Erythrocyten bindet Hämoglobin den Sauerstoff. Somit nehmen die Erythrocyten beziehungsweise das in ihnen enthaltene Hämoglobin eine Schlüsselrolle für die Sauerstoffversorgung des Organismus ein. Ausgelöst werden diese Vorgänge durch die Wechselwirkung von EPO mit einem entsprechenden Rezeptor auf der Zelloberfläche der Erythrocyten-Vorläuferzellen (Graber und Krantz, Ann. Rev. Med. 29 (1978), 51-56).

Der hier verwendete Begriff "Erythropoietin" umfasst EPO jeglicher Herkunft, insbesondere menschliches oder tierisches EPO. Der hier verwendete Begriff erfasst nicht nur die natürlich vorkommenden, das heißt Wildtyp-Formen von EPO, sondern auch seine Derivate, Analoga, Modifikationen, Muteine, Mutanten oder Sonstiges, solange sie die biologischen Wirkungen von Wildtyp-Erythropoietin zeigen.

Im Zusammenhang mit der vorliegenden Erfindung werden unter "Derivaten" funktionelle Äquivalente oder Abkömmlinge von Erythropoietin verstanden, die unter Beibehaltung der Erythropoietin-Grundstruktur durch Substitution von einem oder mehreren Atomen oder Molekülgruppen beziehungsweise -resten, insbesondere durch Substitution von Zuckerketten wie Ethylenglycol, erhalten werden und/oder deren Aminosäuresequenzen sich von der des natürlicherweise vorkommenden menschlichen oder tierischen Erythropoietin-Proteins an mindestens einer Position unterscheiden, die aber im wesentlichen einen hohen Grad an Homologie auf der Aminosäureebene und vergleichbare biologische Aktivität aufweisen. Derivate des Erythropoietins, wie sie beispielsweise in der vorliegenden Erfindung eingesetzt werden können, sind unter anderem bekannt aus der WO 94/25055, der EP 0 148 605 B1 oder der WO 95/05465.

"Homologie" bedeutet insbesondere eine Sequenzidentität von mindestens 80 %, vorzugsweise mindestens 85 % und besonders bevorzugt mindestens mehr als 90 %, 95 %, 97 % und 99 %. Der dem Fachmann bekannte Ausdruck der "Homologie" bezeichnet somit den Grad der Verwandtschaft zwischen zwei oder mehreren Polypeptid-Molekülen, der durch die Übereinstimmung zwischen den Sequenzen bestimmt wird. Dabei kann eine Übereinstimmung sowohl eine identische Übereinstimmung als auch ein konservativer Aminosäureaustausch bedeuten.

Erfindungsgemäß umfasst der Begriff "Derivat" auch Fusionsproteine, bei denen am N-terminalen Teil beziehungsweise am C-terminalen Teil funktionelle Domänen eines anderen Proteins vorhanden sind. In einer Ausführungsform der Erfindung kann es sich bei diesem anderen Protein beispielsweise um GM-CSF, VEGF, PIGF, ein Statin oder einen anderen Faktor handeln, der eine stimulierende Wirkung auf endotheliale Vorläuferzellen aufweist. In einer weiteren Ausführungsform der Erfindung kann es sich bei dem anderen Protein auch um einen Faktor handeln, der stimulierende Wirkung auf ausdifferenzierte Endothelzellen hat, beispielsweise um Angiogenin, VEGF (vascular endothelial growth factor) oder bFGF (basic fibroblast growth factor). Von bFGF und VEGF ist bekannt, dass dieser Wachstumsfaktor eine starke mitogene und chemotaktische Aktivität auf Endothelzellen ausübt.

Die Unterschiede zwischen einem Erythropoietin-Derivat und nativem Erythropoietin können beispielsweise durch Mutationen, wie zum Beispiel Deletionen, Substitutionen, Insertionen, Anlagerungen, Basenaustausche und/oder Rekombinationen der die Erythropoietin-Aminosäuresequenzen codierenden Nucleotidsequenzen entstanden sein. Erfindungsgemäß bevorzugt wird als Erythropoietin (EPO-) alpha, (EPO-) beta, Aranesp (Darbepoetin alpha) oder Cera (continuous erythropoietin receptor agonist) eingesetzt. Selbstverständlich kann es sich dabei auch um natürlicherweise auftretende Sequenzvariationen handeln, beispielsweise um Sequenzen aus einem anderen Organismus oder um Sequenzen, die auf natürliche Weise mutiert wurden, oder Mutationen, die mit Hilfe üblicher, auf dem Fachgebiet bekannter Mittel, beispielsweise chemische Agenzien und/oder physikalische Agenzien, gezielt in die Erythropoietin codierenden Nucleinsäuresequenzen eingeführt wurden. Im Zusammenhang mit der Erfindung umfasst der Begriff "Derivat" daher auch mutierte Erythropoietin-Moleküle, also Erythropoietin-Muteine.

Erfindungsgemäß können auch Peptid- oder Protein-Analoga von Erythropoietin eingesetzt werden. Im Zusammenhang mit der vorliegenden Erfindung umfasst der Begriff "Analoga" Verbindungen, die keine mit der Erythropoietin-Aminosäuresequenz identische Aminosäuresequenz aufweisen, deren dreidimensionale Struktur jedoch der von Erythropoietin stark ähnelt und die daher eine vergleichbare biologische Aktivität aufweisen. Bei Erythropoietin-Analoga kann es sich beispielsweise um Verbindungen handeln, die die für die Bindung von Erythropoietin an dessen Rezeptoren verantwortlichen A-minosäurereste in geeigneter Konformation enthalten und die daher die essentiellen Oberflächeneigenschaften des Erythropoietin-Bindungsbereiches nachahmen können. Derartige Verbindungen sind beispielsweise in Wrighton et al., Science, 273 (1996), 458, beschrieben. Das erfindungsgemäß eingesetzte EPO kann auf verschiedene Weise hergestellt werden, beispielsweise durch Isolierung aus menschlichem Urin oder aus dem Urin oder Plasma (einschließlich Serum) von an aplastischer Anämie leidenden Patienten (Miyake et al., J.B.C. 252 (1977), 5558). Menschliches EPO kann beispielsweise auch aus Gewebekulturen von menschlichen Nierenkrebszellen (JA-OS 55790/1979), aus menschlichen Lymphoblastenzellen, die die Fähigkeit zur Bildung von menschlichem EPO aufweisen (JA-OS 40411/1982) und aus einer durch Zellfusion einer menschliche Zelllinien erhaltenen Hybridomakultur gewonnen werden. EPO kann auch durch gentechnologische Verfahren hergestellt werden, indem man mittels geeigneter DNA oder RNA, die für die entsprechende Aminosäuresequenz des EPO codiert, gentechnisch das erwünschte Protein herstellt, zum Beispiel in einem Bakterium, einer Hefe, einer Pflanzen-, Tier-, oder Humanzelllinie. Derartige Verfahren sind beispielsweise in der EP 0 148 605 B2 oder der EP 0 205 564 B2 sowie der EP 0 411 678 B1 beschrieben.

Die vorliegende Erfindung betrifft insbesondere die Verwendung von niedrig dosiertem Erythropoietin und/oder Derivaten davon zur Stimulierung der physiologischen Mobilisierung endothelialer Vorläuferzellen, der Proliferation endothelialer Vorläuferzellen, der Differenzierung der endothelialen Vorläuferzellen zu Endothelzellen und/oder zur Migration endothelialer Vorläuferzellen in Richtung eines vaskulogenen oder angiogenen Stimulus in einem menschlichen oder tierischen Körper, insbesondere einem adulten Organismus.

Des weiteren betrifft die Erfindung die sequenzielle Verwendung von niedrig dosiertem Erythropoietin und mindestens einem weiteren geeigneten chemischen, thermischen, mechanischen oder biologischen Agens oder Wirkstoff, insbesondere pharmakologischen Wirkstoff, der die Funktion und Zahl endothialer Vorläuferzellen erhöht, sowie die organprotektive und regenerative Wirkung von niedrig dosiertem Erythropoietin verstärkt.

Des weiteren betrifft die Erfindung daher vorzugsweise die sequenzielle, zeitlich aufeinanderfolgende oder gleichzeitige Gabe von niedrig dosiertem Erythropoietin sowie einer oder mehrerer anderer pharmakologischer Wirkstoffe, z.B. VEGF; GM-CSF, M-CSF, Thrombopoietin, SCF, SDF-1, NGF, PIGF, einem HMG-Co- Reduktase-Inhibitor, einem ACE-Hemmer, einem AT-1 Hemmer und einem NO- Donator, um so die Zahl und Funktion endothelialer Vorläuferzellen zu erhöhen und/oder die Regeneration bzw. Progressionsverlangsamung von Gewebeschäden herbeizuführen. Dabei soll erfindungsgemäß die Sequenz A) Quantitative und qualitative Optimierung von Stammzellen und/oder endotheliale Vorläuferzellen im Knochenmark oder in gewebsstämmigen "Stammzell"-Nischen B) Mobilisierung von Stammzellen und/oder endotheliale Vorläuferzellen aus dem Knochenmark oder anderen "Stammzell"Nischen ins periphere Blut C) Quantitative und qualitative Optimierung von Stammzellen und/oder endotheliale Vorläuferzellen im peripheren Blut und/oder ex vivo unter selektiven Kulturbedingungen, vorzugsweise die Kultur unter hypoxischen Bedingungen mit einem Sauerstoffanteil von 0,1% bis 10% D) Homing von Stammzellen und/oder endothelialen Vorläuferzellen an den Ort der Schädigung E) Adhäsion und Migration von Stammzellen und/oder endotheliale Vorläuferzellen in das Zielgewebe F) Neovaskularisierung durch endotheliale Vorläuferzellen beeinflusst werden.

Die vorliegende Erfindung betrifft also die gleichzeitige wie auch zeitlich versetzte Applikation von endothelialen Vorläuferzellen und einer oder mehreren zelltherapeutisch einsetzbaren Zellpopulationen, insbesondere Hepatozyten, Myocyten, Cardiomyocyten oder Inselzelltransplantaten, nach vorheriger Inkubation mit niedrig dosiertem Erythropoietin in vitro und/oder lokaler sowie systemischer Applikation von niedrig dosiertem Erythropoietin in vivo, welches die Funktion, das Einheilen sowie die Vaskularisierung und den Anschluss an den Blutkreislauf des Empfängers dieser zelltherapeutisch eingesetzten Zellpopulationen verbessert und beschleunigt.

Die vorliegende Erfindung betrifft die Verwendung von Erythropoietin insbesondere in niedriger Dosis oder geeigneter Wirkstoffe zur topischen Anwendung im Sinne der "Schönheitspflege", insbesondere der Prophylaxe bzw. zeitnahen Reduktion von Falten und Fältchen, Stärkung des Bindegewebes, Schutz sowie Straffung der Haut, insbesondere der Gesichtshaut, gegen schädliche Umweltfaktoren und als Make-up Unterlage. Des weiteren wirkt die topische Anwendung von Erythropoietin der Entstehung und Weiterentwicklung von Altersflecken entgegen, verfeinert das Hautbild, sowie unterstützt den Erneuerungsprozeß der Haut, insbesondere die Reepithelialisierung. Darüber hinaus beschleunigt Erythropoietin den Haarwuchs.

Die vorliegende Erfindung betrifft auch die Verwendung von niedrig dosiertem Erythropoietin zur Herstellung einer pharmazeutischen Zusammensetzung geeignet und bestimmt zur Anwendung in Anpassung an dessen circardianen körpereigenen Rhythmus. Die endogene Erythropoietinproduktion besitzt ihre Akrophase (Tagesmaximum) am späten Nachmittag, daher sollte die Gabe von niedrig dosiertes Erythropoietin vorzugsweise vormittags, insbesondere zwischen 6:00 und 10:00 erfolgen, um so einen maximalen biologischen, therapeutischen oder kosmetischen Effekt zu erzielen.

Die vorliegende Erfindung betrifft die Verwendung von niedrig dosiertem Erythropoietin zur Stimulierung der physiologischen Mobilisierung, oder/und zur Proliferation und Differenzierung endothelialer Vorläuferzellen, oder/und zur Stimulierung der Vaskulogenese, oder/und zur Therapie von Krankheiten, die mit einer Dysfunktion endothelialer Vorläuferzellen im Zusammenhang stehen, oder/und zur Herstellung pharmazeutischer Zusammensetzungen zur Behandlung solcher Krankheiten sowie pharmazeutische Zusammensetzungen, die Erythropoietin und andere geeignete Wirkstoffe zur Stimulation endothelialer Vorläuferzellen umfassen, bei Patienten, die a) mindestens eine Dysfunktion endothelialer Vorläuferzellen, und b) mindestens einen kardiovaskulären Risikofaktor wie Hypertonie, Hypercholesterinämie, Insulinresistenz, Hyperhomocysteinämie, erhöhter ADMA-Spiegel und c) mindestens einen Endorganschaden wie linksventrikuläre Hypertrophie, Mikroalbuminurie, kognitive Dysfunktion, Zunahme der Intima-Media-Dicke in der A. carotis, Proteinurie oder eine glomeruläre Filtrationsrate (GFR) von kleiner 80 ml/min, insbesondere 30, bevorzugt 40, bis 80 ml/min aufweisen.

Die Erfindung betrifft auch in bevorzugter Ausführung die sequenzielle, zeitlich aufeinanderfolgende oder gleichzeitige Gabe von niedrig dosiertem Erythropoietin sowie einer oder mehrerer anderer chemischer, thermischer, mechanischer sowie biologischer Agenzien, um so die Zahl und Funktion endothelialer Vorläuferzellen zu erhöhen und/oder die Regeneration bzw. Progressionsverlangsamung von Gewebeschäden herbeizuführen. Hierbei kann es sich bei den mechanischen Agenzien um Endoprothesen, bevorzugt um Implantationskörper, zum Beispiel für den Zahn, Knochen oder Band/Sehnenersatz, handeln. Ferner kann es sich bei den biologischen Agenzien um solide Organe, wie Leber, Niere, Herz, Pankreas oder Haut handeln oder auch um Haarimplantate. Die Erfindung sieht also vor, dass EPO insbesondere in niedrig dosierter Weise verwendet wird, um gleichzeitig, anschließend oder vorab implantierte mechanische Agenzien wie Endoprothesen oder biologische Agenzien besser, schneller und effizienter in die umgebende Körperstruktur einwachsen, beziehungsweise integrieren zu lassen. Die Erfindung betrifft daher auch die Verwendung von Erythropoietin zur -herstellung einer pharmazeutischen Zusammensetzung oder eines Kits für die Verbesserung, insbesondere zur Förderung und/oder Beschleunigung, der Integration von biologischen Agenzien oder Endoprothesen in umgebende Körperstrukturen, insbesondere von Zähnen, Zahnersatz, Zahnimplantaten oder sonstigen Endoprothesen, wie Knochenersatz, Knochenimplantaten, insbesondere Hüftgelenkprothesen oder Band/Sehnenersatz, wie z.B. Kreuzbänder. Gegebenenfalls kann dabei vorgesehen sein, das Erythropoietin zusammen mit zelltherapeutisch geeigneten Zellpopulationen und/oder endothelialen Vorläuferzellen zu verwenden. In der vorgenannten Verwendung von Erythropoietin zur Herstellung einer pharmazeutischen Zusammensetzung oder eines Kits für die Verbesserung, insbesondere Förderung und/oder Beschleunigung der Integration von biologischen oder mechanischen Agenzien in Zielstrukturen, insbesondere Zielgewebe, Zielknochen oder Zielknorpel eines Patienten, kann in einer weiteren bevorzugten Ausführungsform vorgesehen sein, dass die einzusetzenden mechanischen Agenzien zum Beispiel aus Stahl, Keramik, Kunststoff oder einem sonstigen Matrix-Material hergestellt sind. Darüber hinaus kann vorgesehen sein, dass in der genannten Verwendung als zelltherapeutisch geeignete Zellpopulationen, Osteoblasten, Zellen mit osteogenem Potential, Thrombozyten, Blutzellen oder ähnlich eingesetzt werden. In weiterer bevorzugter Ausführungsform kann vorgesehen sein, dass insbesondere das einzusetzende mechanische Agens zusammen mit organischem Klebstoff, zum Beispiel einem Fibrinkleber in der pharmazeutischen Zusammensetzung oder dem pharmazeutischen Kit enthalten sind.

Im Zusammenhang mit der vorliegenden Erfindung werden unter "endothelialen Vorläuferzellen" (endotheliale Progenitorzellen; EPC) im Blutkreislauf zirkulierende Zellen verstanden, die die Fähigkeit zur Differenzierung zu Endothelzellen besitzen. Bei den während der Embryonalentwicklung vorkommenden endothelialen Vorläuferzellen handelt es sich um Angioblasten. Bei den im adulten Organismus vorkommenden endothelialen Vorläuferzellen handelt es sich um Angioblasten-ähnliche Zellen, die aus mononucleären Zellen, insbesondere CD34-CD14+-Monozyten, und/oder CD34+-Stammzellen, die aus peripherem Blut isoliert wurden, gewonnen werden können.

Im Zusammenhang mit der vorliegenden Erfindung wird unter "Mobilisierung" oder "physiologischer Mobilisierung" der Prozess des Aktivierens von Stammzellen und/oder Progenitorzellen aus dem Knochenmark oder aus alternativen "Stammzell"-Nischen durch Wachstumsfaktoren verstanden, wobei die Stammzellen beziehungsweise Progenitorzellen in den Blutkreislauf, insbesondere ins periphere Blut, gelangen.

Im Zusammenhang mit der vorliegenden Erfindung wird unter "Proliferation" die Fähigkeit von Zellen zur Vergrößerung und zur anschließenden Teilung in zwei oder mehr Tochterzellen verstanden. Die EPO-vermittelte Stimulierung endothelialer Vorläuferzellen betrifft somit insbesondere die Anzahl und damit das Teilungsverhalten endothelialer Vorläuferzellen.

Im Zusammenhang mit der vorliegenden Erfindung wird unter "Differenzierung" endothelialer Vorläuferzellen die Entwicklung von aus dem Knochenmark oder gewebeständigen Nischen stammenden mononukleären Zellen über endotheliale Vorläuferzellen zu Endothelzellen verstanden. Unter "Endothelzellen" werden die Zellen verstanden, die das Endothel, das heißt die einschichtige zeitige Auskleidung von Gefäßen und serösen Höhlen, bilden. Endothelzellen sind dadurch charakterisiert, dass sie gefäßaktive Substanzen, beispielsweise gefäßerweiternde Substanzen wie EDRF (endothelial derived relaxing factor) oder konstringierenden Substanzen wie Endothelin, Faktoren zur Hemmung oder Aktivierung der Blutgerinnung und Faktoren zur Regulation der Gefäßpermeabilität freisetzen. Endothelzellen synthetisieren auch Komponenten des subendothelialen Bindegewebes, insbesondere Collagene vom Typ IV und V, Zellhaftproteine wie Laminin, Fibronektin und Thrombospondin, Wachstumsfaktoren, beispielsweise für glatte Muskelzellen, und Faktoren zur Gefäßneubildung.

Im Zusammenhang mit der vorliegenden Erfindung wird unter "Migration" der endothelialen Vorläuferzellen verstanden, dass die im Blutkreislauf befindlichen endothelialen Vorläuferzellen in Richtung eines vaskulogenen oder angiogenen Stimulus wandern und sich im Bereich des vaskulogenen oder angiogenen Stimulus konzentrieren. Unter einem "vaskulogenen Stimulus" wird ein chemischer Reiz in einem Gewebe oder Blutgefäß eines menschlichen oder tierischen Körpers verstanden, der spezifisch auf endotheliale Vorläuferzellen wirkt und deren Wanderung zu der Stelle des Körpers bewirkt, von der der chemische Reiz ausgeht. Durch den vaskulogenen Stimulus wird auf diese Weise der Prozess der Vaskulogenese induziert. Unter einem "angiogenen Stimulus" wird ein chemischer Reiz in einem Gewebe oder Blutgefäß eines menschlichen oder tierischen Körpers verstanden, der spezifisch auf ausdifferenzierte Endothelzellen wirkt und deren Migration zu der Stelle des Körpers bewirkt, von der der chemische Reiz ausgeht. Der angiogene Stimulus bewirkt auf diese Weise eine Induzierung von Angiogenese.

In einer weiteren Ausführungsform der Erfindung ist die Verwendung von niedrig dosiertem Erythropoietin und/oder Derivaten davon zur Erhöhung der Adhäsionsfähigkeit sich differenzierender endothelialer Vorläuferzellen vorgesehen. Erfindungsgemäß wird Erythropoietin insbesondere verwendet, um die Fähigkeit endothelialer Vorläuferzellen zur Adhäsion, das heißt zur Zell-Zell-Adhäsion zu verbessern. Die Adhäsion von sich differenzierenden endothelialen Vorläuferzellen beziehungsweise ausdifferenzierten Endothelzellen ist eine der Grundvoraussetzungen zur Bildung neuer Gefäße oder eines neuen Endothelgewebes. Die Zelladhäsion wird durch Proteinmoleküle vermittelt.

Die vorliegende Erfindung betrifft auch die Verwendung von niedrig dosiertem Erythropoietin zur Stimulation der Gefäßneubildung, insbesondere die Stimulation von Vaskulogenese. Im Zusammenhang mit der vorliegenden Erfindung wird unter "Vaskulogenese" die Gefäßneubildung aus sich in situ differenzierenden endothelialen Vorläuferzellen verstanden. Erfindungsgemäß wird durch den Einsatz von niedrig dosiertem Erythropoietin also erreicht, dass sich endotheliale Vorläuferzellen in verstärktem Maße an einer Gefäßneubildung beziehungsweise an einer lokalen Gefäßneubildung zur Wiederstellung geschädigter Gefäßbereiche beteiligen. Erfindungsgemäß ist also vorgesehen, dass die Verwendung von niedrig dosiertem Erythropoietin und/oder dessen Derivaten die Bildung neuer Blutgefäße und/oder den Ersatz geschädigter Gefäßbereiche durch lokale Blutgefäßneubildung fördert.

In einer weiteren Ausführungsform der Erfindung ist die Verwendung von niedrig dosiertem Erythropoietin und/oder Derivaten davon zur Stimulation endothelialer Vorläuferzellen zur Bildung von Endothelgewebe vorgesehen.

In einer besonders bevorzugten Ausführungsform der Erfindung ist die Verwendung von niedrig dosiertem Erythropoietin und/oder Derivaten davon zur Therapie von Krankheitszuständen oder Krankheiten des menschlichen oder tierischen Körpers, die im Zusammenhang mit einer Dysfunktion von endothelialen Vorläuferzellen stehen, oder von Folgerkrankungen davon vorgesehen.

Im Zusammenhang mit der vorliegenden Erfindung werden unter "Krankheiten", "Krankheitszuständen" oder "Erkrankungen" Störungen der Lebensvorgänge in Organen oder im gesamten Organismus mit der Folge von subjektiv empfundenen beziehungsweise objektiv feststellbaren körperlichen, seelischen beziehungsweise geistigen Veränderungen verstanden. Erfindungsgemäß handelt es sich insbesondere um Krankheiten, die mit einer Dysfunktion von endothelialen Vorläuferzellen im Zusammenhang stehen, das heißt Krankheiten, die entweder die Folge einer derartigen Dysfunktion dieser Zellen sind oder die durch diese Zellen vermittelt werden. Des weiteren werden erfindungsgemäß unter "Krankheiten", "Krankheitszuständen" oder "Erkrankungen" Störungen der Lebensvorgänge in Organen oder im gesamten Organismus verstanden, die sich durch die Gabe von niedrig dosierten Erythropoietin oder geeigneten Wirkstoffen in ihrer Progredienz aufhalten oder insbesondere verlangsamen lassen. Unter "Folgerkrankungen" werden Sekundärkrankheiten verstanden, das heißt eine zu einem primären Krankheitsbild hinzutretende zweite Erkrankung.

Im Zusammenhang mit der vorliegenden Erfindung wird unter einer "Dysfunktion" von endothelialen Vorläuferzellen eine Störung wesentlicher Zellfunktionen wie Stoffwechselleistungen, Reizbeantwortung, Motilität, Teilungsverhalten oder Differenzierungsverhalten dieser Zellen verstanden. Eine Dysfunktion von endothelialen Vorläuferzellen kann beispielsweise darin bestehen, dass diese Zellen nicht oder nur in ungenügendem Maße proliferieren. Da durch die Verwendung von Erythropoietin die Proliferation endothelialer Vorläuferzellen stimuliert wird, kann dadurch das defizitäre Teilungsverhalten sowohl von endothelialen Vorläuferzellen als auch von bereits ausdifferenzierten Endothelzellen ausgeglichen und die Anzahl von endothelialer Vorläuferzellen beziehungsweise Endothelzellen erhöht werden. Eine Dysfunktion endothelialer Vorläuferzellen kann beispielsweise in der gestörten Fähigkeit dieser Zellen zur Differenzierung zu Endothelzellen bestehen. Die Dysfunktion von endothelialen Vorläuferzellen kann auch bedingt sein durch deren gestörte Fähigkeit zur Adhäsion und/oder deren gestörte Fähigkeit zur Migration in Richtung eines angiogenen oder vaskulogenen Stimulus. Solche Dysfunktionen von endothelialen Vorläuferzellen können dazu führen, dass beispielsweise die Neubildung von Endothelgewebe und/oder Vaskulogenese beeinträchtigt oder verhindert wird. Eine Dysfunktion von endothelialen Vorläuferzellen kann auch pathogen bedingt sein, beispielsweise durch Hypertonie, Hyperlipoproteinämie, erhöhten ADMA- Blutspiegeln, Urämie oder Diabetes. Die Dysfunktion von endothelialen Vorläuferzellen kann sich beispielsweise in einer verminderten Produktion von NO (=EDRF) durch NO-Synthasen (NOS) aus L-Arginin, erhöhter Endothelin-Produktion und/oder in einer verstärkten Produktion von Adhäsionsmolekülen wie ICAM-1 und VCAM-1 äußern.

Erfindungsgemäß handelt es sich bei den Krankheiten, die mit einer Dysfunktion von endothelialen Vorläuferzellen im Zusammenhang stehen, insbesondere um Hypercholesterinämie, Diabetes mellitus, Insulinresistenz, Endothel-vermittelte chronische Entzündungserkrankungen wie Gefäßentzündungen, Endotheliose einschließlich Retikuloendotheliose, Atherosklerose, altersbedingte Herz-Kreislauf-Erkrankung, ischämische Erkrankungen der Extremitäten, Raynaud-Krankheit, Präeklampsie, schwangerschaftsinduzierte Hypertonie, chronische oder akute Niereninsuffizienz, insbesondere terminale Niereninsuffizienz, Nierenfunktionseinschränkungen mit glomerulären Filtrationsraten von 30 bis 80 ml/min, bevorzugt 40 bis 80 ml/min, Mikroalbuminurie, Proteinurie, erhöhten ADMA-Spiegeln Wundheilung sowie Folgeerkrankungen davon.

Eine "Hypercholesterinämie" ist durch erhöhte Konzentrationen von Cholesterin im Blut gekennzeichnet. Die weitaus häufigste Form der primären Hypercholesterinämien ist die polygene Hypercholesterinämie. Sekundäre Hypercholsterinämien treten häufig bei Diabetes mellitus, nephrotischem Syndrom, Hypothyreose und Lebererkrankungen auf.

Unter "Diabetes mellitus" werden verschiedene Formen von Glucose-Stoffwechselstörungen mit unterschiedlicher Ätiologie und Symptomatik zusammengefasst. Für die Entstehung vaskulär bedingter diabetischer Komplikationen ist insbesondere das AGE-RAGE-System verantwortlich. AGEs (Advanced Glycation Endproducts) entstehen über eine Reihe komplexer Reaktionen nach lang andauernder Exposition von Proteinen oder Lipiden mit reduzierenden Zuckern, beispielsweise Glucose. Die Bildung von AGEs findet während des normalen Alterungsprozesses sowie in gesteigertem Maße bei Diabetes mellitus und Alzheimer-Krankheit statt. Über die Bindung von AGEs kommt es zu oxidativem Stress, zur Aktivierung des Transkriptionsfaktors NF-κB und damit zu einer Störung der endothelialen Homöostase.

Unter "Insulinresistenz" versteht man die gestörte Signalübertragung in verschiedenen Körperzellen, die die physiologische Signalkaskade des Insulins ignorieren und betroffene Patienten so keinen normalen Glukosestoffwelchsel aufweisen.

"Endothel-vermittelte chronische Entzündungserkrankungen" sind Erkrankungen oder Zustände eines menschlichen oder tierischen Körpers, die auf einer Abwehrreaktion des Organismus und seiner Gewebe auf schädigende Reize beruhen, wobei bestimmte Signalmoleküle die Eigenschaften von Endothelzellen verändern, so dass im Zusammenspiel mit der Aktivierung anderer Zelltypen Leukozyten an den Endothelzellen haften bleiben, schließlich in das Gewebe eindringen und dort eine Entzündung auslösen. Ein Beispiel für eine Endothel-vermittelte Entzündung ist die leukozytische Vaskulitis. Eine zentrale Rolle bei der Aktivierung eines Endothel-vermittelten entzündlichen Geschehens spielt der Transkriptionsfaktor NF-κB. Ein anderes System, dass zur Entstehung Endothelzell-vermittelter chronischer Entzündungen führt, ist das AGE-RAGE-System.

Unter "Endotheliose" werden degenerative und proliferative Endothelveränderungen bei der nicht-thrombopenischen Purpura verstanden. Unter "Retikuloendotheliose" werden Krankheiten des retikulohistiozytären Systems, wie Retikulum, Retikulose, Retikulohistiozytose und Hand-Schüller-Christian-Krankheit verstanden.

Unter "Raynaud-Krankheit" werden durch Vasokonstriktion, das heißt Gefäßkrämpfe, bedingte, anfallartig auftretende Ischämiezustände meist an den Arterien der Finger verstanden. Die primäre Raynaud-Krankheit ist eine rein funktionelle Störung der kleinen versorgenden Gefäße der Akren, während der sekundären Rynaud-Krankheit eine andere Krankheit zugrunde liegt, beispielsweise eine Gefäßentzündung.

Die "Präeklampsie ist eine Endothel- und Gefässkrankheit des mütterlichen Organismus und scheint die Auswirkung von endotheliotropen Substanzen aus der Plazenta zu sein. Die Präeklampsie ist eine Multisystem-Erkrankung, die zu Funktionsstörungen zahlreicher Organe führen und sich in vielfältigen Symptomen äußern kann. Die für die Erkrankung typischen Durchblutungsstörungen sind die Folge eines erhöhten Gefäßwiderstandes, der lokal unterschiedlich ausgeprägt sein kann. Für die Präeklampsie gilt als gesichert, dass eine endotheliale Dysfunktion zentraler Bestandteil der Pathogenese ist.

Unter "Niereninsuffizienz" wird im Zusammenhang mit der vorliegenden Erfindung die eingeschränkte Fähigkeit der Nieren zur Ausscheidung harnpflichtiger Substanzen verstanden, wobei in fortgeschrittenen Stadien auch die Regulationsfähigkeit des Elektrolyt-, Wasser- und Säure-Basen-Haushalts verloren geht. Die terminale Niereninsuffizienz ist durch einen Zusammenbruch der exkretorischen und endokrinen Nierenfunktion gekennzeichnet.

Bei der Niereninsuffizienz kann es sich erfindungsgemäß um akute Niereninsuffizienz handeln, die auch als akutes Nierenversagen, Schockniere oder Schockanurie bezeichnet wird. Akute Niereninsuffizienz ist durch einen plötzlichen partiellen oder totalen Verlust der exkretorischen Nierenfunktion als Folge eines meist reversiblen Nierenschadens gekennzeichnet. Ursache können eine Minderperfusion durch Hypovolämie, Hypotonie und Dehydratation in Folge von Blutverlusten (Polytrauma, gastrointestinale oder postpartale Blutung, große operative Eingriffe an Herz, Gefäßen, Abdomen oder Prostata), Schock (Myokardinfarkt, Embolie), schwerer Infektionen (Sepsis, Peritonitis, Cholezystitis), Hämolyse (hämolytisch-urämisches Syndrom, paroxysmale Hämoglobinurie, Transfusionszwischenfall), Myolyse (Crush-Syndrom, Rhabdomyolyse, Myositis, Verbrennungen), Wasser- und Elektrolytverlusten (massives Erbrechen, Durchfälle, exzessives Schwitzen, Ileus, akute Pankreatidis). Weitere Ursachen können Nephrotoxine wie exogene Toxine, beispielsweise Anilin, Glykolverbindungen, Methanol und ähnliche, oder endogene Toxine, beispielsweise Myoglobin und Oxalate, sein. Weitere Ursachen für akute Niereninsuffizienz sind Nierenkrankheiten, beispielsweise entzündliche Nephropatien oder Abstoßungsreaktionen nach Nierentransplantation, sein. Akute Niereninsuffizienz kann auch durch eine Harnstauung in Folge von Harnabflussbehinderungen verursacht werden. Die erfindungsgemäße Behandlung akuter Niereninsuffizienz mit, vorzugsweise niedrig dosiertem, Erythropoietin führt erfindungsgemäß zur Verhinderung oder zumindest zur Verminderung der Progression der akuten Niereninsuffizienz.

Bei der Niereninsuffizienz kann es sich erfindungsgemäß auch um eine chronische Niereninsuffizienz handeln. Ursachen der chronischen Niereninsuffizienz sind vaskuläre, glomeruläre und tubulointerstitielle Nierenerkrankungen, Infektionen sowie angeborene oder erworbene Strukturdefekte. Ursachen der chronischen Niereninsuffizienz sind unter anderem chronische Glomerulopathie, chronische Pyelonephritis, Analgetika-Nephropathie, obstruktive Uropathie sowie Arterio- und Arteriolosklerose. Die chronische Niereninsuffizienz geht terminal in die Urämie über. Die erfindungsgemäße Behandlung von chronischer Niereninsuffizienz mit niedrig dosiertem Erythropoietin führt erfindungsgemäß zur Verminderung der Progression von chronischer Niereninsuffizienz.

Insbesondere betrifft die Erfindung daher die Verwendung von vorzugsweise niedrig dosiertem EPO zur Herstellung eines Arzneimittels zur Verhinderung, Verminderung oder Verlangsamung der Schädigung von Nierengewebe und/oder zur Regeneration von geschädigtem Nierengewebe bei akuter oder chronischer Niereninsuffizienz.

Unter Nierenfunktionseinschränkung werden erfindungsgemäß Zustände verstanden, bei denen die glomeruläre Filtrationsrate 80ml/min bereits unterschritten hat. Die Nierenfunktionseinschränkung bezieht sich also auf die Frühphase glomerulärer, tubulointerstitieller sowie vaskulärer Nierenerkrankungen. Die erfindungsgemäße Behandlung von Nierenfunktionseinschränkungen mit niedrig dosiertem Erythropoietin führt erfindungsgemäß zur Verminderung der Progression sowie zur Regeneration des einsetzenden Nierengewebe- und/oder -funktionsschadens.

Im Zusammenhang mit der vorliegenden Erfindung wird unter "Mikroalbuminurie" ein Krankheitsbild verstanden, bei dem betroffene Patienten eine unphysiologische Ausscheidung von Albumin im Urin von mehr als 30mg/24h aufweisen. Diese vermehrte Ausscheidung von Albumin geht als ein Frühzeichen mit einer einsetzenden Nierenfunktionsverschlechterung einher und ist Folge erster pathologischer Umbauvorgänge in der Niere, begleitet von strukturellen Veränderungen der Nierenarchitektur.

Im Zusammenhang mit der vorliegenden Erfindung wird unter "Proteinurie" ein Krankheitsbild verstanden, bei dem betroffene Patienten eine unphysiologische Ausscheidung von Proteinen von mehr als 150mg/24h aufweisen. Diese vermehrte Ausscheidung von Protein über den Harn (>150mg/24h) gilt als pathologisch und bedarf der weiteren medizinischen Abklärung und Therapie.

Im Zusammenhang mit der vorliegenden Erfindung wird unter "hohen ADMA Werten" ein Krankheitsbild verstanden, bei dem betroffene Patienten eine unphysiologische hohe ADMA Blutkonzentratioenen von mehr als 1,3 µmol/l aufweisen. Diese erhöhte ADMA Konzentration geht mit einer endothelialen Dysfunktion einher und ist Folge metabolischer Dysfunktionen im Abbau bzw. Ausscheidungsvorgängen diesem Moleküls.

Im Zusammenhang mit der vorliegenden Erfindung werden unter "Wundheilung" die physiologischen Vorgänge zur Regeneration zerstörten Gewebes beziehungsweise zum Verschluss einer Wunde, insbesondere die Neubildung von Bindegewebe und Kapillaren verstanden. Bei der Wundheilung kann es sich um eine primäre Wundheilung (Sanatio per primam intentionem) handeln, die durch einen raschen und komplikationslosen Verschluss und weitgehende Restitutio ad integrum infolge minimaler Bindegewebeneubildung zwischen den gut durchbluteten und gegebenenfalls adaptierten Wundrändern einer sauberen Wunde gekennzeichnet ist. Bei Wunden mit weiter auseinanderliegenden, insbesondere gequetschten oder nekrotischen Wundrändern beziehungsweise Wundinfektionen erfolgt eine verzögerte sekundäre Wundheilung (Sanatio per secundam intentionem), bei der es infolge einer (a)bakteriellen Entzündung zur Auffüllung des Gewebedefektes mit Granulationsgewebe und ausgedehnteren Bildung von Narbengewebe kommt. Die Epithelisierung vom Rand her bildet den Abschluss der Wundheilung. Die Wundheilung untergliedert sich in die drei Phasen, nämlich Latenzphase, Proliferationsphase und Reparationsphase. Die Latenzphase wiederum untergliedert sich in die exsudative Phase mit Schorfbildung, insbesondere in den ersten Stunden nach Entstehung der Wunde, und die resorptive Phase mit kataboler Autolyse, die sich über einen Zeitraum von ein bis drei Tagen nach Entstehung der Wunde erstreckt. Die Proliferationsphase ist durch eine anabole Reparation mit Bildung von Collagen durch Fibroblasten gekennzeichnet und tritt am vierten bis siebten Tag nach Entstehung der Wunde auf. Ab dem achten Tag nach Entstehung der Wunde tritt die Reparationsphase auf, die durch Umwandlung des Granulationsgewebes in eine Narbe gekennzeichnet ist.

Unter einer "Wunde" wird im Zusammenhang mit der vorliegenden Erfindung eine Unterbrechung des Zusammenhangs von Körpergeweben mit oder ohne Substanzverlust verstanden, die durch mechanische Verletzung oder physikalisch bedingte Zellschädigung verursacht wird. Im Sinne der vorliegenden Erfindung wird eine Wunde auch als Krankheit bezeichnet. Wundformen sind mechanische Wunden, thermische Wunden, chemische Wunden, strahlenbedingte Wunden und krankheitsbedingte Wunden. Mechanische Wunden entstehen durch äußere Gewalteinwirkung und treten vor allem als Schnitt- und Stichwunden, Quetsch-, Platz-, Riss- und Schürfwunden, Kratz- und Bisswunden und Schusswunden auf. Thermische Wunden entstehen durch Einwirkung von Hitze oder Kälte. Chemische Wunden entstehen insbesondere durch Verätzung mit Säuren oder Laugen. Strahlenbedingte Wunden entstehen beispielsweise durch Einwirkung aktinischer und ionisierender Strahlung. Krankheitsbedingt auftretende Wunden sind insbesondere stauungsbedingte Wunden, traumatische Wunden, diabetische Wunden etc.. Erfindungsgemäß ist insbesondere vorgesehen, dass zur Wundheilung niedrig dosiertes Erythropoietin vorzugsweise topisch oder intravenös appliziert wird.

Die vorliegende Erfindung betrifft die Verwendung von niedrig dosiertem Erythropoietin zur Therapie von Hypercholesterinämie, Diabetes mellitus, Insulinresistenz, Endothel-vermittelten chronischen Entzündungserkrankungen, Endotheliose einschließlich Retikuloendotheliose, Atherosklerose, altersbedingten Herz-Kreislauf-Erkrankungen, ischämischen Erkrankungen der Extremitäten, Präeklampsie, Raynaud-Krankheit, Lebererkrankungen wie Hepatitis, Leberzirrhose, akutes oder chronisches Leberversagen, Knochen- und Knorpelerkrankungen oder -verletzungen, Schleimhauterkrankungen oder - verletzungen, insbesondere im gastrointestinalen Trakt, Morbus Crohn, Colitis ulcerosa, schwangerschaftsinduzierter Hypertonie, chronischer oder akuter Niereninsuffizienz, insbesondere terminaler Niereninsuffizienz, Nierenfunktionseinschränkungen mit glomerulären Filtrationsraten < 80ml/min, insbesondere 30 bis 80 ml/min, bevorzugt 40 bis 80 ml/min, Mikroalbuminurie, Proteinurie, erhöhten ADMA- Spiegeln, oder Wunden und Folgeerkrankungen davon.

Erfindungsgemäß ist vorgesehen, dass Erythropoietin in einer therapeutisch wirksamen Dosis an einen Patienten verabreicht wird, die ausreicht, den Zustand einer vorgenannten Krankheit, insbesondere einer Krankheit, die im Zusammenhang mit einer Dysfunktion von endothelialen Vorläuferzellen steht, zu heilen oder diesem Zustand vorzubeugen, die Progression einer solchen Krankheit zu stoppen und/oder die Symptome einer solchen Krankheit zu lindern. Die an einen Patienten zu verabreichende Dosis hängt von vielen Faktoren, beispielsweise dem Alter, Körpergewicht und Geschlecht des Patienten, der Schwere der Erkrankungen usw. ab.

### "Erfindungsgemäße Dosierung von EPO"

Erfindungsgemäß bevorzugt wird Erythropoietin in allen Verwendungen, Verfahren und Zusammensetzungen der vorliegenden Lehre in geringen Mengen, die unterhalb der bekanntermaßen eingesetzten Mengen für die Therapie der renalen Anämie liegen, verwendet, wobei im Sinne der vorliegenden Lehre unter einer geringen oder niedrigen Dosis oder Dosierung, insbesondere in vivo, d.h. pro Patient, EPO-Dosen von 1 bis 2000, vorzugsweise 20 bis 2000, Einheiten (IU; International Units)/Woche, vorzugsweise Dosen von 20 bis 1500 IU/Woche, insbesondere Dosen von 20 bis 1000 IU/Woche, insbesondere Dosen von 20 bis 950 IU/Woche, insbesondere Dosen von 20 bis 900 IU/Woche, insbesondere Dosen von 20 bis 850 IU/Woche, insbesondere Dosen von 20 bis 800 IU/Woche, insbesondere Dosen von 20 bis 750 IU/Woche, insbesondere Dosen von 20 bis 700 IU/Woche, insbesondere Dosen von 20 bis 650 IU/Woche, insbesondere Dosen von 20 bis 600 IU/Woche, insbesondere Dosen von 20 bis 550 IU/Woche, insbesondere Dosen von 20 bis 500 IU/Woche, insbesondere Dosen von 20 bis 450 IU/Woche, insbesondere Dosen von 20 bis 400 IU/Woche, insbesondere Dosen von 20 bis 350 IU/Woche, insbesondere Dosen von 20 bis 300 IU/Woche, insbesondere Dosen von 20 bis 250 IU/Woche, insbesondere Dosen von 20 bis 200 IU/Woche, insbesondere Dosen von 20 bis 150 IU/Woche je nach Schwere der Erkrankung und in Abhängigkeit von der Nierenfunktion, verstanden werden. Erfindungsgemäß ist auch vorgesehen Dosen von 1 bis 450, vorzugsweise 1 bis 9 IU/Woche einzusetzen. Bei allen vorstehenden erfindungsgemäß vorgesehenen Dosen, zum Beispiel von 1 bis 2000 Einheiten (IU)/Woche und pro Patient, insbesondere zum Beispiel von 500 bis 2000 IU/Woche und pro Patient, handelt es sich um subpolycythämische Dosen, das heißt Dosen, die nicht zu einer Erhöhung des Hämatokritwertes führen, insbesondere gegenüber dem Hämatokrit-Wert vor der Behandlung mit EPO zu keinem Anstieg des Hämatokrit-Wertes von mehr als 10 %, insbesondere 5 %, bevorzugt 2 % führen. Die erfindungsgemäß vorgesehenen subpolycythämischen Dosen entsprechen wöchentlichen Dosen von etwa 1 bis 90 Einheiten (IU) EPO/kg Körpergewicht, insbesondere 1 bis 45, insbesondere 1 bis 30 IU EPO/kg Körpergewicht, insbesondere 1 bis 20 IU EPO/kg Körpergewicht, insbesondere 1 bis 15 IU EPO/kg Körpergewicht, insbesondere 1 bis 10 IU EPO/kg Körpergewicht, insbesondere 1 bis 4 IU EPO/kg Körpergewicht, oder einer vergleichbaren Wochendosis Aranesp von 0,001 bis 0,4 µg/kg Körpergewicht, 0,001 bis 0,3 µg/kg Körpergewicht, 0,001 bis 0,25 µg/kg Körpergewicht, 0,001 bis 0,2 µg/kg Körpergewicht, 0,001 bis 0,15 µg/kg Körpergewicht, 0,001 bis 0,1 µg/kg Körpergewicht, 0,001 bis 0,09 µg/kg Körpergewicht, 0,001 bis 0,08 µg/kg Körpergewicht, 0,001 bis 0,07 µg/kg Körpergewicht, 0,001 bis 0,06 µg/kg Körpergewicht, 0,001 bis 0,05 µg/kg Körpergewicht, 0,001 bis 0,04 µg/kg Körpergewicht, 0,001 bis 0,03 µg/kg Körpergewicht, 0,001 bis 0,02 µg/kg Körpergewicht, 0,001 bis 0,01 µg/kg Körpergewicht, 0,001 bis 0,009 µg/kg Körpergewicht, 0,001 bis 0,008 µg/kg Körpergewicht, 0,001 bis 0,007 µg/kg Körpergewicht, 0,001 bis 0,006 µg/kg Körpergewicht, 0,001 bis 0,005 µg/kg Körpergewicht, 0,001 bis 0,004 µg/kg Körpergewicht, 0,001 bis 0,003 µg/kg Körpergewicht, 0,001 bis 0,002 µg/kg Körpergewicht. Bei Aranesp handelt es sich um ein doppelt PEG-yliertes EPO.

Erfindungsgemäß besonders bevorzugt wird Erythropoietin in allen Verwendungen, Verfahren und Zusammensetzungen der vorliegenden Lehre in geringen Mengen, die unterhalb der bekanntermaßen eingesetzten Mengen für die Therapie der renalen Anämie liegen, verwendet, wobei im Sinne der vorliegenden Lehre unter einer geringen oder niedrigen Dosis oder Dosierung, insbesondere in vivo, d.h. pro Patient, EPO-Dosen von 0,001 bis 90, vorzugsweise von 0,001 bis 50 Einheiten (IU; International Units) je Kilogramm Körpergewicht und Woche, insbesondere Dosen von 0,05 bis 45 IU/kg/Woche, insbesondere Dosen von 0,05 bis 40 IU/kg/Woche, insbesondere Dosen von 0,05 bis 35 IU/kg/Woche, insbesondere Dosen von 0,05 bis 33 IU/kg/Woche, insbesondere Dosen von 0,05 bis 31 IU/kg/Woche, insbesondere Dosen von von 0,05 bis 29 IU/kg/Woche, insbesondere Dosen von 0,05 bis 27 IU/kg/Woche, insbesondere Dosen von 0,05 bis 25 IU/kg/Woche, insbesondere Dosen von 0,05 bis 23 IU/kg/Woche, insbesondere Dosen von 0,05 bis 21 IU/kg/Woche, insbesondere Dosen von 0,05 bis 20 IU/kg/Woche, insbesondere Dosen von 0,05 bis 19 IU/kg/Woche, insbesondere Dosen von 0,05 bis 17 IU/kg/Woche, insbesondere Dosen von 0,05 bis 15 IU/kg/Woche, insbesondere Dosen von 0,05 bis 13 IU/kg/Woche, insbesondere Dosen von 0,05 bis 11 IU/kg/Woche, insbesondere Dosen von 0,05 bis 9 IU/kg/Woche, insbesondere Dosen von 0,05 bis 7 IU/kg/Woche, insbesondere Dosen von 0,05 bis 5 IU/kg/Woche, insbesondere Dosen von 0,05 bis 3 IU/kg/Woche, insbesondere Dosen von 0,05 bis 1 IU/kg/Woche je nach Schwere der Erkrankung und in Abhängigkeit von der Nierenfunktion, verstanden werden. Erfindungsgemäß ist auch vorgesehen, Dosen von 0,001 bis 20, vorzugsweise 0,05 bis 10 IU/kg/Woche einzusetzen. Bei allen vorstehenden erfindungsgemäß vorgesehenen Dosen, zum Beispiel von 0,01 bis 90 Einheiten (IU)/kg/Woche und pro Patient, insbesondere zum Beispiel von 0,01 bis 50 Einheiten IU/kg/Woche und pro Patient, handelt es sich um subpolycythämische Dosen, das heißt Dosen, die nicht zu einer Erhöhung des Hämatokritwertes führen, insbesondere gegenüber dem Hämatokrit-Wert vor der Behandlung mit EPO zu keinem Anstieg des Hämatokrit-Wertes von mehr als 10 %, insbesondere 5 %, bevorzugt 2 % führen. Die erfindungsgemäß vorgesehenen subpolycythämischen Dosen entsprechen wöchentlichen Dosen von etwa 0,001 bis 90 Einheiten (IU) EPO/kg Körpergewicht, insbesondere 0,001 bis 50, insbesondere 0,001 bis 45 IU EPO/kg Körpergewicht, insbesondere 1 bis 15 IU EPO/kg Körpergewicht, insbesondere 1 bis 10 IU EPO/kg Körpergewicht, insbesondere 1 bis 4 IU EPO/kg Körpergewicht, oder einer vergleichbaren Wochendosis Aranesp von 0,000005 bis 0,45µg je Kilogramm Körpergewicht, 0,00025 bis ,250 µg/kg Körpergewicht, 0,00025 bis ,225 µg/kg Körpergewicht, 0,00025 bis 0,2 µg/kg Körpergewicht, 0,00025 bis 0,175 µg/kg Körpergewicht, 0,00025 bis 0,165 µg/kg Körpergewicht, 0,00025 bis 0,155 µg/kg Körpergewicht, 0,00025 bis 0,145 µg/kg Körpergewicht, 0,00025 bis 0,135 µg/kg Körpergewicht, 0,00025 bis 0,125 µg/kg Körpergewicht, 0,00025 bis 0,115 µg/kg Körpergewicht, 0,00025 bis 0,105 µg/kg Körpergewicht, 0,00025 bis 0,095 µg/kg Körpergewicht, 0,00025 bis 0,085 µg/kg Körpergewicht, 0,00025 bis 0,075 µg/kg Körpergewicht, 0,00025 bis 0,065 µg/kg Körpergewicht, 0,00025 bis 0,055 µg/kg Körpergewicht, 0,00025 bis 0,055 µg/kg Körpergewicht, 0,00025 bis 0,045 µg/kg Körpergewicht, 0,00025 bis 0,035 µg/kg Körpergewicht, 0,00025 bis 0,025 µg/kg Körpergewicht, 0,00025 bis 0,015 µg/kg Körpergewicht, 0,00025 bis 0,005 µg/kg Körpergewicht. Bei Aranesp handelt es sich um ein doppelt PEG-yliertes EPO. Die vorstehend genannten geringen Dosen zum Beispiel die Dosis von 0,001 bis 90 Einheiten/kg/Woche pro Patient, insbesondere zum Beispiel von 0,001 bis 50 Einheiten/kg/Woche und pro Patient, die erfindungsgemäß zur Behandlung von Krankheiten oder Krankheitszuständen vorgesehen ist, die mit einer Dysfunktion endothelialer Vorläuferzellen einhergehen, ist im Vergleich zu der üblicherweise zur Therapie renaler Anämie eingesetzten Anfangsdosis von 90-150 IU/kg Körpergewicht/Woche (in der Regel beginnend mit 4000-8000 IU/Woche, bei nicht befriedigendem Therapieergebnis jedoch auch wesentlich höher) sehr niedrig.

Die angegebenen Dosierungen sind, wenn nicht anders angegeben, wöchentlich zu verabreichender Einmaldosen, können aber auch auf mehrere Einzeldosen in einer Woche verteilt werden, also als Mehrfachdosierung eingesetzt werden.

Eine besonders bevorzugte Ausführungsform der Erfindung betrifft die Verwendung von wie im vorstehenden Abschnitt "Erfindungsgemäße Dosierung von EPO" definiertem niedrig dosiertem Erythropoietin und/oder dessen Derivaten als Wirkstoff zur Herstellung einer pharmazeutischen Zusammensetzung oder eines Arzneimittels zur Therapie von Krankheitszuständen oder Krankheiten, die mit einer Dysfunktion von endothelialen Vorläuferzellen im Zusammenhang stehen.

Erfindungsgemäß wird unter einem "Wirkstoff" ein körpereigener oder körperfremder Stoff verstanden, der bei Kontakt mit Zielmolekülen oder Zielzellen oder Zielgeweben spezifische Funktionen von Geweben, Organen oder Organismen in differenzierter Weise beeinflusst. Erfindungsgemäß ist also vorgesehen, dass Erythropoietin als Wirkstoff der erfindungsgemäßen pharmazeutischen Zusammensetzung bei Kontakt mit endothelialen Vorläuferzellen deren Proliferations-, Differenzierungs- und/oder Migrationsverhalten in einem menschlichen oder tierischen Organismus so beeinflusst, dass Dysfunktionen von endothelialen Vorläuferzellen ausgeglichen und die infolge dieser Dysfunktionen auftretenden Krankheiten wirksam bekämpft, gelindert oder beseitigt werden können beziehungsweise diesen Krankheiten wirksam vorgebeugt werden kann, sowie dass der Einsatz von niedrig dosiertem Erythropoietin sowohl zur Organregeneration als auch zur Progressionsverlangsamung von Funktionseinschränkungen in unterschiedlichen Organen und Organsystemen führt.

Im Zusammenhang mit der vorliegenden Erfindung wird unter einer "pharmazeutischen Zusammensetzung" oder einem "Arzneimittel" ein zu diagnostischen, therapeutischen und/oder prophylaktischen Zwecken verwendetes, also ein die Gesundheit eines menschlichen oder tierischen Körpers förderndes oder wiederherstellendes Gemisch verstanden, das mindestens einen natürlichen oder synthetisch hergestellten Wirkstoff umfasst, der die therapeutische Wirkung hervorruft. Die pharmazeutische Zusammensetzung kann sowohl ein festes als auch ein flüssiges Gemisch sein. Beispielsweise kann eine den Wirkstoff umfassende pharmazeutische Zusammensetzung einen oder mehrere pharmazeutisch verträgliche Komponenten enthalten. Darüber hinaus kann die pharmazeutische Zusammensetzung üblicherweise auf dem Fachgebiet verwendete Zusatzstoffe, beispielsweise Stabilisatoren, Fertigungsmittel, Trennmittel, Sprengmittel, Emulgatoren oder andere üblicherweise zur Herstellung pharmazeutischer Zusammensetzung verwendete Stoffe umfassen.

Erfindungsgemäß ist insbesondere die Verwendung von Erythropoietin in vorzugsweise niedriger Dosierung und/oder eines Derivats davon als Wirkstoff zur Herstellung eines Arzneimittels zur Therapie von Hypercholesterinämie, Diabetes mellitus, Insulinresistenz, Endothel-vermittelten chronischen Entzündungserkrankungen wie Gefäßentzündungen, Endotheliose einschließlich Retikuloendotheliose, Atherosklerose, altersbedingter Herz-Kreislauf-Erkrankung, ischämischen Erkrankungen der Extremitäten, Raynaud-Krankheit, Lebererkrankungen wie Hepatitis, Leberzirrhose, akutem oder chronischem Leberversagen, Knochen- und Knorpelerkrankungen oder -verletzungen, Schleimhauterkrankungen oder -verletzungen, insbesondere im gastrointestinalen Trakt, Morbus Crohn, Colitis ulcerosa, Präeklampsie, schwangerschaftsinduzierter Hypertonie, akuter oder chronischer Niereninsuffizienz, insbesondere terminaler Niereninsuffizienz, Nierenfunktionseinschränkungen mit glomerulären Filtrationsraten < 80 ml/min, insbesondere 30, vorzugsweise 40, bis 80 ml/min, Mikroalbuminurie, Proteinurie, erhöhten ADMA-Spiegeln oder Wunden sowie Folgeerkrankungen davon vorgesehen.

Die erfindungsgemäße pharmazeutische Zusammensetzung kann sowohl zur topischen als auch zur systemischen Verabreichung geeignet sein.

In einer bevorzugten Ausführungsform der Erfindung ist vorgesehen, dass die pharmazeutische Zusammensetzung zur parenteralen, insbesondere intravenösen, intramuskulären, intrakutanen oder subkutanen Verabreichung verwendet wird. Vorzugsweise weist das Erythropoietin enthaltende Arzneimittel die Form einer Injektion oder Infusion auf.

In einer weiteren Verwendung ist vorgesehen, dass die Erythropoietin enthaltende pharmazeutische Zusammensetzung oral verabreicht wird. Beispielsweise wird das Erythropoietin enthaltende Arzneimittel in einer flüssigen Darreichungsform wie einer Lösung, Suspension oder Emulsion, oder einer festen Darreichungsform wie einer Tablette verabreicht.

In einer weiteren Verwendung ist vorgesehen, dass die pharmazeutische Zusammensetzung zur pulmonalen Verabreichung oder zur Inhalation geeignet ist. Erfindungsgemäß ist also vorgesehen, dass Erythropoietin in therapeutisch wirksamer Weise direkt an die Lungen des Patienten verabreicht wird. Diese Form der Verabreichung von Erythropoietin ermöglicht eine schnelle Abgabe einer Erythropoietin-Dosis an einen Patienten, ohne dass eine Injektion vorgenommen werden muss. Bei Aufnahme von Erythropoietin über die Lungen können erhebliche Erythropoietin-Mengen über die Lunge an den Blutkreislauf abgegeben werden, was im Blutkreislauf zu erhöhten Erythropoietin-Mengen führt. In einer bevorzugten Ausführungsform der Erfindung handelt es sich bei der über die Lunge aufzunehmenden pharmazeutischen Zusammensetzung um eine wässrige oder nichtwässrige Lösung oder um ein Trockenpulver. Bei Vorliegen des pulmonal zu verabreichende, Erythropoietin enthaltenden Arzneimittel in Form eines Trockenpulvers umfasst dieses vorzugsweise Erythropoietin-enthaltende Partikel, wobei die Partikel einen Durchmesser von weniger als 10 µm aufweisen, so dass das Medikament auch distale Bereiche der Lunge des Patienten erreichen kann. In einer besonders bevorzugten Ausführungsform der Erfindung ist vorgesehen, dass das pulmonal zu verabreichende Medikament in Form eines Aerosols vorliegt.

Eine besonders bevorzugte Ausführungsform der Erfindung betrifft die Verwendung von Erythropoietin zur Herstellung einer pharmazeutischen Zusammensetzung zur Therapie von Krankheiten, die mit einer Dysfunktion von endothelialen Vorläuferzellen im Zusammenhang stehen, wobei die pharmazeutische Zusammensetzung neben Erythropoietin als Wirkstoff mindestens einen weiteren zusätzlichen Wirkstoff zur Stimulierung von endothelialen Vorläuferzellen enthält.

Vorzugsweise handelt es sich bei dem weiteren Wirkstoff um einen Wirkstoff, der insbesondere die physiologische Mobilisierung endothelialer Vorläuferzellen aus dem Knochenmark oder "anderen Stammzell"- Nischen stimuliert. Erfindungsgemäß kann es sich bei dem weiteren Wirkstoff aber auch um einen Wirkstoff handeln, der insbesondere das Teilungsverhalten, also die Proliferation endothelialer Vorläuferzellen stimuliert. Erfindungsgemäß besteht jedoch auch die Möglichkeit, dass der weitere Wirkstoff insbesondere das Differenzierungsverhalten und/oder das Migrationsverhalten endothelialer Vorläuferzellen stimuliert. Besonders bevorzugt handelt es sich bei dem weiteren, endotheliale Vorläuferzellen stimulierenden Wirkstoff um VEGF, PIGF, GM-CSF, einen HMG-CoA-Reduktase-Inhibitor, insbesondere ein Statin wie Simvastatin, Mevastatin oder Atorvastatin, einen ACE-Hemmer wie Enalapril, Ramipril oder Trandolapril, einen AT-1-Blocker wie Irbesartan, Lorsartan oder Olmesaratan und/oder einen NO-Donator, insbesondere L-Arginin.

Erfindungsgemäß ist auch vorgesehen, dass der mindestens eine weitere Wirkstoff insbesondere ausdifferenzierte Endothelzellen, das heißt deren Proliferation und/oder Migration stimuliert, nicht jedoch endotheliale Vorläuferzellen. Besonders bevorzugt handelt es sich dabei um bFGF (basic fibroblast growth factor) oder Angiogenin.

Eine weitere Ausführungsform der Erfindung betrifft die Verwendung von Erythropoietin und/oder Derivaten davon als Wirkstoff zur Herstellung einer pharmazeutischen Zusammensetzung zur Stimulierung endothelialer Vorläuferzellen, insbesondere zur Stimulierung der Mobilisierung, Proliferation, Differenzierung zu Endothelzellen und/oder zur Migration in Richtung eines vaskulogenen oder angiogenen Stimulus. Erfindungsgemäß ist weiterhin die Verwendung von Erythropoietin und/oder dessen Derivaten als Wirkstoff zur Herstellung einer pharmazeutischen Zusammensetzung zur Stimulierung von Vaskulogenese und/oder Endothelbildung, insbesondere im adulten menschlichen oder tierischen Organismus vorgesehen.

Die vorliegende Erfindung betrifft daher auch pharmazeutische Zusammensetzungen zur Stimulierung von endothelialen Vorläuferzellen, insbesondere zur Stimulation von deren Mobilisierung, Proliferation, Differenzierung zu Endothelzellen und/oder Migration in Richtung eines vaskulogenen oder angiogenen Stimulus, zur Stimulation der Vaskulogenese und/oder Endothelbildung und zur Behandlung von Krankheiten des menschlichen oder tierischen Körpers, die mit einer Dysfunktion von endothelialen Vorläuferzellen und/oder Endothelzellen im Zusammenhang stehen. Insbesondere betrifft die vorliegende Erfindung pharmazeutische Zusammensetzungen oder Arzneimittel, die Erythropoietin als Wirkstoff und mindestens einen weiteren Wirkstoff zur Stimulation von endothelialen Vorläuferzellen und/oder ausdifferenzierten Endothelzellen umfassen. In bevorzugter Ausführungsform betrifft die vorliegende Erfindung pharmazeutische Zusammensetzungen, die Erythropoietin und mindestens einen weiteren Wirkstoff ausgewählt aus der Gruppe bestehend aus VEGF, PIGF, GM-CSF, einen HMG-CoA-Reduktase-Inhibitor, insbesondere ein Statin wie Simvastatin, Mevastatin oder Atorvastatin, einen ACE-Hemmer wie Enalapril, Ramipril oder Trandolapril, einen AT-1-Blocker wie Irbesartan, Lorsartan oder Olmesaratan, einen NO-Donator, insbesondere L-Arginin, bFGF und Angiogenin umfassen.

Eine weitere bevorzugte Ausführungsform der Erfindung betrifft die Verwendung von Erythropoietin zur Herstellung eines transplantierbaren Endothelzell-Präparates. Erfindungsgemäß ist dabei insbesondere vorgesehen, dass Endothelzellen in vitro durch Kultivierung endothelialer Vorläuferzellen in Gegenwart von Erythropoietin hergestellt und anschließend in einen Empfängerorganismus, insbesondere einen Organismus, der an einer Krankheit leidet, die im Zusammenhang mit einer Dysfunktion von endothelialen Vorläuferzellen steht, transplantiert werden. Beispielsweise können mononukleäre Zellen (MNC) aus Blut mittels Dichtegradienten-Zentrifugation isoliert und in geeigneten Kulturmedien in vitro kultiviert werden. Verfahren zur Isolierung und in vitro-Kultivierung mononukleärer Zellen sind beispielsweise in Asahara, Science, 275 (1997), 964-967; Dimmeler et al., J. Clin. Invest., 108 (2001), 391-397, und Llevadot et al., J. Clin. Invest., 108 (2001), 399-405, beschrieben. Anschließend werden die mononukleären Zellen in Gegenwart von Erythropoietin weiter kultiviert, um die in den MNCs enthaltenen endothelialen Vorläuferzellen bezüglich ihres Proliferations- und Differenzierungsverhaltens zu stimulieren und insbesondere die Anzahl differenzierter adhärenter Endothelzellen zu erhöhen. Erfindungsgemäß ist auch vorgesehen, dass die Kultivierung der MNCs in Gegenwart von Erythropoietin und mindestens einer weiteren Substanz, die die Proliferation und Differenzierung endothelialer Vorläuferzellen stimuliert, erfolgt. Besonders bevorzugt wird als weitere Substanz VEGF, PIGF, GM-CSF, ein NO-Donator wie L-Arginin, einen ACE-Hemmer wie Enalapril, Ramipril oder Trandolapril, einen AT-1-Blocker wie Irbesartan, Lorsartan oder Olmesaratan oder ein HMG-CoA-Reduktase-Inhibitor wie ein Statin, insbesondere Simvastatin, Mevastatin oder Atorvastatin, eingesetzt.

In einer weiteren bevorzugten Anwendungsform der Erfindung werden endotheliale Vorläuferzellen zeitgleich mit anderen zelltherapeutischen einsetzbaren Zellpopulationen, wie Hepatozyten, Myocyten, Cardiomyocyten oder Inselzellen, entsprechenden Patienten appliziert unter vorheriger Inkubation mit niedrig dosiertem Erythropoietin in vitro und/oder lokaler sowie systemischer Applikation von niedrig dosiertem Erythropoietin in vivo, um so das Einheilen der zelltherapeutischen Gewebszellen mit suffizienter Anbindung an das Gefäßsystem sicherzustellen.

Eine weitere bevorzugte Anwendungsform der Erfindung betrifft auch die Verwendung von Erythropoietin zur Herstellung einer pharmazeutischen Zusammensetzung oder eines Kits für die sequenzielle, zeitlich aufeinanderfolgende oder gleichzeitige Gabe von niedrig dosiertem Erythropoietin sowie einer oder mehrerer anderer chemischer, thermischer, mechanischer sowie biologischer Agenzien, um bestimmte Orte des Körpers eines Patienten, zum Beispiel Implantationszielorte zu mobilisieren, um die Zahl und Funktion endothelialer Vorläuferzellen zu erhöhen und/oder die Regeneration beziehungsweise Progressionsverlangsamung von Gewebeschäden herbeizuführen. Hierbei kann es sich bei den mechanischen Agenzien um zum Beispiel Endoprothesen, bevorzugt um Implantationskörper zum Beispiel für den Zahn, Knochen oder Band/Sehnenersatz, handeln. Ferner kann es sich bei den biologischen Agenzien um solide Organe, wie Leber, Niere, Herz, Pankreas oder Haut handeln oder auch um Haarimplantate. Die Erfindung sieht also vor, dass EPO insbesondere in niedrig dosierter Weise verwendet wird, um gleichzeitig, anschließend oder vorab implantierte mechanische Agenzien wie Endoprothesen oder biologische Agenzien besser, schneller und effizienter in die umgebende Körperstruktur einwachsen, beziehungsweise integrieren zu lassen. Die Erfindung betrifft daher auch die Verwendung von Erythropoietin zur Herstellung einer pharmazeutischen Zusammensetzung oder eines Kits für die Verbesserung, insbesondere zur Förderung und/oder Beschleunigung, der Integration von biologischen Agenzien oder Endoprothesen in umgebende Körperstrukturen, insbesondere von Zähnen, Zahnersatz, Zahnimplantaten oder sonstigen Endoprothesen, wie Knochenersatz, Knochenimplantaten, insbesondere Hüftgelenkprothesen oder Band/Sehnenersatz, wie z.B. Kreuzbänder. Gegebenenfalls kann dabei vorgesehen sein, das Erythropoietin zusammen mit zelltherapeutisch geeigneten Zellpopulationen und/oder endothelialen Vorläuferzellen zu verwenden. In der vorgenannten Verwendung von Erythropoietin zur Herstellung einer pharmazeutischen Zusammensetzung oder eines Kits für die Verbesserung, insbesondere Förderung und/oder Beschleunigung der Integration von biologischen oder mechanischen Agenzien in Zielstrukturen, insbesondere Zielgewebe, Zielknochen oder Zielknorpel eines Patienten, kann in einer weiteren bevorzugten Ausführungsform vorgesehen sein, dass die einzusetzenden mechanischen Agenzien, zum Beispiel aus Stahl, Keramik, Kunststoff oder einem sonstigen Material hergestellt sind. Darüber hinaus kann vorgesehen sein, dass in der genannten Verwendung als zelltherapeutisch geeignete Zellpopulationen, Osteoblasten, Zellen mit osteogenem Potential, Thrombozyten, Blutzellen oder ähnlich eingesetzt werden. In weiterer bevorzugter Ausführungsform kann vorgesehen sein, dass insbesondere das einzusetzende mechanische Agens zusammen mit organischem Klebstoff, zum Beispiel einem Fibrinkleber in der pharmazeutischen Zusammensetzung oder dem pharmazeutischen Kit enthalten sind.

In einer weiteren bevorzugten Ausführung der Erfindung ist die Verwendung von Erythropoietin oder geeigneter Wirkstoffe zur topischen Anwendung im Sinne der "Schönheitspflege", insbesondere der Prophylaxe beziehungsweise zeitnahen Reduktion von Falten und Fältchen, Stärkung des Bindegewebes, Schutz sowie Straffung der Haut, insbesondere der Gesichtshaut, gegen schädliche Umweltfaktoren und als Make up-Unterlage vorgesehen. Duch die topische Anwendung von Erythropoietin soll der Entstehung und Weiterentwicklung von Altersflecken entgegengewirkt werden, das Hautbild verfeinert, sowie der Erneuerungsprozess der Haut, bevorzugt durch eine beschleunigte Reepithelialisierung aber auch das Haarwachstum unterstützt werden.

In einer weiteren Anwendungsform ist die Gabe von niedrig dosiertem Erythropoietin in Anpassung an dessen circadianen Rhythmus vorgesehen. Die endogene Erythropoietinproduktion besitzt ihre Akrophase (Tagesmaximum) am späten Nachmittag, daher sollte die Gabe von niedrig dosiertes Erythropoietin vorzugsweise vormittags, insbesondere zwischen 6:00 und 10:00 erfolgen, um so einen maximalen biologischen Effekt zu erzielen.

In einer weiteren bevorzugten Ausführungsform der Erfindung ist die Verwendung von niedrig dosiertem Erythropoietin zur Vorbehandlung und/oder Weiterbehandlung von zu transplantierenden Geweben oder Organen vorgesehen. Dabei werden die Transplantate vor der Transplantation, vorzugsweise unmittelbar davor, noch im Spenderorganismus mit niedrig dosiertem Erythropoietin behandelt. Der Empfängerorganismus kann ebenfalls mit niedrig dosiertem Erythropoietin vom Zeitpunkt der Transplantation an behandelt werden. Durch diese Behandlung der zu transplantierenden Organe oder Gewebe sowohl direkt vor als auch nach Transplantation mit Erythropoietin wird erfindungsgemäß erreicht, dass sich in dem Transplantat nach erfolgter Transplantation in einen Körper aufgrund der induzierten Vaskulogenese schnell neue Blutgefäße bilden und diese neu gebildeten Blutgefäße schnell mit dem Blutsystem des Empfängerorganismus verbunden werden. Ebenfalls wird auf diese Weise schnell die Bildung von Endothelien erreicht. Die Behandlung von Organ- oder Gewebetransplantaten mit niedrig dosiertem Erythropoietin bewirkt somit ein schnelleres Einwachsen dieser Systeme in den Körper, wodurch das Risiko einer Abstoßung erheblich vermindert wird. Darüber hinaus wird durch die Gabe von niedrig dosiertem Erythropoietin die Organregeneration stimuliert.

In einer weiteren Ausgestaltung der Erfindung ist vorgesehen, dass die Organ- oder Gewebetransplantate vor Transplantation mit niedrig dosiertem Erythropoietin in Kombination mit mindestens einem weiteren Faktor, der endotheliale Vorläuferzellen stimuliert, behandelt werden. Vorzugsweise handelt es sich bei diesem Faktor um eine Substanz ausgewählt aus der Gruppe bestehend aus VEGF, PIGF, GM-CSF, einem HMG-CoA-Reduktase-Inhibitor, beispielsweise ein Statin, insbesondere Simvastatin, Mevastatin oder Atorvastatin, einen ACE-Hemmer wie Enalapril, Ramipril oder Trandolapril, einen AT-1-Blocker wie Irbesartan, Lorsartan oder Olmesaratan oder einen NO-Donator, insbesondere L-Arginin. In einer weiteren Ausgestaltung ist vorgesehen, dass die Organ- oder Gewebetransplantate vor Transplantation neben Erythropoietin mit einer weiteren Substanz, die die Proliferation und Migration ausdifferenzierter Endothelzellen stimuliert, behandelt werden. Besonders bevorzugt handelt es sich bei dieser Substanz um Angiogenin oder bFGF. In einer weiteren Ausgestaltung ist vorgesehen, dass die Vorbehandlung der Organ- oder Gewebetransplantate mit Erythropoietin unter Verwendung isolierter und gegebenenfalls in vitro expandierter endothelialer Vorläuferzellen erfolgt.

In einer weiteren besonders bevorzugten Ausführungsform der Erfindung ist vorgesehen, dass niedrig dosiertes Erythropoietin zur Herstellung von implantierbaren oder transplantierbaren, zellhaltigen in vitro-Organen oder -Geweben verwendet wird. Erfindungsgemäß ist insbesondere vorgesehen, dass das in vitro hergestellte Organ oder Gewebe vor der Transplantation oder Implantation mit niedrig dosiertem Erythropoietin in vitro behandelt wird, um die im Körper des Empfängerorganismus vorhandenen endothelialen Vorläuferzellen, insbesondere deren physiologische Mobilisierung, Migration, Proliferation und Differenzierung, zu stimulieren. Vorzugsweise wird der Empfängerorganismus nach Transplantation beziehungsweise Implantation des in vitro-Organs oder -Gewebes weiter mit niedrig dosierten Erythropoietin in den erfindungsgemäßen Dosen behandelt. Durch die Behandlung des in vitro-Organs- oder Gewebes vor Transplantation beziehungsweise Implantation mit Erythropoietin und gegebenenfalls die Nachbehandlung des Empfängerorganismus mit Erythropoietin wird erfindungsgemäß erreicht, dass sich in dem in vitro-Organ- oder -Gewebesystem nach erfolgter Transplantation oder Implantation in einen Körper aufgrund der induzierten Vaskulogenese schnell neue Blutgefäße bilden und diese neu gebildeten Blutgefäße schnell mit dem Blutsystem des Empfängerorganismus verbunden werden. Ebenfalls wird auf diese Weise schnell die Bildung von Endothelien und damit eine Reendothelialisierung erreicht. Die Behandlung der in vitro-Organ- oder -Gewebesysteme mit niedrig dosiertem Erythropoietin bewirkt somit ein schnelleres Einwachsen dieser Systeme in den Körper, wodurch das Risiko einer Abstoßung erheblich vermindert wird, und dient der Protektion des Transplantats.

Unter einem "in vitro-Organ- oder -Gewebesystem" wird ein transplantierbares oder implantierbares zellhaltiges Gewebe oder Organ verstanden, das in vitro unter Verwendung definierter Zellen und/oder definierter Gewebe und unter definierten Kulturbedingungen hergestellt wird. Unter einem "implantierbaren in vitro-Organ- oder -Gewebesystem" wird ein System verstanden, das neben Zellen körperfremde Materialien umfasst. Unter einem "transplantierbaren in vitro-Organ- oder -Gewebesystem" wird insbesondere ein zellhaltiges System verstanden, das neben Zellen, Geweben oder Organen des gleichen oder eines anderen Individuums körpereigene Substanzen enthält. In vitro-Organe oder -Gewebe sind insbesondere **dadurch gekennzeichnet, dass** sie bezüglich ihres Aufbaus weitgehend den nativen zu ersetzenden Organen oder Geweben entsprechen und somit die Funktion der ersetzten nativen Organe oder Gewebe in vivo übernehmen können.

In einer erfindungsgemäßen Ausgestaltung ist vorgesehen, dass die in vitro-Organ- oder -Gewebesysteme vor Transplantation beziehungsweise Implantation mit Erythropoietin in Kombination mit mindestens einem weiteren Faktor, der endotheliale Vorläuferzellen stimuliert, behandelt werden. Vorzugsweise handelt es sich bei diesem Faktor um eine oder mehrere Substanzen ausgewählt aus der Gruppe bestehend aus VEGF, PIGF, GM-CSF, einen HMG-CoA-Reduktase-Inhibitor, insbesondere Simvastatin, Mevastatin oder Atorvastatin, einen ACE-Hemmer wie Enalapril, Ramipril oder Trandolapril, einen AT-1-Blocker wie Irbesartan, Lorsartan oder Olmesaratan, und einem NO-Donator. In einer weiteren Ausgestaltung ist vorgesehen, dass die in vitro-Organ- oder -Gewebesysteme vor Transplantation beziehungsweise Implantation neben Erythropoietin mit einer weiteren Substanz, die die Proliferation und Migration ausdifferenzierter Endothelzellen stimuliert, behandelt werden. Besonders bevorzugt handelt es sich bei dieser Substanz um Angiogenin oder bFGF. In einer weiteren Ausgestaltung ist vorgesehen, dass die in vitro-Organ- oder -Gewebesysteme zusätzlich isolierte und gegebenenfalls in vitro expandierte endotheliale Vorläuferzellen enthalten.

Eine weitere bevorzugte Ausführungsform der Erfindung betrifft die Verwendung von niedrig dosiertem Erythropoietin zur Herstellung von Gefäßprothesen oder Herzklappen, wobei die Gefäßprothesen oder Herzklappen vor Insertion in einen Körper, insbesondere einen menschlichen Körper, mit Erythropoietin beschichtet werden. Durch die Beschichtung der Gefäßprothesen oder Herzklappen mit Erythropoietin wird erreicht, dass endotheliale Vorläuferzellen im Körper des Empfängerorganismus stimuliert werden, wobei insbesondere ihre Mobilisierung aus dem Knochenmark, ihre Proliferation, ihre Differenzierung zu Endothelzellen und ihre Migration zu den eingesetzten Gefäßprothesen oder Herzklappen stimuliert werden. Nach Einführung der so hergestellten Gefäßprothese oder Herzklappen in einen Körper kann dieser mit Erythropoietin, insbesondere in den erfindungsgemäßen Dosen, weiterbehandelt werden. Dadurch bedingt, kommt es schneller zu einer Ausbildung von Endothelschichten auf den eingesetzten Gefäßprothesen und zu einem schnelleren Einwachsen in die betroffenen Körperbereiche. In einer bevorzugten Ausgestaltung ist vorgesehen, dass zur Beschichtung der Gefäßprothesen und Herzklappen zusätzlich isolierte und gegebenenfalls in vitro expandierte endotheliale Vorläuferzellen eingesetzt werden.

Die vorliegende Erfindung betrifft ebenfalls ein Verfahren zur Stimulation der Endothelzell-Bildung in vitro, umfassend
a) die Isolierung endotheliale Vorläuferzellen enthaltender Zellpopulationen aus Blut mittels Dichtegradienten-Zentrifugation,
b) die Kultivierung der isolierten, endotheliale Vorläuferzellen enthaltenden Zellpopulationen in Zellkulturmedium, und
c) die Kultivierung der Zellpopulationen in Gegenwart von niedrig dosiertem Erythropoietin.

Erfindungsgemäß kann die Kultivierung der Zellpopulationen in Gegenwart einer weiteren Substanz, die endotheliale Vorläuferzellen stimuliert, erfolgen.

Die vorliegende Erfindung betrifft ferner ein Verfahren zur Behandlung von Krankheiten, die mit einer Dysfunktion von endothelialen Vorläuferzellen im Zusammenhang stehen, durch Verabreichung von Erythropoietin in einer geringen Dosis wie im Absatz "Erfindungsgemäße Dosierung von EPO" erläutert, allein oder in Kombination mit mindestens einem anderen chemischen, thermischen, mechanischen sowie biologischen Agenzien an einen Patienten mit einer solchen Krankheit. Das erfindungsgemäße Verfahren ist insbesondere zur Behandlung von Krankheiten des menschlichen Körpers wie Hypercholesterinämie, Diabetes mellitus, Insulinresistenz, Endothelvermittelten chronischen Entzündungserkrankungen wie Gefäßentzündungen, Endotheliose einschließlich Retikuloendotheliose, Atherosklerose, altersbedingter Herz-Kreislauf-Erkrankung, ischämischen Erkrankungen der Extremitäten, Raynaud-Krankheit, Lebererkrankungen wie Hepatitis, Leberzirrhose, akutes oder chronisches Leberversagen, Knochen- und Knorpelerkrankungen oder -verletzungen, Schleimhauterkrankungen oder -verletzungen, insbesondere im gastrointestinalen Trakt, Morbus Crohn, Colitis ulcerosa, Präeklampsie, schwangerschaftsinduzierter Hypertonie, akuter oder chronischer Niereninsuffizienz, insbesondere terminaler Niereninsuffizienz, Nierenfunktionseinschränkungen mit glomerulären Filtrationsraten < 80 ml/min, insbesondere 30 bis 80, bevorzugt 40 bis 80 ml/min Mikroalbuminurie, Proteinurie, erhöhten ADMA-Spiegeln oder Wunden sowie Folgeerkrankungen geeignet.

In einer bevorzugten Ausführungsform des erfindungsgemäßen Verfahrens zur Behandlung von Krankheiten, die mit einer Dysfunktion von endothelialen Vorläuferzellen im Zusammenhang stehen, ist vorgesehen, dass dem Patienten neben Erythropoietin mindestens ein weiterer Wirkstoff ausgewählt aus der Gruppe bestehend aus VEGF, PIGF, GM-CSF, einem HMG-CoA-Reduktase-Inhibitor und einem NO-Donator verabreicht wird. Vorzugsweise handelt es sich bei dem verabreichten HMG-CoA-Reduktase-Inhibitor um ein Statin wie Simvastatin, Mevastatin oder Atorvastatin. Bei dem verabreichten ACE-Hemmer handelt es sich um einen Wirkstoff wie Enalapril, Ramipril oder Trandolapril, bei dem verabreichten AT-1 Blocker um Wirkstoffe wie Irbesartan, Lorsartan oder Olmesaratan. Bei dem verabreichten NO-Donator handelt es sich vorzugsweise um L-Arginin.

In einer weiteren bevorzugten Ausführungsform des erfindungsgemäßen Verfahrens zur Behandlung von Krankheiten, die mit einer Dysfunktion von endothelialen Vorläuferzellen im Zusammenhang stehen, ist vorgesehen, endotheliale Vorläuferzellen aus dem Blut eines menschlichen Organismus zu isolieren, unter Verwendung von niedrig dosiertem Erythropoietin in vitro zu expandieren und zu Endothelzellen zu differenzieren und nach Aufreinigung und Isolierung der ausdifferenzierten Endothelzellen beziehungsweise der sich differenzierenden endothelialen Vorläuferzellen diese anschließend gezielt in einen Körperbereich, ein Gewebe oder ein Organ eines Patienten zu transplantieren, der/das aufgrund der Dysfunktion endothelialer Vorläuferzellen und/oder Endothelzellen geschädigt ist, um dort eine lokale Endothel-Neubildung zu induzieren. Auf diese Weise ist eine gezieltere und schnellere Behandlung der geschädigten Körperbereiche, Gewebe und/oder Organe des Patienten möglich. Diese Ausführungsform des erfindungsgemäßen Verfahrens zur Behandlung von Krankheiten, die mit einer Dysfunktion von endothelialen Vorläuferzellen im Zusammenhang stehen, umfasst die folgenden Schritte:
a) die Isolierung endotheliale Vorläuferzellen enthaltender Zellpopulationen aus Blut mittels Dichtegradienten-Zentrifugation,
b) die Kultivierung der endotheliale Vorläuferzellen enthaltenden Zellpopulationen in Zellkulturmedium,
c) die Kultivierung der endotheliale Vorläuferzellen enthaltenden Zellpopulationen in Gegenwart von niedrig dosiertem Erythropoietin zur Stimulation der Proliferation endothelialer Vorläuferzellen und/oder deren Differenzierung zu Endothelzellen,
d) die Isolierung und Aufreinigung der ausdifferenzierten Endothelzellen, und
e) die Transplantation der ausdifferenzierten Endothelzellen in einen Körper mit einer Krankheit, die mit einer Dysfunktion von endothelialen Vorläuferzellen im Zusammenhang steht.

Nach Transplantation der ausdifferenzierten Endothelzellen in einen Körper kann dieser mit Erythropoietin, insbesondere in den erfindungsgemäß vorgesehenen niedrigen Dosen, das heißt in den im Abschnitt "Erfindungsgemäße Dosierung von EPO" definierten Dosen von zum Beispiel 1, vorzugsweise 0,001 bis 90 IU/kg/Woche oder 20 bis 2000 IU/Woche, weiterbehandelt werden.

Erfindungsgemäß können die endotheliale Vorläuferzellen enthaltenden Zellpopulationen in vitro in Gegenwart mindestens eines weiteren Wirkstoffes ausgewählt aus der Gruppe bestehend aus VEGF, PIGF, GM-CSF, einem HMG-CoA-Reduktase-Inhibitor, einem ACE-Hemmer, einem AT-1 Blocker und einem NO-Donator kultiviert werden. Vorzugsweise handelt es sich bei dem zur Kultivierung verwendeten HMG-CoA-Reduktase-Inhibitor um ein Statin wie Simvastatin, Mevastatin oder Atorvastatin, bei den ACE-Hemmern um Substanzen wie Enalapril, Ramipril oder Trandolapril und bei dem AT-1 Blocker um Substanzen wie Irbsartan, Lorsartan oder Olmesaratan.

Erfindungsgemäß können endotheliale Vorläuferzellen enthaltene Zellpopulationen mit einer sequenziellen, zeitlich aufeinanderfolgenden oder gleichzeitigen Gabe von niedrig dosiertem Erythropoietin sowie einer oder mehrerer anderer chemischer, thermischer, mechanischer sowie biologischer Agenzien behandelt werden, um so die Zahl und Funktion endothelialer Vorläuferzellen zu erhöhen und/oder die Regeneration beziehungsweise Progressionsverlangsamung von Gewebeschäden herbeizuführen.

Eine weitere bevorzugte Ausführungsform der Erfindung betrifft ein Verfahren zur Behandlung von Gefäßerkrankungen, umfassend:
a) die Isolierung endotheliale Vorläuferzellen enthaltender Zellpopulationen aus Blut mittels Dichtegradienten-Zentrifugation,
b) die Kultivierung der endotheliale Vorläuferzellen enthaltenden Zellpopulationen in Zellkulturmedium,
c) die Kultivierung der endotheliale Vorläuferzellen enthaltenden Zellpopulationen in Gegenwart von Erythropoietin zur Stimulation der Proliferation endothelialer Vorläuferzellen und/oder deren Differenzierung zu Endothelzellen,
d) die Isolierung und Aufreinigung der ausdifferenzierter Endothelzellen, und
e) die Transplantation der Endothelzellen in einen Körper mit einer Gefäßerkrankung.

Nach Transplantation der Endothelzellen in den Körper mit einer Gefäßerkrankung kann dieser mit Erythropoietin, vorzugsweise in den erfindungsgemäßen niedrigen Dosen, das heißt in den im Absatz" Erfindungsgemäße Dosierung von EPO" definierte Dosen von zum Beispiel 0,001 bis 90 Einheiten/kg/Woche oder 20 IU/Woche bis 2000 IU/Woche, weiterbehandelt werden.

Erfindungsgemäß besteht die Möglichkeit, die endotheliale Vorläuferzellen enthaltenden Zell-Populationen in Gegenwart mindestens eines weiteren Wirkstoffes ausgewählt aus der Gruppe bestehend aus VEGF, PIGF, GM-CSF, einem ACE-Hemmer, einem AT-1 Blocker und/oder einem HMG-CoA-Reduktase-Inhibitor zu kultivieren. Vorzugsweise handelt es sich bei dem zur Kultivierung verwendeten ACE-Hemmer um Substanzen wie Enalapril, Ramipril oder Trandolapril, bei dem zur Kultivierung verwendeten AT-1 Blocker um Substanzen wie Irbesartan, Lorsartan oder Olmesaratan und bei dem zur Kultivierung verwendeten HMG-CoA-Reduktase-Inhibitor um ein Statin wie Simvastatin, Mevastatin oder Atorvastatin.

Erfindungsgemäß können endotheliale Vorläuferzellen enthaltene Zellpopulationen mit eine sequenziellen, zeitlich aufeinanderfolgenden oder gleichzeitigen Gabe von niedrig dosiertem Erythropoietin sowie einer oder mehrerer anderer chemischer, thermischer, mechanischer sowie biologischer Agenzien behandelt werden, um so die Zahl und Funktion endothelialer Vorläuferzellen zu erhöhen und/oder die Regeneration beziehungsweise Progressionsverlangsamung von Gewebeschäden herbeizuführen. Hierbei kann es sich bei den mechanischen Agenzien um Endoprothesen, bevorzugt um lmplantationskörper für den Zahn, Knochen oder Band/Sehnenersatz, handeln. Ferner kann es sich bei den biologischen Agenzien um solide Organe, wie Leber, Niere, Herz, Pankreas oder Haut handeln oder auch um Haarimplantate. Die Erfindung sieht also auch vor, dass EPO insbesondere in niedrig dosierter Weise verwendet wird, um gleichzeitig, anschließend oder vorab implantierte mechanische Agenzien wie Endoprothesen oder biologische Agenzien besser, schneller und effizienter in die umgebende Körperstruktur einwachsen, beziehungsweise integrieren zu lassen. Die Erfindung betrifft daher auch die Verwendung von Erythropoietin zur -herstellung einer pharmazeutischen Zusammensetzung oder eines Kits für die Verbesserung, insbesondere zur Förderung und/oder Beschleunigung, der Integration von biologischen Agenzien oder Endoprothesen in umgebende Körperstrukturen, insbesondere von Zähnen, Zahnersatz, Zahnimplantaten oder sonstigen Endoprothesen, wie Knochenersatz, Knochenimplantaten, insbesondere Hüftgelenkprothesen oder Band/Sehnenersatz, wie z.B. Kreuzbänder. In bevorzugter Ausführungsform kann dabei vorgesehen sein, das Erythropoietin zusammen mit zelltherapeutisch geeigneten Zellpopulationen und/oder endothelialen Vorläuferzellen zu verwenden. In der vorgenannten Verwendung von Erythropoietin zur Herstellung einer pharmazeutischen Zusammensetzung oder eines Kits für die Verbesserung, insbesondere Förderung und/oder Beschleunigung der Integration von biologischen oder mechanischen Agenzien in Zielstrukturen, insbesondere Zielgewebe, Zielknochen oder Zielknorpel eines Patienten, kann in einer weiteren bevorzugten Ausführungsform vorgesehen sein, dass die einzusetzenden mechanischen Agenzien, zum Beispiel aus Stahl, Keramik, Kunststoff oder einem sonstigen Material hergestellt sind. Darüber hinaus kann vorgesehen sein, dass in der genannten Verwendung als zelltherapeutisch geeignete Zellpopulationen, Osteoblasten, Zellen mit osteogenem Potential, Thrombozyten, Blutzellen oder ähnlich eingesetzt werden. In weiterer bevorzugter Ausführungsform kann vorgesehen sein, dass insbesondere das einzusetzende mechanische Agens zusammen mit organischem Klebstoff, zum Beispiel einen Fibrinkleber in der pharmazeutischen Zusammensetzung oder dem pharmazeutischen Kit enthalten sind.

Das erfindungsgemäße Verfahren zur Behandlung von Gefäßerkrankungen sieht also vor, endotheliale Vorläuferzellen aus dem Blut eines menschlichen Organismus zu isolieren, unter Verwendung von niedrig dosiertem Erythropoietin in vitro zu expandieren und zu Endothelzellen zu differenzieren und nach Aufreinigung und Isolierung der ausdifferenzierten Endothelzellen beziehungsweise der sich differenzierenden endothelialen Vorläuferzellen diese anschließend gezielt in ein geschädigtes Blutgefäß oder einen ischämischen Bereich zu transplantieren, um dort eine lokale Neovaskularisierung zu induzieren. Auf diese Weise ist eine gezieltere und schnellere Behandlung geschädigter Blutgefäße oder ischämischer Gewebe möglich. Das erfindungsgemäße Verfahren zur Behandlung von Gefäßerkrankungen ist insbesondere zur Behandlung von Gefäßerkrankungen wie Ischämie, insbesondere cerebraler Ischämie, ischämischer Erkrankungen der Extremitäten, Schlaganfall, akuten Arterienverschluss, arterielle Verschlusskrankheit, Raynaud-Krankheit und Ergotismus geeignet.

Weitere vorteilhafte Ausgestaltungen der Erfindung ergeben sich aus den Unteransprüchen.

Die Erfindung wird anhand der folgenden Figuren und Beispiele näher erläutert.

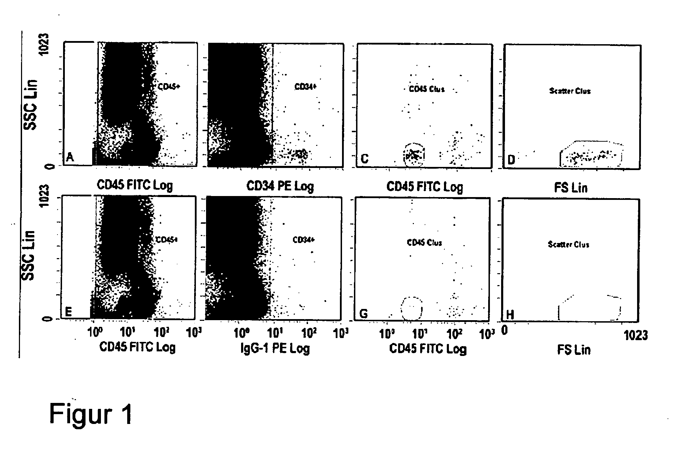

Figur 1 zeigt die Ergebnisse einer FACS-Analyse zirkulierender CD34+-Stammzellen (cSC). (A-D): Patientenproben; (E-F): isotypische Kontrollen. cSC wurden anhand der zusätzlichen Expression des CD34-Markers (B und F), anhand der charakteristischen geringen bis mittleren CD45-Antigen-Expression (C und G) und anhand der charakteristischen Lichtstreuungs-Eigenschaften (D und H) identifiziert. Die absolute cSC-Anzahl wurde pro 100.000 Mono- und Lymphozyten berechnet.

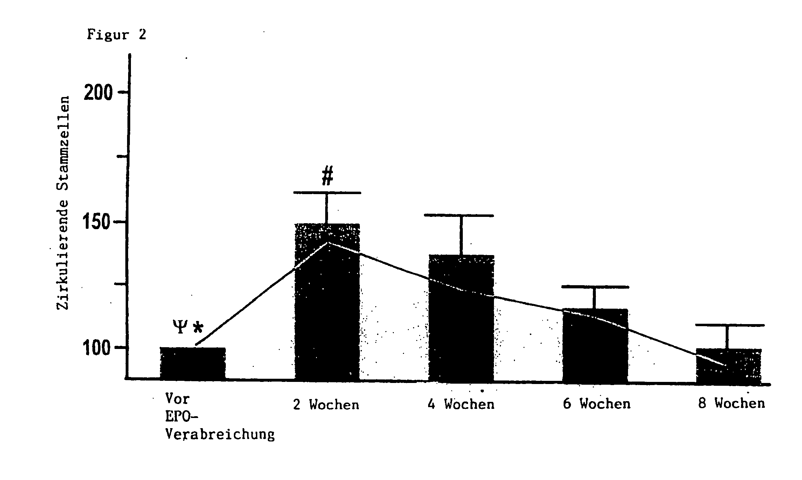

Figur 2 zeigt eine quantitative Bestimmung zirkulierender Stammzellen mittels Durchflusszytometrie. Die Figur zeigt die zeitabhängige Wirkung einer Erythropoietin-Behandlung unter Verwendung von rhEPO (rekombinantes menschliches Erythropoietin) nach 0, 2, 4, 6 und 8 Wochen. n=11, die Werte entsprechen Mittelwerten +/Standardabweichung. Mediane als Linie dargestellt.
*: p < 0,01 im Vergleich zu 2 Wochen: Ψ: p < 0,05 im Vergleich zu 4 Wochen, #: p < 0,05 im Vergleich zu 8 Wochen.

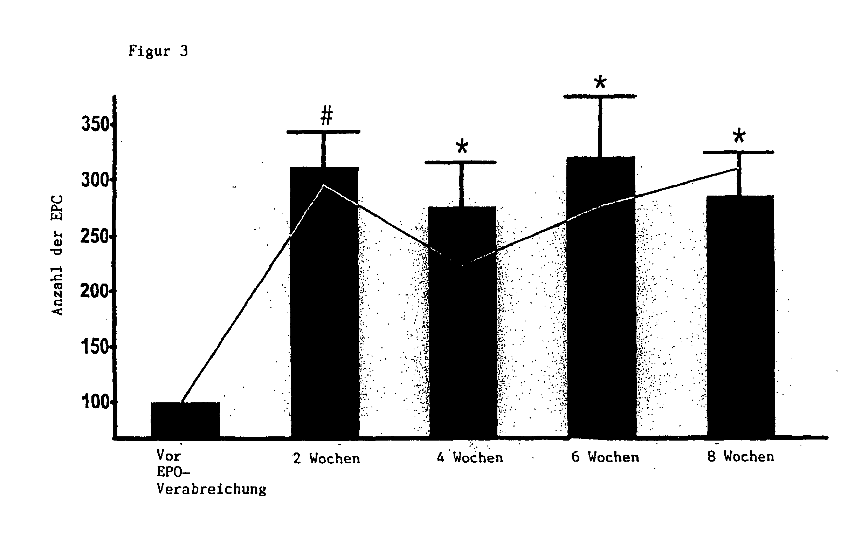

Figur 3 zeigt eine quantitative Bestimmung kultivierter endothelialer Vorläuferzellen (EPC). Die Figur zeigt, dass die rhEPO-Behandlung die relative Anzahl von EPCs erhöht. EPCs wurden vor der Behandlung renaler Patienten mit rhEPO sowie 2, 4, 6 und 8 Wochen nach Behandlung der Patienten mit rhEPO isoliert und anhand ihrer Adhäsionsfähigkeit und der beiden Marker acLDL-Dil und UEA-1 FITC charaketrisiert. n=11, die Werte entsprechen Mittelwerten +/Standardabweichung. Mediane als Linie dargestellt.
*: p < 0,01 gegenüber dem Zeitraum vor Behandlung; #: p < 0,001 gegenüber dem Zeitraum vor Behandlung.

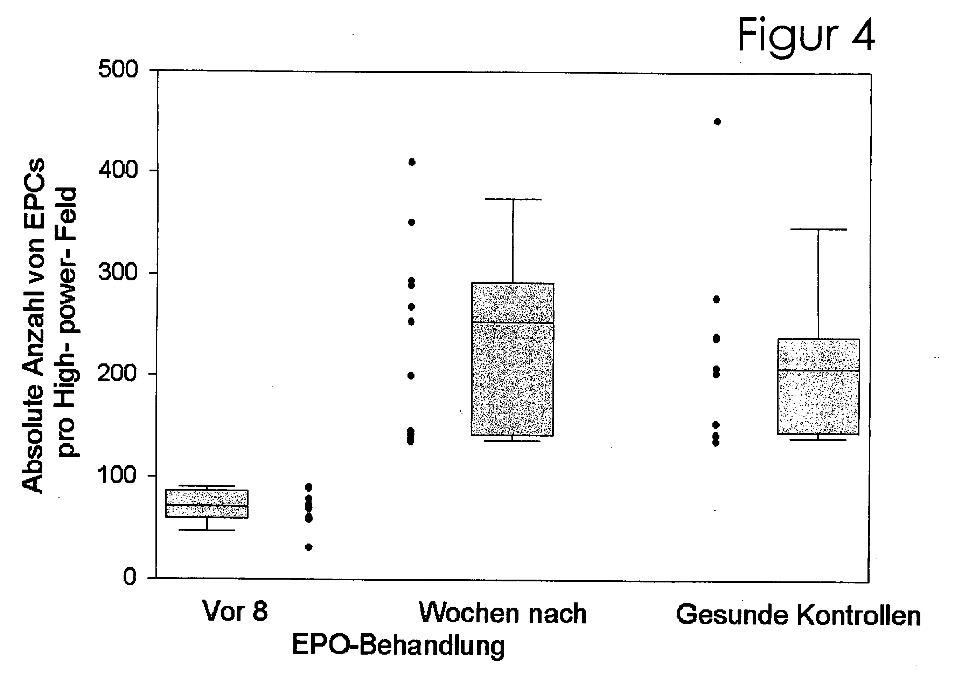

Figur 4 zeigt die quantitative Bestimmung kultivierter endothelialer Vorläuferzellen (EPC). Die Figur zeigt, dass die absolute Anzahl von EPCs vor Einsetzen einer rhEPO-Therapie signifikant gegenüber gesunden alters- und geschlechtsgematchten Probanden verringert ist. Patienten mit renaler Anämie zeigen also eine deutliche EPC-Dysfunkion gegenüber Kontrollpersonen. 8 Wochen nach Beginn der rhEPO-Therapie zur renalen Anämie wird diese verminderte Anzahl funktioneller EPC ausgeglichen. EPCs wurden vor der Behandlung renaler Patienten mit rhEPO sowie 2, 4, 6 und 8 Wochen nach Behandlung der Patienten mit rhEPO isoliert und anhand ihrer Adhäsionsfähigkeit und der beiden Marker acLDL-Dil und UEA-1 FITC charaketrisiert. n=11. Dargestellt ist exemplarisch der 8-Wochenverlauf und die gesamten Kontrollen. Die absoluten Werte sind einerseits als Einzelwerte dargestellt. Zusätzlich sind Boxplots dargestellt (90-er/75-er/50-er/25-er und 10-er Percentile sowie der Mittelwert). Als gesunde Kontrolle dienten alters- und geschlechtsgematchte Probanden, bei denen analog EPCs isoliert und charakterisiert wurden (n =11).

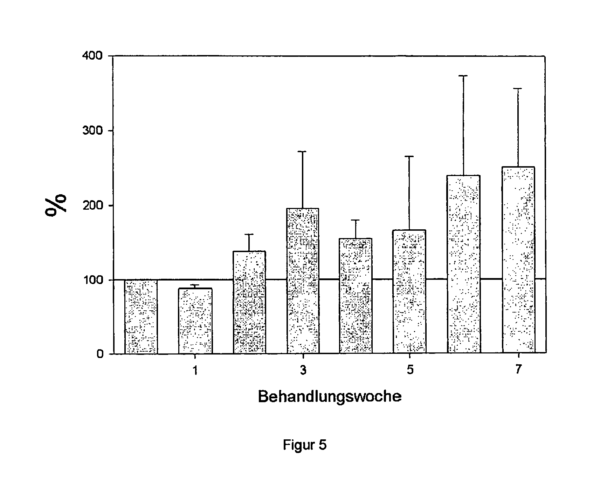

Figur 5 zeigt die quantitative Bestimmung kultivierter endothelialer Vorläuferzellen (EPC) bei gesunden jungen Probanden. Die Figur zeigt, dass eine Behandlung mit rhEPO-Behandlung (30IU Epoetin beta je kg Körpergewicht und Woche) die relative Anzahl von EPCs erhöht. EPCs wurden vor der Behandlung der Probanden mit rhEPO sowie wöchentlich nach 1, 2, 3, 4, 5, 6 und 7 Wochen nach Behandlung der Probanden mit rhEPO isoliert und anhand ihrer Adhäsionsfähigkeit und der beiden Marker acLDL-Dil und UEA-1 FITC charaketrisiert. n=4, die Werte entsprechen Mittelwerten +/Standardabweichung.

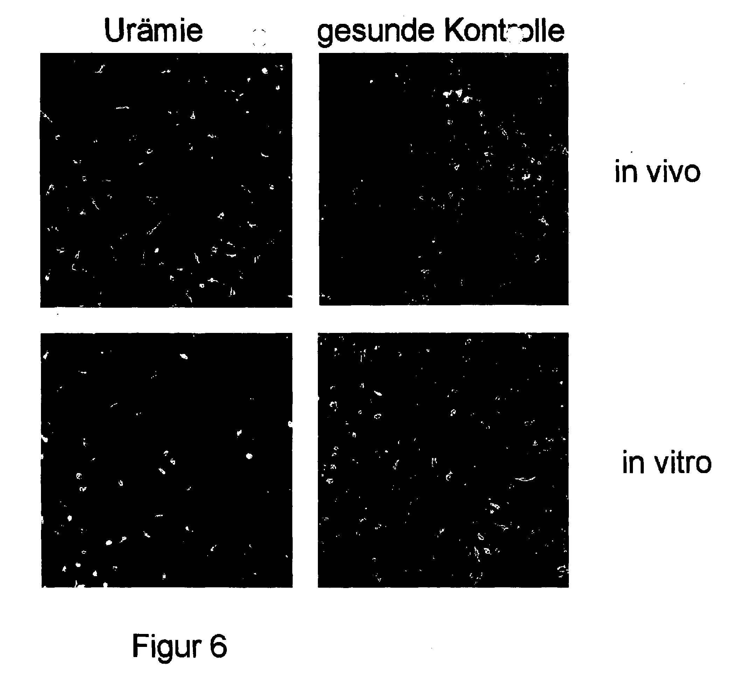

Figur 6 zeigt eine quantitative Bestimmung kultivierter endothelialer Vorläuferzellen (EPCs). Die repräsentativen Fotographien zeigen, dass die absolute Anzahl von EPCs bei urämischen Patienten signifikant gegenüber gesunden alters- und geschlechtsgematchten Probanden verringert ist (obere Reihe- in vivo). Patienten mit eingeschränkter Nierenfunktion zeigen also eine deutliche EPC-Dysfunkion gegenüber Kontrollpersonen. Werden endotheliale Vorläuferzellen eines gesunden Probanden mit Serum urämischer Patienten cokultiviert, so vermindert sich die Differenzierungsfähigkeit seiner endothelialen Vorläuferzellen (untere Reihe- in vitro). Eine eingeschränkte Nierenfunktion mit sich daraus ableitender Urämie führt also zu einer Dysfunktion endothelialer Vorläuferzellen.

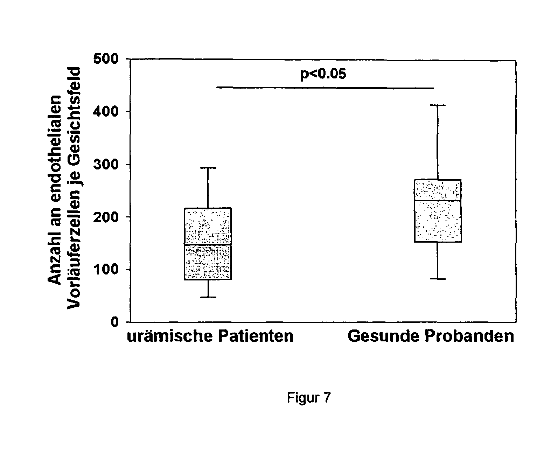

Figur 7 zeigt eine quantitative Bestimmung kultivierter endothelialer Vorläuferzellen (EPCs) bei 46 urämischen Patienten mit eingeschränkter Nierenfunktion im Vergleich mit 46 alters- und geschlechtsgematchten Probanden als Boxplots dargestellt (90-er/75-er/50-er/25-er und 10-er Percentile sowie der Mittelwert). Die Zahl der endothelialen Vorläuferzellen ist dabei signifikant bei den Urämikern gegenüber den gesunden Probanden verringert. Patienten mit eingeschränkter Nierenfunktion zeigen also eine deutliche EPC-Dysfunktion gegenüber Kontrollpersonen.

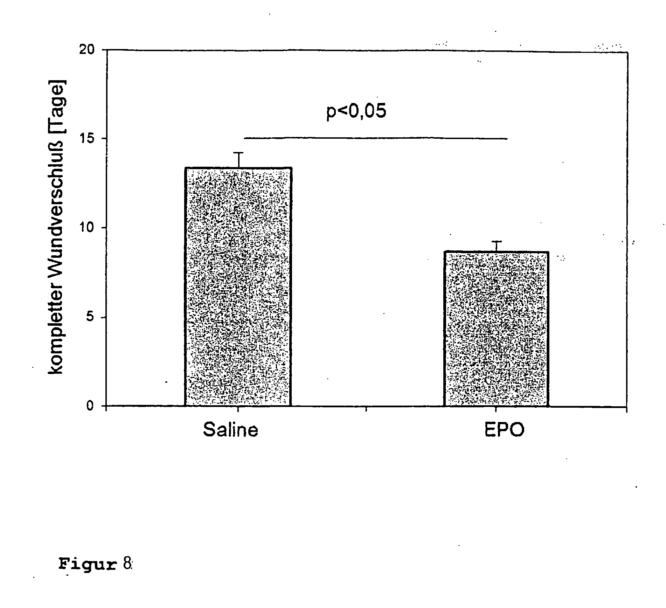

Figur 8 zeigt die Wirkung von Erythropoietin auf die Wundheilung. Die Figur zeigt, dass bei Behandlung einer standardisierten Hautwunde, die bei Mäusen mittels einer Gewebestanze gesetzt worden war, mit Erythropoietin die Wunde bereits nach sieben bis acht Tagen vollständig verschlossen war, während bei Behandlung der Wunde mit physiologischer Kochsalzlösung (Saline) die Wunde erst nach dreizehn bis vierzehn Tagen vollständig verschlossen war. Die Behandlung mit Erythropoietin beziehungsweise physiologischer Kochsalzlösung begann 7 Tage vor Setzen der Hautwunde. Rekombinantes humanes Erythropoietin wurde einmalig wöchentlich mittels einer s.c. (subcutan)-Injektion (0,1 mg/kg Aranesp) verabreicht (je Gruppe n = 5).

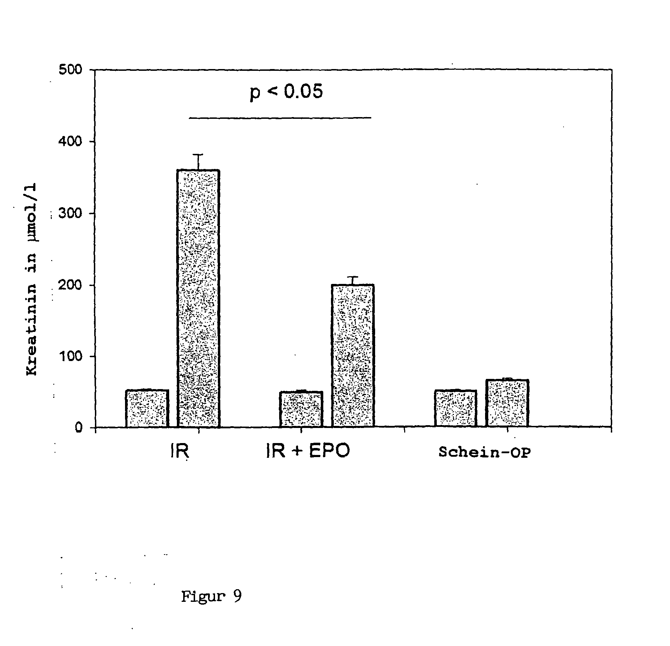

Figur 9 zeigt, dass Erythropoietin den Nierenfunktionsverlust nach akutem Nierenversagen (akute Niereninsuffizienz) vermindert. Sprague Dawley-Ratten (250-300 g) wurden in die Studie eingeschlossen. Die Ratten wurden mit Ketamin (120 mg/kg) und Rompun (10 mg/kg) narkotisiert. Eine der Versuchsgruppen erhielt 0,1 µg/kg Körpergewicht Aranesp einmalig am Tag vor Induktion des akuten Nierenversagens. Als Vergleich diente eine Gruppe von Versuchstieren, denen jeweils zeitgleich Kochsalz s.c. injiziert wurde. Mittels Setzen einer Arterienklemme auf die rechte Nierenarterien wurde der Blutfluss in die Niere für 45 Minuten unterbrochen. In dieser Zeit wurde linksseitig eine Nephrektomie durchgeführt. Eine Scheinoperation wurde bei einer weiteren Kontrollgruppe durchgeführt. Hierbei wurde das Abdomen eröffnet, die linke Nierenarterie frei-präpariert, jedoch die Blutversorgung nicht unterbrochen und die kontralaterale rechte Niere entnommen. Alle Tiere wurde für 60 min narkotisiert und 24 h nach Operation getötet. Die 45-minütige Ischämie mit nachfolgender Reperfusion der verbleibenden rechten Niere führte in den Kochsalzbehandelten Tieren zu einem massiven akuten Nierenfunktionsverlust. Dieser reflektiert sich in einem Serum-Kreatinin-Wert, der 24 h nach der Ischämie-Reperfusion 7-mal größer ist als der Wert vor der Ischämie-Reperfusion (p < 0,05). Demgegenüber zeigten die mit dem Erythropoietin-Analog Aranesp behandelten Tiere nur einen vierfachen Anstieg der Serum-Kreatinin-Werte einen Tag nach Induktion des Ischämie-Reperfusionsschadens. Bei den linksseitig nephrektomierten Tieren mit scheinoperierter rechter Niere kam es zu keinem Anstieg der Retentionswerte. Die Figur zeigt die Kreatinin-Konzentration im Serum von EPO-behandelten Tieren (IR + EPO), NaCl-behandelten Tieren (IR) und scheinoperierten Tieren (Schein-OP) vor Ischämie-Reperfusions (IR)-Verletzung und 24 Stunden danach. Aus der Figur geht hervor, dass die Serum-Kreatinin-Konzentration bei den Aranesp-behandelten Tieren 24 Stunden nach Ischämie-Reperfusions-Verletzung gegenüber der Kontrolle ohne (NaCl-Behandlung) fast halbiert ist.

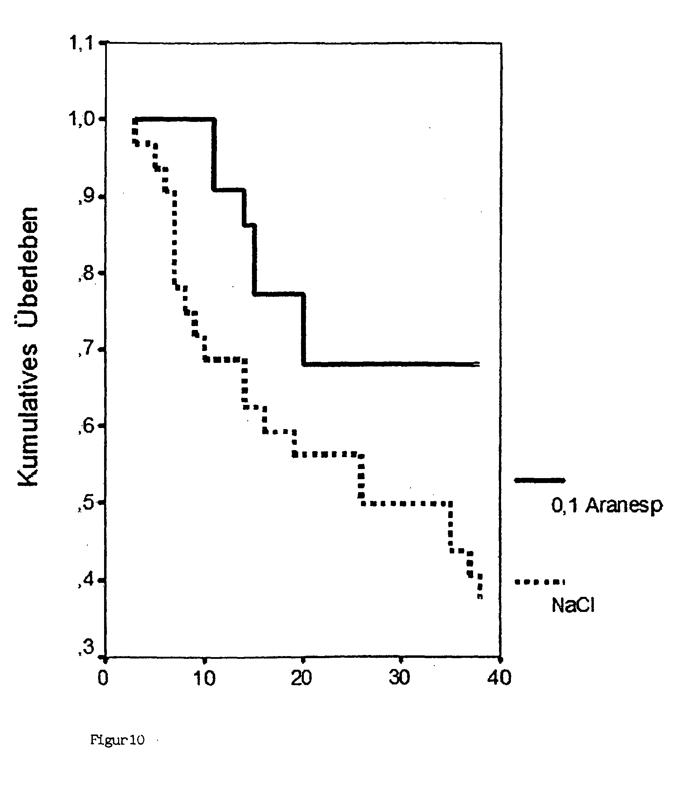

Figur 10 zeigt die Kaplan-Mayer-Überlebenskurven von zwei Versuchsgruppen nach Induktion einer chronischen Niereninsuffizienz, die entweder mit Aranesp oder NaCl behandelt wurden. 8 Wochen alte Spraque Dawley-Ratten wurden in die Studie eingeschlossen. Die Ratten wurden mit Ketamin (120 mg/kg) und Rompun (10 mg/kg) narkotisiert. Ihnen wurde am Tag 0 die rechte Niere entnommen, welche sofort nach Entnahme für eine histologische Untersuchung in Formalin fixiert wurde. Bei der linken Niere wurden die Segmentarterien, welche den oberen und unteren Nierenpol versorgen, ligiert. Dadurch kommt es zu einem Niereninfarkt der entsprechenden Nierenareale und nur das mittlere Nierendrittel bleibt funktionell erhalten. Einmal wöchentlich erhielten die Ratten Aranesp (0,1 µg/kg Körpergewicht) oder NaCl s.c. gespritzt. Die mit dem Erythropoietin-Analog Aranesp behandelten Tiere weisen einen signifikanten Überlebensvorteil gegenüber den kochsalzbehandelten Tieren auf (p = 0,027;Log Rank-Test).

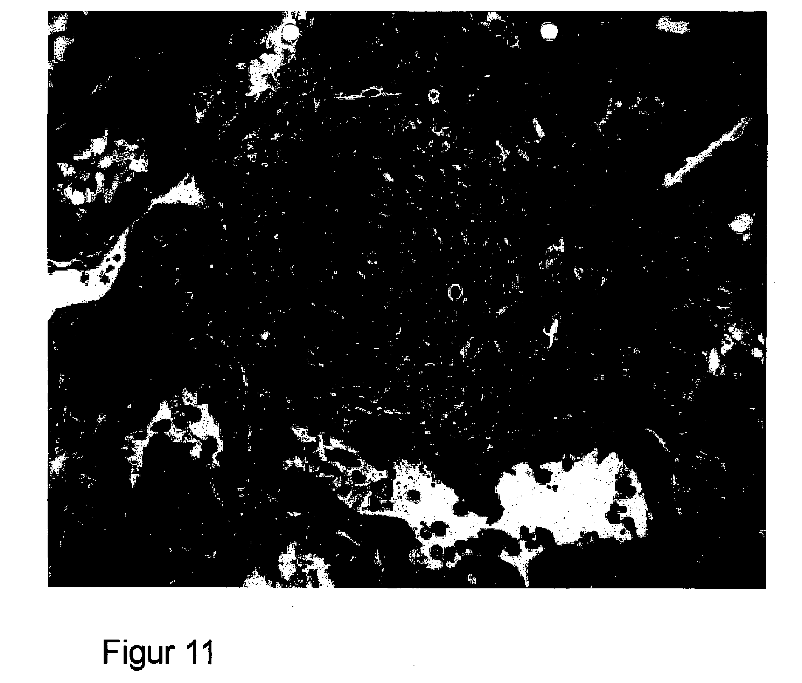

Die Figuren 11 - 18 zeigen lichtmikroskopische Nierenschnitte 6 Wochen nach Induktion einer chronischen Niereninsuffizienz von zwei Versuchsgruppen, die entweder mit Aranesp oder NaCl behandelt wurden und deren Kaplan-Mayer-Überlebenskurven in Figur 10 dargestellt sind.

Figur 11 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt eine mittelgroße präglomeruläre Arterie mit charakteristischer zwiebelschalenartiger Gefäßwand-Proliferation bei schwerer hypertensiver Schädigung, einer sogenannten malignen Nephrosklerose mit Endarteriitis Fahr.

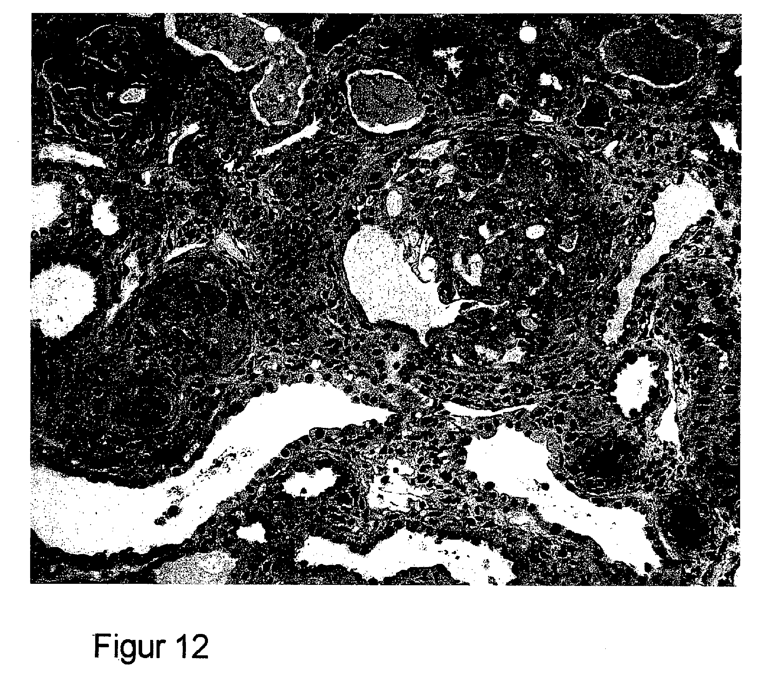

Figur 12 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt floride fokalsegmentale Glomerulosklerose als sogenannte proliferative FSGS (Glomerulum rechts). Das andere Glomerulum (links) zeigt ischämischen Kollaps des Schlingenkonvolutes. Am unteren des Bildes ist ein kleines Gefäß mit schwerem Endothelschaden zu sehen. Die beobachteten histologischen Veränderungen entsprechen einem hypertensiven Organschaden beziehungsweise Veränderungen im Rahmen einer Überlastungsnephropatie nach 5/6 Nephrektomie.

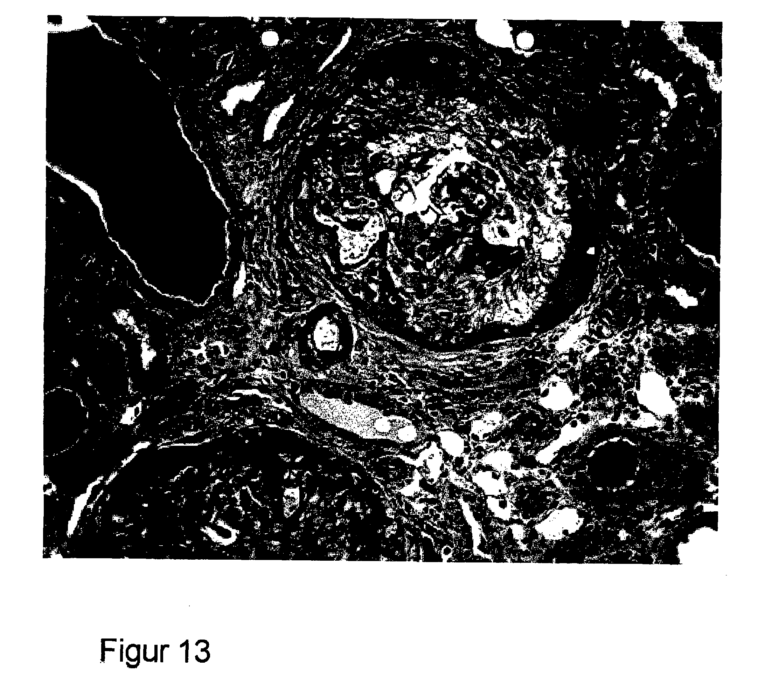

Figur 13 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt eine nahezu vollständige Sklerose beziehungsweise Destruktion eines kompensatorisch vergrößerten Glomerulums mit ausgeprägter Hyalinose beziehungsweise fibrinoider Nekrose der dazugehörigen afferenten Arteriole.

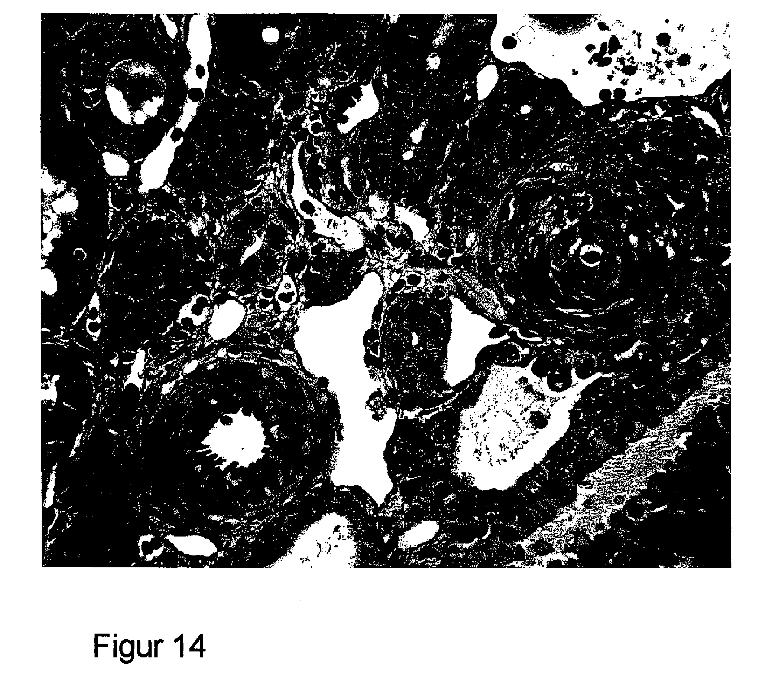

Figur 14 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt eine kleine präglomeruläre Arterie mit charakteristischer zwiebelschalenartiger Gefäßwandproliferation und Wandnekrose bei schwerer hypertensiver Schädigung, einer sogenannten malignen Nephrosklerose (vergleiche Bild rechts). Links ist eine regelrechte (noch) nicht geschädigte Arteriole zu sehen.

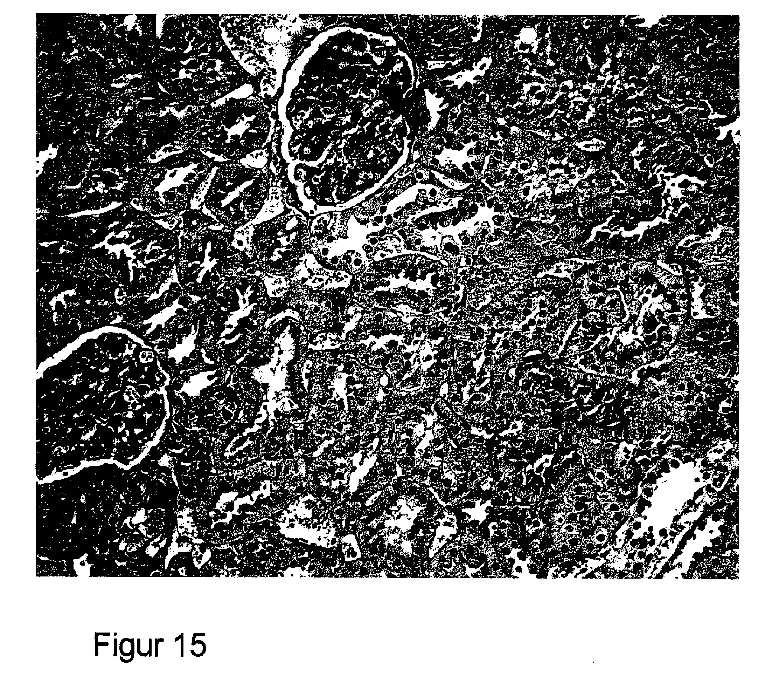

Figur 15 zeigt die histologischen Veränderungen bei einer Spraque-Dawley-Ratte mit chronischer Niereninsuffizienz nach jeweils einmal wöchentlicher Aranesp (EPO)-Behandlung (0,1 mg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß. Im Tubulointerstitium ist kein pathologischer Befund festzustellen.

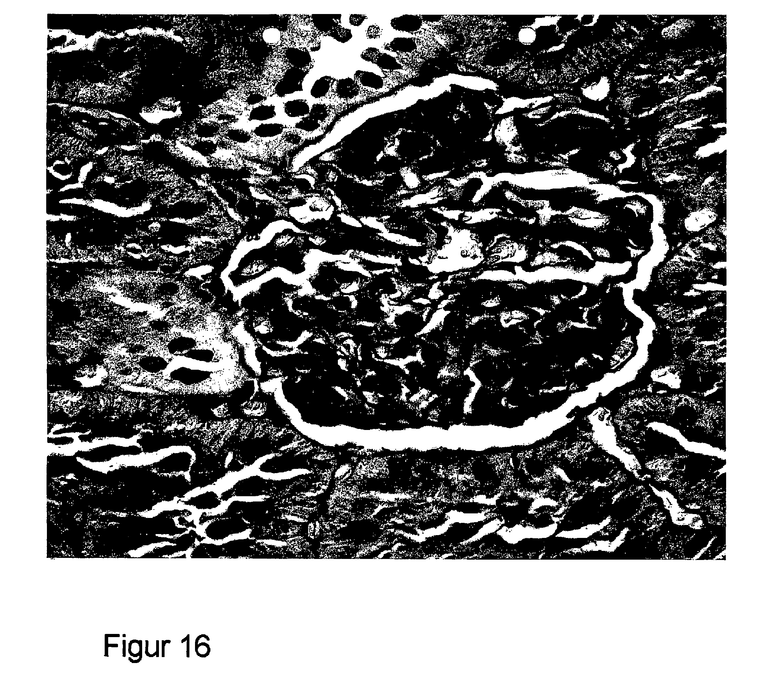

Figur 16 zeigt die histologischen Veränderungen bei einer Spraque-Dawley-Ratte mit chronischer Niereninsuffizienz nach jeweils einmal wöchentlicher Aranesp (EPO)-Behandlung (0,1 mg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß (630-fache Vergrößerung). Im Tubolointerstitium ist kein pathologischer Befund festzustellen.

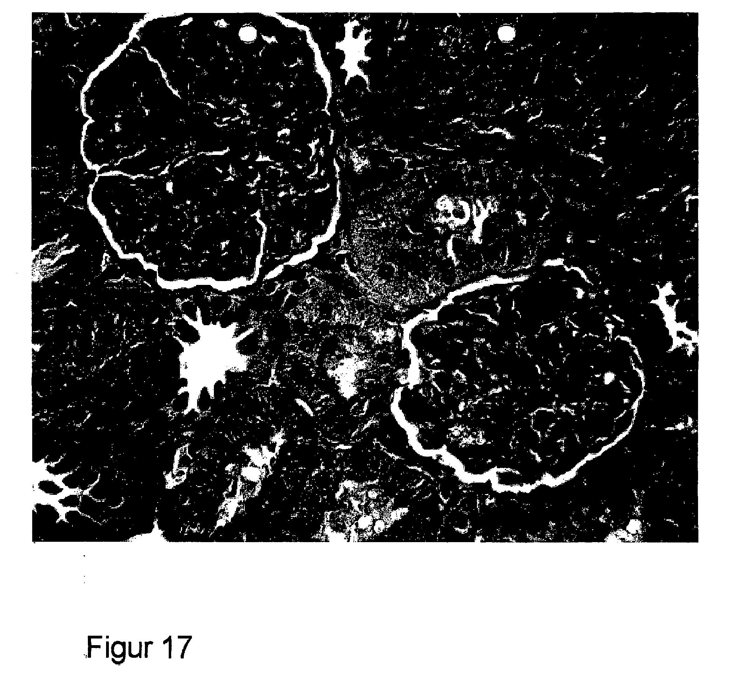

Figur 17 zeigt die histologischen Veränderungen bei einer Spraque-Dawley-Ratte mit chronischer Nieren-insuffizienz nach jeweils einmal wöchentlicher Aranesp (EPO)-Behandlung (0,1 mg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß. Im Tubolointerstitium ist kein pathologischer Befund festzustellen.

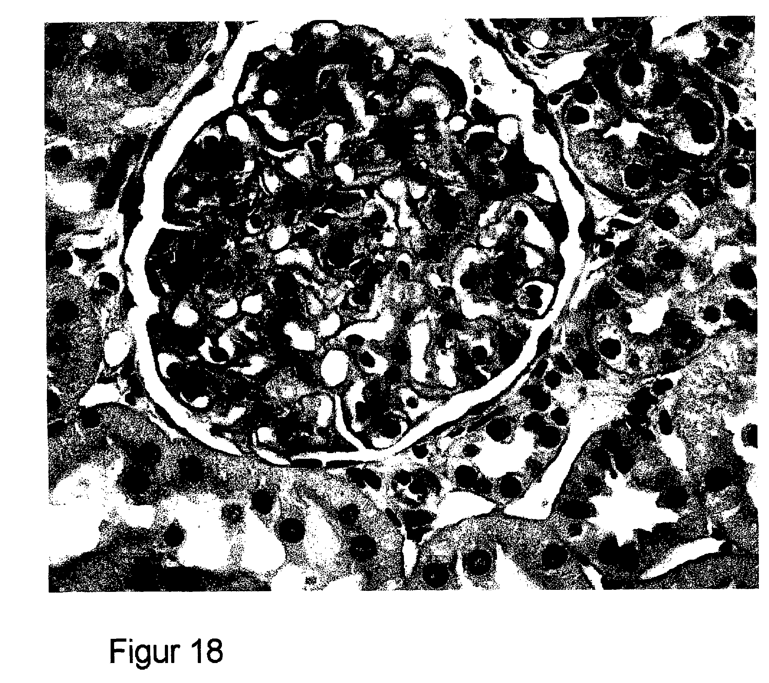

Figur 18 zeigt die histologischen Veränderungen bei einer Spraque-Dawley-Ratte mit chronischer Niereninsuffizienz nach jeweils einmal wöchentlicher Aranesp (EPO)-Behandlung (0,1 mg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß (630-fache Vergrößerung). Im Tubolointerstitium ist kein pathologischer Befund festzustellen.

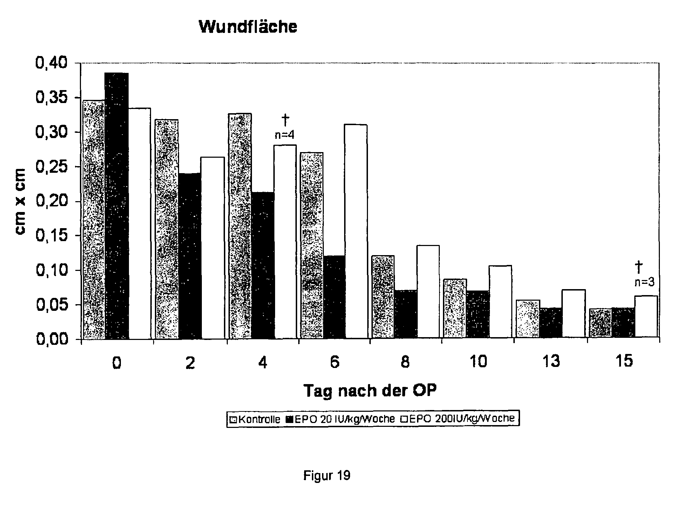

Figur 19 zeigt die Wirkung von EPO auf den Wundheilungsprozess.

### Beispiel 1

### Wirkung von EPO bei Patienten mit renaler Anämie

Die Wirkung von Erythropoietin bei Patienten mit renaler Anämie (Hb < 10,5 g/dl) als Folge von Nierenkrankheit im Endstadium (präterminale Niereninsuffizienz; Creatinin-Clearance < 35 ml/min) wurde untersucht. 11 Patienten wurden über einen Zeitraum von mindestens 8 Wochen mit Erythropoietin in wöchentlichen Dosen von durchschnittlich 5000 IU rhE-PO (rekombinantes humanes Erythropoietin) intravenös beziehungsweise subkutan behandelt. Nach Erythropoietin-Behandlung wurden die endothelialen Vorläuferzellen im Blut der Patienten über einen Zeitraum von 20 Wochen untersucht, wobei nach 0, 2, 4, 6 und 8 Wochen endotheliale Vorläuferzellen bezüglich ihrer Anzahl als auch ihres Differenzierungsstatus mittels Durchflusszytometrie und eines Kulturtestes analysiert wurden.

Bei zirkulierenden peripheren Blutstammzellen (CPBSC) handelt es sich um eine kleine Zellpopulation, die sowohl das CD34-Antigen als auch das CD45-Antigen exprimieren. Auf der Basis der ISHAGE-Richtlinien wurde ein Test zur Bestimmung der Anzahl von CPBSC mittels Durchflusszytometrie entwickelt (Sutherland et al., J. Hematother., 5 (1996), 213-226). Unter Verwendung dieses Tests wurden so-wohl dies Expressionsmuster von CD34- und CD45-Zellen als auch die Morphologie der Stammzellen bestimmt. Auf diese Weise wurde sowohl die absolute Anzahl von CPBSC pro µl als auch der prozentuale Anteil von CPBSC an der Gesamtleukozytenzahl bestimmt.

Figur 1 zeigt die Ergebnisse einer FACS-Analyse zirkulierender CD34+-Stammzellen auf der Basis der ISHAGE-Richtlinien.

Figur 2 zeigt die Anzahl der mittels FACS-Analyse ermittelten CD34+-Stammzellen über einen Zeitraum von 8 Wochen.

### Zellkultur-Test

Mononucleäre periphere Blutzellen (PBMCs) wurden mittels Dichtezentrifugation über Ficoll aus menschlichen Blutproben entsprechend dem in Asahara, Science, 275 (1997), 964-967, beschriebenen Verfahren isoliert. Die Zellen wurden auf Kulturplatten mit Fibronectin ausplattiert und in EC-Basalmedium gehalten. EC-Basalmedium besteht aus EBM-2-Basalmedium (Fa. Clonetics) und EGM-2 Quots (hEGF; GA-1000 (Gentamicin, Amphotericin-B) FBS, VEGF, hFGF-B (w/heparin), R3-IGF-1, Ascorbic Acid, Heparin). Nach 4-tägiger Kultivierung wurden nicht-adhärente Zellen durch Waschen der Platten entfernt. Die verbleibenden adhärenten Zellen wurden mit Trypsin behandelt und erneut ausgesät. Anschließend wurden sie für weitere 3 Tage kultiviert. Zellen mit endothelialem Phänotyp wurden durch Positivfärbung bezüglich zwei unterschiedlicher Endothelialmarker am Tag 7 nach Isolation identifiziert. Dabei handelt es sich um Dil-markiertes acetyliertes Lipoprotein geringer Dichte (acLDL-Dil) und Ulex europaeus-Aglutinin-1 (UEA-1). Die Ergebnisse dieser Untersuchung sind in Figur 3 dargestellt.

Die Ergebnisse zeigen, dass Erythropoietin endotheliale Vorläuferzellen mobilisieren und die Anzahl zirkulierender endothelialer Vorläuferzellen erhöhen kann. Dabei werden funktionelle Defizite, die unter bestimmten pathologischen Zuständen wie renaler Anämie auftreten, ausgeglichen. Diese Ergebnisse sind in Figur 4 dargestellt

Mittels Durchflusszytometrie wurde ermittelt, dass bei Patienten mit Nierenkrankheit im Endstadium die Anzahl zirkulierender CD34+-Stammzellen der Anzahl zirkulierender CD34+-Stammzellen im Blut von gesunden Probanden entspricht. Nach Beginn der Erythropoietin-Behandlung erhöht sich die Anzahl CD34+-Stammzellen im Blutkreislauf signifikant um mehr als 50 %. Unter Verwendung des Zellkulturtestes wurde bestimmt, dass nach Behandlung mit Erythropoietin die Anzahl der Zellen, die einen endothelialen Phänotyp entwickeln, dramatisch ansteigt. In einem funktionellen Zellkulturtest erhöhte sich die stark beeinträchtigte Fähigkeit von endothelialen Vorläuferzellen um das mehr als 3-fache.

### Beispiel 2

### Verbesserte Wundheilung durch den systemischen Einsatz von rhE-PO

FVB/N-Mäuse wurden durch Inhalationsanästhesie mit Isofloran narkotisiert. Die Behaarung der beiden Hinterläufe wurde mit Hilfe einer Enthaarungslotion entfernt und mit 70%igen Alkohol desinfiziert. Mittels einer sterilen, 4 mm-Einweg-Biopsiegewebestanze wurde jeweils eine Hautwunde auf der rechten Flanke der Mäuse gesetzt. Die gegenüberliegende Seite diente als interne Kontrolle. Einmalig wurde eine postoperative antibiotische Abschirmung mit Penicillin G (20000 Einheiten/kg) durchgeführt. Über den gesamten Untersuchungszeitraum erfolgten einmalig wöchentliche subkutane Injektionen von rekombinantem humanen Erythropoietin-Analog Aranesp (0,1 µg/kg Körpergewicht) über den gesamten Studienzeitraum. Die Behandlung begann sieben Tage vor Entnahme der Gewebestanze. Die Ergebnisse sind in Figur 8 dargestellt. Sie zeigen, dass die Verabreichung von EPO den Wundheilungsprozess erheblich beschleunigt.

Figur 19 zeigt die Wirkung von Erythropoietin auf die Wundheilung. Die Figur zeigt, dass bei Behandlung einer standardisierten Hautwunde, die bei Mäusen mittels einer Gewebestanze gesetzt worden war, mit niedrig dosiertem Erythropoietin (20IU EPO/kg/Woche) die Wunde bereits nach sieben bis acht Tagen vollständig verschlossen war, während bei Behandlung der Wunde mit physiologischer Kochsalzlösung (Saline) die Wunde erst nach dreizehn bis vierzehn Tagen vollständig verschlossen war. Die Behandlung der Versuchstiere mit hoch dosiertem Erythropoietin (200IU EPO/kg/Woche) konnte keine Beschleunigung der Wundheilung im Vergleich zur Kontrollgruppe beobachtet werden. Zwei der mit der hohen Dosis Erythropoietin behandelten Versuchstiere verstarben im Beobachtungszeitraum. Die Behandlung mit Erythropoietin beziehungsweise physiologischer Kochsalzlösung begann nach Setzen der Hautwunde am Operationstag. Rekombinantes humanes Erythropoietin wurde einmalig wöchentlich mittels einer s.c. (subcutan)-Injektion (20 IU/kg EPO bzw. 200 IU/kg EPO) verabreicht (je Gruppe n = 5).

### Beispiel 3

### Verminderung der Progression der chronischen Niereninsuffizienz durch Erythropoietin-Behandlung

Acht Wochen alte Spraque-Dawley-Ratten wurden mit Ketamin (120 mg/kg) und Rompun (10 mg/kg) narkotisiert. Den Ratten wurden am Tage 0 die rechte Niere entnommen, welche sofort nach Entnahme für eine histologische Untersuchung in Formalin fixiert wurde. Bei der linken Niere wurden die Segmentarterien, welche den oberen und unteren Nierenpol versorgen, ligiert. Dadurch kommt es zu einem Niereninfarkt der entsprechenden Nierenareale, wobei nur das mittlere Nierendrittel funktionell erhalten bleibt. Einmal wöchentlich erhielten die Ratten das Erythropoietin-Analog Aranesp in einer Dosis von 0,1 µg/kg Körpergewicht oder zur Kontrolle NaCl subkutan (s.c.) gespritzt.

Figur 10 zeigt die Kaplan-Mayer-Überlebenskurve beider Versuchsgruppen. Die Aranesp-behandelten Tiere haben ein deutlich verbessertes Überleben verglichen mit den Kochsalz-behandelten Kontrolltieren.

In den Figuren 15 - 18 ist gezeigt, dass nach Behandlung mit Erythropoietin das Nierengewebe keine pathologischen Veränderungen zeigt, während nach Behandlung mit NaCl schwere pathologische Veränderungen sichtbar sind (vergleiche Figuren 8-11). Weitere histologische Untersuchungen ergaben, dass bei Aranesp-behandelten Tieren eine deutlich höhere Gefäßdichte (CD31) zu beobachten ist als bei Kochsalz-behandelten Tieren (Daten nicht gezeigt).

### Beispiel 4

### Verminderung der Progression akuter Niereninsuffizienz

Für diese Untersuchung wurden Spraque-Dawley-Ratten mit einem Körpergewicht von 250 bis 300 g einge-setzt. Eine der Versuchsgruppen erhielt einmalig am Tag vor Induktion des akuten Nierenversagens Aranesp in einer Dosis von 0,1 µg/kg Körpergewicht. Die Ratten wurden mit Ketamin (120 mg/kg Körpergewicht) und Rompun (10 mg/kg) narkotisiert. Als Vergleich diente eine Gruppe von Versuchstieren, denen jeweils zeitgleich Kochsalz s.c. injiziert wurde. Der Blutfluss in der Niere wurde für 45 Minuten unterbrochen, indem eine Arterienklemme auf die rechte Nierenarterie gesetzt wurde. In dieser Zeit wurde linksseitig eine Nephrektomie durchgeführt. Bei einer weiteren Kontrollgruppe wurde eine Scheinoperation durchgeführt. Hier wurde das Abdomen eröffnet, die linke Nierenarterie frei-präpariert, jedoch die Blutversorgung nicht unterbrochen, und die kontralaterale rechte Niere entnommen. Alle Tiere wurden für einen Zeitraum von 60 Minuten narkotisiert und 24 Stunden nach Operation getötet.

Die 45-minütige Ischämie mit nachfolgender Reperfusion der verbleibenden rechten Niere führte in den kochsalzbehandelten Tieren zu einem massiven akuten Nierenfunktionsverlust. Dieser fand seinen Ausdruck in einem um den Faktor 7 angestiegenen Serumkreatinin-Wert (p < 0,05). Demgegenüber zeigten die mit dem Erythropoietin-Analog Aranesp behandelten Tiere nur einen vierfachen Anstieg des Serumkreatinin-Wertes einen Tag nach Induktion des Ischämie-Reperfusions-Schadens. Bei den linksseitig nephrektonierten Tieren mit scheinoperierter rechter Niere kam es zu keinem Anstieg der Retentionswerte. Die Ergebnisse sind in Figur 9 dargestellt.

### Beispiel 5

### Verminderte Differenzierungsfähigkeit von endothelialen Vorläuferzellen bei Patienten mit eingeschränkter Nierenfunktion

Bei 46 urämischen Patienten sowie 46 alters- und geschlechtsgematchten gesunden Kontrollprobanden wurde der Differenzierungsstatus endotheliale Vorläuferzellen durch einen Kulturtest analysiert. Es wurde dabei überraschenderweise festgestellt, dass die Zahl der endothelialen Vorläuferzellen in diesem Differenzierungsassay bei urämischen Patienten signifikant gegenüber den gesunden Kontrollen verringert ist (Figur 7). Isoliert man mononukleäre Zellen eines gesunden Probanden und kultiviert diese in Gegenwart von Serum eines urämischen Patienten, so verringert sich die Differenzierungsfähigkeit der Zellen zu endothelialen Vorläuferzellen analog (Figur 6).

### Beispiel 6

### Stimulation der Differenzierungsfähigkeit endothelialen Vorläuferzellen bei gesunden Probanden

4 gesunde junge Männer wurden über einen Zeitraum von 8 Woche mit 30IU Epoetin beta je Kilogramm Körpergewicht einmal in der Woche behandelt. Dabei wurde die Differenzierungsfähigkeit der endotheialen Vorläuferzellen in einem Kulturassay vor der Behandlung der Probanden mit rhEPO sowie wöchentlich nach 1, 2, 3, 4, 5, 6 und 7 Wochen nach Behandlung der Probanden mit rhEPO bestimmt anhand ihrer Adhäsionsfähigkeit und der beiden Marker acLDL und UEA. Es wurde dabei eine relative Erhöhung der EPCs um mehr als 50% beobachtet.

## Patentansprüche

1. Verwendung von Erythropoietin und/oder Derivaten zur Herstellung einer pharmazeutischen Zusammensetzung enthaltend eine Dosis von 0,001 bis 90 IU/kg Körpergewicht und Woche, vorzugsweise 0,05 bis 50 IU/kg Körpergewicht/Woche zur Prophylaxe oder Behandlung von Krankheiten, wobei das Erythropoietin in dieser Dosis geeignet und bestimmt ist für die Prophylaxe oder Behandlung eines menschlichen oder tierischen Patienten mit a) mindestens einer Dysfunktion endothelialer Vorläuferzellen, b) mindestens einem cardiovaskulären Risikofaktor wie Hypertonie, Hypercholestrinemie, erhöhte Asymmetrisches Dimethylarginin (ADMA) -Werte, erhöhte Insulinresistenz oder Hyperhomocytenamie und c) mindestens einem Endorganschaden wie linksventrikuläre Hypertrophie, Mikroalbuminurie, kognitive Dysfunction, Zunahme der Intima-Media-Dicke in der A. carotis, Proteinurie oder einer glomeruläre Filtrationsrate < 80 ml/min, vorzugsweise 30 bis 80 ml/min.

2. Verwendung von Erythropoietin und/oder Derivaten zur Herstellung einer pharmazeutischen Zusammensetzung enthaltend eine Dosis von 0,001 bis 90 IU/kg Körpergewicht und Woche, vorzugsweise 0,05 bis 50 IU/kg Körpergewicht/Woche, wobei das Erythropoietin in dieser Dosis geeignet und bestimmt ist für die kosmetische Behandlung des menschlichen oder tierischen Körpers, insbesondere zur Behandlung von Faltenbildung, zur Stärkung des Bindegewebes, zum Schutz und zur Straffung der Haut, zum Schutz vor schädigenden Umwelteinwirkungen, zur Behandlung von Altersflecken, zur Beschleunigung der Reepithelialisierung, zur Beschleunigung des Haarwuchses und/oder als Make-up Unterlage.

3. Verwendung von Erythropoietin und/oder Derivaten zur Herstellung einer pharmazeutischen Zusammensetzung enthaltend eine Dosis von 0,001 bis 90 IU/kg Körpergewicht und Woche, vorzugsweise 0,05 bis 50 IU/kg Körpergewicht/Woche zur Herstellung einer kosmetischen Präparation, insbesondere zur topischen Applikation, wobei das Erythropoietin in dieser Dosis geeignet und bestimmt ist für die kosmetische Behandlung des menschlichen oder tierischen Körpers, insbesondere zur Behandlung von Faltenbildung, zur Stärkung des Bindegewebes, zum Schutz und zur Straffung der Haut, zum Schutz vor schädigenden Umwelteinwirkungen, zur Behandlung von Altersflecken, zur Beschleunigung der Reepithelialisierung, zur Beschleunigung des Haarwuches, und/oder als Make-up Unterlage.

4. Verwendung von Erythropoietin und/oder Derivaten zur Herstellung einer pharmazeutischen Zusammensetzung enthaltend eine Dosis von 0,001 bis 90 IU/kg Körpergewicht/Woche, vorzugsweise 0,05 bis 50 IU/kg Körpergewicht/Woche und/oder einer Mischung von endotelialen Vorläuferzellen mit mindestens einer zelltherapeutisch einsetzbaren Zellpopulation, wobei das Erythropoietin in dieser Dosis geeignet und bestimmt ist für die Regeneration von Gewebe und Gefäßen in einem menschlichen oder tierischen Körper, und wobei die Mischung vor der Applikation in vitro mit Erythropoietin in Kontakt gebracht worden ist.

5. Verwendung von Erythropoietin und/oder Derivaten zur Herstellung einer pharmazeutischen Zusammensetzung enthaltend eine Dosis von 0,001 bis 90 IU/kg Körpergewicht und Woche, vorzugsweise 0,05 bis 50 IU/kg Körpergewicht/Woche und/oder einer Mischung von endotelialen Vorläuferzellen mit mindestens einer zelltherapeutisch einsetzbaren Zellpopulation, wobei das Erythropoietin in dieser Dosis geeignet und bestimmt ist für die Regeneration von Gewebe und Gefäßen in einem menschlichen oder tierischen Körper, und wobei die Mischung vor, nach oder gleichzeitig mit der Applikation der Mischung verabreicht wird

6. Verwendung von Erythropoietin und/oder Derivaten zur Herstellung einer pharmazeutischen Zusammensetzung oder eines Kits enthaltend eine Dosis von 0,001 bis 90 IU/kg Körpergewicht/Woche, vorzugsweise 0,05 bis 50 IU/kg Körpergewicht/Woche und/oder mindestens einem chemischen, thermischen, mechanischen oder biologischen Agens, insbesondere einem pharmakologischen Wirkstoff, für die Herstellung einer pharmazeutischen Zusammensetzung oder einen Kit, enthaltend Erythropoietin in dieser Dosierung und das mindestens eine chemische, thermische, mechanische oder biologische Agens, für die Prophylaxe oder Behandlung von Krankheiten, wobei das Erythropoietin in dieser Dosis geeignet und bestimmt ist für die sequenzielle, zeitlich aufeinanderfolgende oder gleichzeitige Applikation des Erythropoietin mit dem mindestens einen chemischen, thermischen, mechanischen oder biologischen Agens.

7. Verwendung von Erythropoietin nach Anspruch 6, wobei es sich bei den mechanischen Agenzien um Endoprothesen, bevorzugt um Implantationskörper für den Zahn, Knochen oder Band/Sehnenersatz handelt.

8. Verwendung von Erythropoietin nach Anspruch 6, wobei es sich bei den biologischen Agenzien um solide Organe, wie Leber, Niere, Herz, Pankreas, Haut oder Haarimplantate handelt.

9. Verwendung von Erythropoietin und/oder Derivaten zur Herstellung einer pharmazeutischen Zusammensetzung enthaltend eine Dosis von 0,001 bis 90 IU/kg Körpergewicht und Woche, vorzugsweise 0,05 bis 50 IU/kg Körpergewicht/Woche, wobei das Erythropoietin in dieser Dosis geeignet und bestimmt ist für die Prophylaxe oder Behandlung von Krankheiten, wobei die Krankheit Hypercholesterinämie, Diabetes mellitus, Insulinresistenz, Endothelvermittelten chronischen Entzündungserkrankungen, Endotheliose einschließlich Retikuloendotheliose, Atherosklerose, altersbedingter Herz-Kreislauf-Erkrankung, ischämischen Erkrankungen der Extremitäten, Präeklampsie, Raynaud-Krankheit, Lebererkrankungen wie Hepatitis, Leberzirrhose, akutes oder chronisches Leberversagen, Knochen- und Knorpelerkrankungen oder -verletzungen, Schleimhauterkrankungen oder -verletzungen, insbesondere im gastrointestinalen Trakt, Morbus Crohn, Colitis ulcerosa, schwangerschaftsinduzierter Hypertonie, chronischer oder akuter Niereninsuffizienz, insbesondere terminaler Niereninsuffizienz, Nierenfunktionseinschränkungen mit glomerulären Filtrationsraten von 30 bis 80 ml/min, Mikroalbuminurie, Proteinurie oder Wunden und Folgeerkrankungen davon ist.

10. Verwendung von Erythropoietin und/oder Derivaten zur Herstellung einer pharmazeutischen Zusammensetzung enthaltend eine Dosis von 0,001 bis 90 IU/kg Körpergewicht/Woche, vorzugsweise 0,05 bis 50 IU/kg Körpergewicht/Woche zur Herstellung eines Kits, enthaltend Erythropoietin, endotheliale Vorläuferzellen und mindestens eine zelltherapeutisch einsetzbare Zellpopulation, wobei das Erythropoietin vorzugsweise in niedriger Dosierung vorliegt.

11. Verwendung nach einem der Ansprüche 1 bis 10, wobei die pharmazeutische Zusammensetzung zur Stimulation der physiologischen Mobilisierung endothelialer Vorläuferzellen, der Proliferation endothelialer Vorläuferzellen, der Differenzierung endothelialer Vorläuferzellen zu Endothelzellen und/oder der Migration endothelialer Vorläuferzellen in Richtung eines angiogenen oder vaskulogenen Stimulus dient.

12. Verwendung nach Anspruch 1 bis 11, wobei die Fähigkeit sich differenzierender endothelialer Vorläuferzellen zur Adhäsion gesteigert wird.

13. Verwendung nach Anspruch 1 bis 12, wobei die Stimulation endothelialer Vorläuferzellen zur Bildung von Endothelgewebe führt.

14. Verwendung nach einem der Ansprüche 1 bis 13, wobei die Stimulation der endothelialen Vorläuferzellen zur Ausbildung neuer Blutgefäße führt.

15. Verwendung von Erythropoietin in einer geringen Dosierung von 0,001 bis 90 IU/kg Körpergewicht/Woche zur Therapie von Krankheitszuständen oder Krankheiten des menschlichen oder tierischen Körpers, die im Zusammenhang mit einer Dysfunktion von endothelialen Vorläuferzellen stehen.

16. Verwendung nach Anspruch 15, wobei die Dysfunktion der endothelialen Vorläuferzellen in ihrer gestörten Fähigkeit zur Proliferation, ihrer gestörten Fähigkeit zur Differenzierung zu Endothelzellen, ihrer gestörten Fähigkeit zur Adhäsion und/oder ihrer gestörten Fähigkeit zur Migration in Richtung eines vaskulogenen oder angiogenen Stimulus besteht.

17. Verwendung nach Anspruch 15 oder 16, wobei die Dysfunktion von endothelialen Vorläuferzellen die Bildung von Endothelgewebe und/oder Blutgefäßen beeinträchtigt oder verhindert.

18. Verwendung nach einem der Ansprüche 15 bis 17, wobei die Dysfunktion von endothelialen Vorläuferzellen pathogen bedingt ist.

19. Verwendung nach einem der Ansprüche 15 bis 18, wobei es sich bei den Krankheitszuständen oder Krankheiten, die mit einer Dysfunktion von endothelialen Vorläuferzellen im Zusammenhang stehen, um Hypercholesterinämie, Diabetes mellitus, Insulinresistenz, Endothelvermittelte chronische Entzündungserkrankungen, Endotheliose einschließlich Retikuloendotheliose, Atherosklerose, altersbedingte Herz-Kreislauf-Erkrankung, ischämische Erkrankungen der Extremitäten, Präeklampsie, Raynaud-Krankheit, Lebererkrankungen wie Hepatitis, Leberzirrhose, akutes oder chronisches Leberversagen, Knochen- und Knorpelerkrankungen oder - verletzungen, Schleimhauterkrankungen oder -verletzungen, insbesondere im gastrointestinalen Trakt, Morbus Crohn, Colitis ulcerosa, schwangerschaftsinduzierte Hypertonie, chronische oder akute Niereninsuffizienz, insbesondere terminale Niereninsuffizienz, Nierenfunktionseinschränkungen mit glomerulären Filtrationsraten von 30 bis 80 ml/min, Mikroalbuminurie, Proteinurie, erhöhte ADMA- Werte, oder Wunden und/oder Folgeerkrankungen davon handelt.

20. Verwendung von Erythropoietin in einer geringen Dosis, insbesondere von 0,001 bis 90 IU/kg Körpergewicht/Woche zur Therapie von Hypercholesterinämie, Diabetes mellitus, Insulinresistenz, Endothelvermittelten chronischen Entzündungserkrankungen, Endotheliose einschließlich Retikuloendotheliose, Atherosklerose, altersbedingter Herz-Kreislauf-Erkrankung, ischämischen Erkrankungen der Extremitäten, Präeklampsie, Raynaud-Krankheit, Lebererkrankungen wie Hepatitis, Leberzirrhose, akutes oder chronisches Leberversagen, Knochen- und Knorpelerkrankungen oder -verletzungen, Schleimhauterkrankungen oder -verletzungen, insbesondere im gastrointestinalen Trakt, Morbus Crohn, Colitis ulcerosa, schwangerschaftsinduzierter Hypertonie, chronischer oder akuter Niereninsuffizienz, insbesondere terminaler Niereninsuffizienz, Nierenfunktionseinschränkungen mit glomerulären Filtrationsraten von 30 bis 80 ml/min, Mikroalbuminurie, Proteinurie, erhöhte ADMA Werte oder Wunden und/oder Folgeerkrankungen davon.

21. Verwendung nach einem der Ansprüche 1 bis 20, wobei Erythropoietin pro Patient in einer Dosis von 0,001 bis 90 Einheiten/kg Körpergewicht/Woche verabreicht wird.

22. Verwendung nach Anspruch 21, wobei Erythropoietin pro Patient in einer Dosis von 0,05 bis 50 Einheiten/Woche verabreicht wird.

23. Verwendung nach einem der Ansprüche 1 bis 22, wobei die pharmazeutische Zusammensetzung sowohl zur parenteralen, insbesondere intravenösen, intramuskulären, intrakutanen oder subkutanen als auch topischen Verabreichung geeignet ist.

24. Verwendung nach Anspruch 23, wobei die pharmazeutische Zusammensetzung als Injektion oder Infusion vorliegt.

25. Verwendung nach einem der Ansprüche 1 bis 22, wobei die pharmazeutische Zusammensetzung zur pulmonalen Verabreichung geeignet ist.

26. Verwendung nach Anspruch 25, wobei die pharmazeutische Zusammensetzung als wässrige Lösung, nicht-wässrige Lösung oder Pulver vorliegt.

27. Verwendung nach Anspruch 25 oder 26, wobei die pharmazeutische Zusammensetzung in Form eines Aerosol-Präparates vorliegt.

28. Verwendung nach einem der Ansprüche 1 bis 22, wobei die pharmazeutische Zusammensetzung zur oralen Verabreichung geeignet ist.

29. Verwendung nach Anspruch 28, wobei die pharmazeutische Zusammensetzung als Lösung, Suspension, Emulsion oder Tablette vorliegt.

30. Verwendung nach einem der Ansprüche 1 bis 29, wobei die pharmazeutische Zusammensetzung mindestens einen weiteren Wirkstoff zur Stimulation endothelialer Vorläuferzellen enthält.

31. Verwendung nach Anspruch 30, wobei es sich bei dem weiteren Wirkstoff um VEGF, PIGF, GM-CSF, einen ACE-Hemmer, einen AT-1 Blocker, einen HMG-CoA-Reduktase-Inhibitor und/oder einen NO-Donator handelt.

32. Verwendung nach Anspruch 31, wobei der HMG-CoA-Reduktase-Inhibitor ein Statin wie Simvastatin, Mevastatin oder Atorvastatin, der ACE-Hemmer ein Wirkstoff wie Enalapril, Ramipril oder Trandolapril und/oder der AT-1 Blocker ein Wirkstoff wie Irbesartan, Lorsartan oder Olmesaratan ist.

33. Verwendung von Erythropoietin zur Herstellung eines transplantierbaren Endothelzell-Präparates.

34. Verwendung nach Anspruch 33, wobei Endothelzellen in vitro durch Kultivierung endothelialer Vorläuferzellen in Gegenwart von Erythropoietin in einer geringen Dosis, nämlich von 0,001 bis 90 IU/kg/Woche, hergestellt werden.

35. Verwendung nach Anspruch 33 oder 34, wobei die Kultivierung der endothelialen Vorläuferzellen in Gegenwart mindestens eines weiteren Wirkstoffes ausgewählt aus der Gruppe bestehend aus VEGF, PIGF, GM-CSF, einem ACE-Hemmer wie Enalapril, Ramipril oder Trandolapril, einem AT-1 Blocker wie Irbesartan, Lorsartan oder Olmesaratan, einem HMG-CoA-Reduktase-Inhibitor, insbesondere Simvastatin, Mevastatin oder Atorvastatin, und einem NO-Donator, insbesondere L-Arginin, erfolgt.

36. Verwendung von Erythropoietin in niedriger Dosierung, nämlich von 0,001 bis 90 IU/kg/Woche, zur Vorbehandlung und/oder Weiterbehandlung von Gewebe- oder Organtransplantaten.

37. Verwendung nach Anspruch 36, wobei die Vorbehandlung der Gewebe- oder Organtransplantate unter Verwendung isolierter endothelialer Vorläuferzellen erfolgt.

38. Verwendung von Erythropoietin zur Herstellung implantierbarer oder transplantierbarer zellhaltiger in vitro-Organ- oder Gewebesysteme, wobei die in vitro-Organ- oder Gewebesysteme vor Transplantation oder Implantation zur Induktion von Vaskulogenese und/oder Endothelzell-Bildung mit Erythropoietin behandelt werden.

39. Verwendung nach Anspruch 38, wobei die in vitro-Organ- oder Gewebesysteme endotheliale Vorläuferzellen enthalten.

40. Verwendung von Erythropoietin zur Herstellung von Gefäßprothesen oder Herzklappen, wobei die Gefäßprothesen oder Herzklappen mit Erythropoietin beschichtet werden.

41. Verwendung nach Anspruch 40, wobei die Beschichtung der Gefäßprothesen oder Herzklappen endotheliale Vorläuferzellen enthält.

42. Verwendung nach einem der Ansprüche 1 bis 41, wobei Erythropoietin menschliches oder tierisches Erythropoietin ist.

43. Verwendung nach Anspruch 42, wobei Erythropoietin ein Derivat, ein Analog, eine Modifikation oder ein Mutein von Erythropoietin ist.

44. Verwendung nach Anspruch 42 oder 43, wobei Erythropoietin aus menschlichem Urin, dem Urin oder Plasma von an aplastischer Anämie leidenden Patienten, Gewebekulturen von menschlichen Nierenkrebszellen, die Fähigkeit zur Bildung von menschlichem Erythropoietin aufweisenden menschlichen Lymphoblastenzellen oder einer durch Zellfusion einer menschlichen oder tierischen Zelllinie erhaltenen Hybridomkultur isoliert ist.

45. Verwendung nach Anspruch 42 oder 43, wobei Erythropoietin ein mittels DNA-Rekombinationstechniken hergestelltes Erythropoietin ist.

46. Pharmazeutische Zusammensetzung zur Stimulation endothelialer Vorläuferzellen, zur Stimulation der Bildung von Endothelgewebe, zur Stimulation von Vaskulogenese und/oder zur Behandlung von Krankheiten oder Krankheitszuständen, die im Zusammenhang mit einer Dysfunktion endothelialer Vorläuferzellen stehen, umfassend Erythropoietin und/oder ein Derivat, ein Analog, eine Modifikation oder ein Mutein davon als Wirkstoff sowie mindestens einen weiteren Wirkstoff ausgewählt aus der Gruppe bestehend aus VEGF, PIGF, GM-CSF, einem ACE-Hemmer wie Enalapril, Ramipril oder Trandolapril, einem AT-1 Blocker wie Irbesartan, Lorsartan oder Olmesaratan, einem HMG-CoA-Reduktase-Inhibitor und einem NO-Donator, vorzugsweise in einer geringen Dosis, insbesondere von 0,001 bis 90 IU/kg Körpergewicht/Woche.

47. Pharmazeutische Zusammensetzung zur Prophylaxe und/oder Therapie von Hypercholesterinämie, Diabetes mellitus, Insulinresistenz, Endothel-vermittelten chronischen Entzündungserkrankungen, Endotheliose einschließlich Retikuloendotheliose, Atherosklerose, altersbedingter Herz-Kreislauf-Erkrankung, ischämischen Erkrankungen der Extremitäten, Präeklampsie, Raynaud-Krankheit, Lebererkrankungen wie Hepatitis, Leberzirrhose, akutes oder chronisches Leberversagen, Knochen- und Knorpelerkrankungen oder - verletzungen, Band und Sehnenerkrankungen oder -verletzungen, Schleimhauterkrankungen oder -verletzungen, insbesondere im gastro-intestinalen Trakt, Morbus Crohn, Colitis ulcerosa, schwangerschaftsinduzierter Hypertonie, chronischer oder akuter Niereninsuffizienz, insbesondere terminaler Niereninsuffizienz, Nierenfunktionseinschränkungen mit glomerulären Filtrationsraten von 30 bis 80 ml/min, Mikroalbuminurie, Proteinurie, erhöhte ADMA-Werte oder Wunden und Folgeerkrankungen davon, umfassend Erythropoietin und/oder ein Derivat, ein Analog, eine Modifikation oder ein Mutein davon als Wirkstoff, vorzugsweise in einer geringen Dosis, insbesondere von 1 bis 90 IU/kg Körpergewicht/Woche.

48. Pharmazeutische Zusammensetzung nach Anspruch 47, zusätzlich umfassend mindestens einen weiteren Wirkstoff ausgewählt aus der Gruppe bestehend aus VEGF, PIGF, GM-CSF, einem ACE-Hemmer wie Enalapril, Ramipril oder Trandolapril, einem AT-1 Blocker wie Irbesartan, Lorsartan oder Olmesaratan, einem HMG-CoA-Reduktase-Inhibitor und einem NO-Donator.

49. Pharmazeutische Zusammensetzung nach Anspruch 46 oder 437, wobei der HMG-CoA-Reduktase-Inhibitor ein Statin wie Simvastatin, Mevastatin oder Atorvastatin, der ACE-Hemmer ein Wirkstoff wie Enalapril, Ramipril oder Trandolapril und/oder der AT-1 Blocker ein Wirkstoff wie Irbesartan, Lorsartan oder Olmesaratan ist.

50. Pharmazeutische Zusammensetzung nach Anspruch 46 oder 47, wobei der NO-Donator L-Arginin ist.

51. Verwendung von Erythropoietin nach Anspruch 1 bis 50, zur Herstellung einer pharmazeutischen Zusammensetzung, für die Prophylaxe oder Behandlung von Krankheiten, wobei das Erythropoietin oder/und die pharmazeutische Zusammensetzung geeignet und bestimmt ist für die morgendliche Applikation an einen menschlichen oder tierischen Körper in einem Zeitraum von 6:00 bis 10:00.

52. Kit, enthaltend Erythropoietin, endotheliale Vorläuferzellen und mindestens eine zelltherapeutisch einsetzbare Zellpopulation, wobei das Erythropoietin vorzugsweise in niedriger Dosierung vorliegt.

53. Verwendung von Erythropoietin und/oder Derivaten zur Herstellung einer pharmazeutischen Zusammensetzung oder eines Kits enthaltend eine Dosis von 0,001 bis 90 IU/kg Körpergewicht und Woche, vorzugsweise 0,05 bis 50 IU/kg Körpergewicht/Woche zur Prophylaxe oder Behandlung von Krankheiten des menschlichen oder tierischen Körpers, wobei das Erythropoietin in der genannten niedrigen Dosis geeignet und bestimmt ist, die Integration eines mechanischen oder biologischen Agens, insbesondere einer Endoprothese, insbesondere eines Implantats, zum Beispiel eines Zahnimplantats, Zahnersatz, eines Knochenimplantats, eines Knochenersatzes, insbesondere einer Gelenkprothese, eines Band/Sehnenersatzes, wie z.B. der Kreuzbänder oder eines soliden Organs in das Implantat oder die Endoprothese umgebende Körperstrukturen zu verbessern, insbesondere zu fördern und/oder zu beschleunigen.

54. Verwendung nach Anspruch 53, wobei das pharmazeutische Präparat oder der Kit zusätzlich ein Zelltherapeutikum enthält, insbesondere endotheliale Vorläuferzellen und/oder sonstige zelltherapeutisch einsetzbare Zellpopulation für die Regeneration von Geweben und Gefäßen.

55. Verwendung nach einem der Ansprüche 53 oder 54, wobei die Endoprothese aus Stahl, Keramik, Kunststoff oder einem sonstigen Matrix-Material ausgebaut ist.

56. Kit enthaltend Erythropoietin in einer Dosis von 0,001 bis 90 IU/kg Körpergewicht/Woche, vorzugsweise 0,05 bis 50 IU/kg Körpergewicht/Woche, eine Endoprothese und gegebenenfalls ein Zelltherapeutikum, vorzugsweise endotheliale Vorläuferzellen oder sonstige zelltherapeutisch einsetzbare Zellpopulation.
